(19) Europäisches Patentamt
European Patent Office
Office européen des brevets

(11) **EP 2 603 577 B1**

(12) **EUROPÄISCHE PATENTSCHRIFT**

(45) Veröffentlichungstag und Bekanntmachung des Hinweises auf die Patenterteilung:
**22.03.2017 Patentblatt 2017/12**

(21) Anmeldenummer: **11749146.4**

(22) Anmeldetag: **12.08.2011**

(51) Int Cl.:
**C12N 5/00** *(2006.01)*

(86) Internationale Anmeldenummer:
**PCT/EP2011/063930**

(87) Internationale Veröffentlichungsnummer:
**WO 2012/020121 (16.02.2012 Gazette 2012/07)**

(54) **MAGNETISCHE GLASPARTIKEL ZUM EINSATZ IN BIOGASANLAGEN, FERMENTATIONS- UND SEPARATIONSPROZESSEN**

MAGNETIC GLASS PARTICLES FOR USE IN BIOGAS PLANTS, FERMENTATION PROCESSES AND SEPARATION PROCESSES

PARTICULES DE VERRE MAGNÉTIQUES À EMPLOYER DANS DES INSTALLATIONS DE PRODUCTION DE BIOGAZ, DES PROCESSUS DE FERMENTATION ET DES PROCESSUS DE SÉPARATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.08.2010 DE 102010034083**

(43) Veröffentlichungstag der Anmeldung:
**19.06.2013 Patentblatt 2013/25**

(73) Patentinhaber: **DENNERT PORAVER GmbH 92353 Postbauer-Heng (DE)**

(72) Erfinder:
• **RUF, Friedrich**
  **84184 Tiefenbach-Ast (DE)**
• **SOHLING, Ulrich**
  **85356 Freising (DE)**
• **NEITMANN, Elisabeth**
  **85368 Moosburg (DE)**
• **LINKE, Bernd**
  **14469 Potsdam (DE)**
• **MUMME, Jan**
  **14469 Potsdam (DE)**
• **RAMM, Patrice**
  **12627 Berlin (DE)**
• **MENHORN, Oliver**
  **90475 Nürnberg (DE)**
• **WEINBERGER, Karl**
  **94253 Bischofsmais (DE)**
• **KUMPF, Peter Dr.**
  **64750 Lützelbach (DE)**

(74) Vertreter: **FDST Patentanwälte Nordostpark 16 90411 Nürnberg (DE)**

(56) Entgegenhaltungen:
EP-A1- 1 900 698      WO-A1-93/10162
WO-A2-01/71732      US-A- 4 233 169

• A. SOARES, B. GUIEYSSE, B. MATTIASSON: BIOTECHNOLOGY LETTERS, Bd. 25, 2003, Seiten 927-933, XP002665594, in der Anmeldung erwähnt

**Beschreibung**

Die Erfindung betrifft ein Verfahren zur Behandlung eines organischen und/oder anorganischen Substrats, ein magnetisierbares Aggregat, wie es in dem Verfahren zur Behandlung eines organischen Substrats eingesetzt wird, sowie ein Verfahren zur Herstellung eines derartigen magnetisierbaren Aggregats.

Fermentationen werden schon seit sehr früher Zeit genutzt, um beispielsweise Lebensmittel haltbar zu machen. Dabei werden entweder Mikroorganismen eingesetzt, beispielsweise bei der Herstellung von Wein und Bier oder bei der Produktion von Joghurt und Kefir, oder direkt Enzyme, beispielsweise beim Dicklegen von Milch durch den Einsatz von Lab. Fermentationen können sowohl unter aeroben Bedingungen als auch unter anaeroben Bedingungen ablaufen. Eine Fermentation unter anaeroben Bedingungen wird auch als Gärung bezeichnet. Fermentationen werden auch bei der großtechnischen Produktion von Chemikalien eingesetzt, beispielsweise bei der Herstellung von Zitronensäure oder bei der Herstellung von Arzneimitteln, wie Antibiotika oder Insulin. Für diese technischen Prozesse sind eine Vielzahl von Reaktoren und Prozessführungen entwickelt worden. So können die Prozesse kontinuierlich geführt werden oder auch batchweise. Bei den Reaktoren sind Typen entwickelt worden, bei welchen die Mikroorganismen oder das Enzym stationär in einem Reaktor angeordnet sind, beispielsweise in einem gepackten Bett, über welches dann das Substrat in flüssiger Form geleitet wird. Es sind aber auch Prozesse bekannt, bei welchen die Mikroorganismen oder das Enzym homogen im Substrat verteilt sind. Dabei können die Mikroorganismen oder das Enzym auf einem festen Träger immobilisiert sein, welcher im Substrat verteilt ist. Bei vielen Reaktionen ist jedoch kein Träger erforderlich.

Die organischen Substrate, welche bei solchen Fermentationen eingesetzt werden, können sehr unterschiedlich sein. Beispielsweise kann von einer wässrigen Glukoselösung ausgegangen werden, welche dem Mikroorganismus dann bei der Erzeugung der gewünschten Substanz als Substrat bzw. Energiequelle dient. Das organische Substrat kann homogen sein und beispielsweise als Lösung vorliegen. Bei anderen technischen Fermentationen werden sehr heterogene organische Substrate eingesetzt. Beispielsweise werden bei der Biogaserzeugung Substrate eingesetzt, die auch feste Bestandteile umfassen können, wobei diese festen Bestandteile auch in Form relativ großer Stücke vorliegen können. Typische organische Substrate, wie sie bei der Biogaserzeugung eingesetzt werden, sind beispielsweise Tierdung, welcher auch mit Einstreu vermischt sein kann, gehäckselte Pflanzenteile oder auch Klärschlamm.

Für Fermentationen werden unterschiedliche Mikroorganismen eingesetzt, beispielsweise Bakterien, Pilze oder auch Zellkulturen. Wird eine Fermentation mithilfe von Mikroorganismen durchgeführt, können die Mikroorganismen bei einer Variante der Prozessführung zunächst das organische Substrat besiedeln. Dazu wird das Substrat in einer Anfahrphase des Bioreaktors zunächst mit dem betreffenden Mikroorganismus angeimpft, welcher sich dann unter Umsetzung des organischen Substrats vermehrt und das organische Substrat besiedelt.

Es sind jedoch auch Mikroorganismen sowie von diesen katalysierte Reaktionen bekannt, bei welchen das Substrat nicht von den Mikroorganismen besiedelt wird. Bei dieser Verfahrensvariante einer Fermentation schwimmen die Mikroorganismen in einer Nährlösung und nehmen gelöste Nährstoffe über ihre Oberfläche auf. Die Mikroorganismen können zuvor Enzyme ausgeschieden haben, um beispielsweise feste Substrate in kleinere wasserlösliche Bausteine zu zerlegen.

Soll der Prozess kontinuierlich geführt werden, wird während der Fermentation frisches organisches Substrat in den Reaktionsraum des Reaktors eingeschleust, während eine entsprechende Menge an verbrauchtem Substrat aus dem Reaktor ausgeschleust wird. Mit dem ausgeschleusten verbrauchten Substrat werden auch Mikroorganismen aus dem Reaktionsraum entfernt. Dieser Verlust an Mikroorganismen muss durch ein entsprechendes Wachstum ausgeglichen werden. Insbesondere bei langsam wachsenden Organismen kann die Wachstumsrate den beschränkenden Faktor für die Leistungsfähigkeit des Reaktors bedeuten. Unterhalb eines kritischen Wertes, bei dem die Menge an nachwachsenden Mikroorganismen größer ist als die Menge an Mikroorganismen, die mit dem verbrauchten Substrat ausgeschleust wird, steht stets eine ausreichende Menge an Mikroorganismen zur Verfügung, um die Fermentation aufrecht zu erhalten. Dann kann beispielsweise die Menge an zugeführtem organischem Substrat den beschränkenden Faktor bilden. Wird die Menge an zugeführtem organischem Substrat weiter gesteigert, wird ein kritischer Wert erreicht, bei welchem die Menge an nachwachsenden Mikroorganismen der Menge an mit dem verbrauchten Substrat ausgeschleusten Mikroorganismen entspricht. Die Fermentation im Reaktionsraum läuft dann stabil ab. Bei einer weiteren Steigerung der Menge an zugeführtem Substrat kann die nachwachsende Menge an Mikroorganismen den Verlust an mit dem verbrauchten Substrat ausgeschleusten Mikroorganismen nicht mehr ausgleichen. Der Inhalt des Reaktors verarmt dann stetig an Mikroorganismen, so dass die Fermentation nicht mehr stabil abläuft. Die Leistung des Reaktors und damit die Ausbeute an Produkt nehmen bei einer weiteren Steigerung der Menge an zugeführtem Substrat ab, bis die Fermentation schließlich zum Erliegen kommt.

Ein Beispiel für eine Fermentation mit sehr langsam wachsenden Mikroorganismen ist die Biogaserzeugung. Bei der Produktion von Biogas wird im letzten Schritt mithilfe methanogener Bakterien aus $H_2$ und $CO_2$ oder aus Essigsäure oder anderen niedermolekularen Verbindungen, wie Methylamin, Methan erzeugt. Diese Umsetzung findet unter strikt anaeroben und reduzierenden Bedingungen statt. Methanogene Bakterien können aus thermodynamischen Gründen nur einen äußerst geringen Energiegewinn pro umgesetztes Substratmolekül erzielen. Lange Generationszeiten sind daher eine zwangsläufige und unabwendbare Folge. Aus diesem Grund dauert auch die Anfahrphase von neu beschickten Biogasreaktoren lange. Hat ein Biogasreaktor schließlich seinen Betriebszustand erreicht, bestimmt die Wachstumsrate der am langsamsten wachsenden Mikroorganismen den maximal möglichen Durchsatz an organischem Substrat.

Um bei einem gegebenen Reaktor die Durchsatzrate und damit die Leistungsfähigkeit des Reaktors zu erhöhen, muss die stationäre Konzentration an Mikroorganismen im Reaktor erhöht werden. Dies kann innerhalb eines sehr beschränkten Rahmens durch eine Optimierung von Prozessparametern erfolgen. Eine weitere Möglichkeit, die stationäre Menge an Mikroorganismen im Reaktionsraum des Bioreaktors zu erhöhen besteht darin, die Mikroorganismen aus dem ausgeschleusten Gärrest zurückzugewinnen und erneut in den Reaktionsraum zurückzuführen. Da der Gärrest jedoch sehr heterogen aufgebaut ist, lassen sich die Mikroorganismen nur sehr schwierig von dem Gärrest abtrennen.

In der DE 10 2005 024 886 B3 wird ein Verfahren beschrieben, bei welchem das organische Substrat mit magnetisierbaren Partikeln versetzt wird. Die Mikroorganismen liegen im Reaktionsraum bzw. im organischen Substrat in Form von Bakterienflocken vor. Die Bakterien werden dabei von einer Schleimschicht umgeben, welche einen Zusammenhalt zwischen den einzelnen Bakterien und den Aufbau größerer Aggregate ermöglicht. In dem von den Mikroorganismen abgesonderten Schleim können magnetisierbare Partikel eingelagert werden, wodurch eine auf die magnetisierbaren Partikel wirkende Kraft auf die Mikroorganismen übertragen werden kann. Es konnte gezeigt werden, dass es möglich ist, die Mikroorganismen aus dem Gärrest abzutrennen, indem der Gärrest an einem stationären Magneten vorbeigeführt wird. Dazu kann ein Abscheiderohr vorgesehen sein, an dessen Außenseite Permanentmagneten befestigt sind. Wird der Gärrest durch das Abscheiderohr geleitet, zieht der stationäre Magnet die im Bakterienschleim eingeschlossenen magnetisierbaren Partikel an, wodurch die Bakterienflocken im Gärrest zum Magneten bewegt und an der Wand des Abscheiderohres abgeschieden werden. Werden die Permanentmagneten von der Außenwand des Abscheiderohres abgenommen, kann die an der Innenwand des Abscheiderohres niedergeschlagene Schicht aus Mikroorganismen aus dem Rohr herausgespült werden. Die gesammelten Mikroorganismen können dann wieder in den Reaktor zurückgeführt werden.

Durch das in der DE 10 2005 024 886 B3 beschriebene Verfahren konnte gezeigt werden, dass die Abscheidung von Mikroorganismen mithilfe magnetisierbarer Partikel möglich ist. Jedoch ermöglicht das Verfahren noch nicht einen so hohen Abscheidungsgrad, dass das Verfahren rentabel in technischen Anlagen eingesetzt werden kann. Der im Verfahren eingesetzte Ferrit wird in sehr feinteiliger Form eingesetzt, sodass seine Sedimentationsgeschwindigkeit niedrig ist und die Partikel in der Schleimschicht der Bakterien eingeschlossen werden können. Diese sehr kleinen magnetisierbaren Partikel bewegen sich im angelegten Magnetfeld wegen der Viskosität des Wassers und der schwachen magnetischen Kräfte nur sehr langsam. Wird versucht, die Abscheiderate der magnetisierbaren Partikel durch eine Steigerung der von den Magneten ausgeübten Kraft zu verbessern, besteht die Gefahr, dass die magnetisierbaren Partikel aus der Schleimhülle der Mikroorganismenflocken herausgerissen werden. Eine Abtrennung der Bakterienflocken aus dem Gärrest ist dann nicht mehr möglich. Wird versucht, die Abscheiderate durch die Verwendung größerer magnetisierbarer Partikel zu verbessern, können diese größeren Partikel zwar von Mikroorganismen besiedelt werden, wodurch sich die Bindung der Mikroorganismen an die magnetisierbaren Partikel verbessert. Es tritt dann aber das Problem auf, dass die Sedimentationsgeschwindigkeit der magnetisierbaren Partikel sehr hoch wird. Die Partikel sind dann während der Fermentation nicht mehr homogen im organischen Substrat verteilt, d.h. die Fermentation kann dann nur noch unter hohem Aufwand gleichmäßig im gesamten Reaktionsraum durchgeführt werden.

Die Möglichkeit, Stoffe oder Mikroorganismen mithilfe magnetisierbarer Partikel aus einem Stoffgemisch heraus anzureichern, wurde schon anhand verschiedener Beispiele gezeigt. Meist betreffen diese Anwendungen jedoch nur die Abtrennung sehr geringer Mengen an Substanz, beispielsweise beim Einsatz in Testkits. Der Einsatz magnetisierbarer Partikel ermöglicht es dann, eine aufwändige Abtrennung durch Zentrifugieren zu vermeiden.

So werden in der WO 01/71732 hochporöse ferromagnetische oder ferrimagnetische Glaspartikel beschrieben, welche Eisenoxid oder eisenhaltige Pigmente enthalten. Die Glaspartikel weisen vorzugsweise einen Durchmesser von 5 bis 25 $\mu$m, insbesondere bevorzugt 7 bis 10 $\mu$m auf. Zur Herstellung der Glaspartikel wird zunächst eine Suspension der magnetisierbaren Partikel in zum Beispiel Glycerin oder Glykol hergestellt. Zu dieser Suspension wird dann eine hydrolisierbare Siliziumverbindung gegeben, beispielsweise ein Tetraalkoxysilan. Die Siliziumverbindung wird dann mit einem alkalischen oder sauren Puffer hydrolisiert, so dass sich $SiO_2$ auf den magnetisierbaren Partikeln niederschlägt und eine offenporige Struktur ausbildet. Die Partikel werden abgetrennt und unterhalb der Curietemperatur getrocknet, bevorzugt im Bereich von 100 bis 500 °C. Die Glaspartikel können dazu verwendet werden, eine bestimmte Substanz, beispielsweise ein Protein, aus einem Probengemisch heraus anzureichern. Dazu wird das poröse Glaspartikel, welches ggf. auf seiner Oberfläche durch Bereitstellung geeigneter Gruppen modifiziert wurde, um die Affinität zwischen der Substanz und der Oberfläche des Glaspartikels zu erhöhen, mit dem Probengemisch vermischt. Die in der Mischung enthaltene Substanz wird auf der Oberfläche der magnetisierbaren Glaspartikel adsorbiert. Durch Anlegen eines externen magnetischen Felds können dann die porösen Glaspartikel zusammen mit den auf diesen adsorbierten interessierenden Molekülen beispielsweise an der Wand eines Probengefäßes abgeschieden werden. Die flüssige Phase kann dann sehr einfach abgetrennt werden, sodass im Wesentlichen nur die magnetisierbaren Glaspartikel mit der darauf adsorbierten Substanz im Probengefäß zurückbleiben.

M.-H. Liao, B.-H. Chen, Biotechnology Letters 24, 1913-1917, 2002 beschreiben ein magnetisierbares Adsorptionsmittel, welches beispielsweise dazu verwendet werden kann, ein Protein aus einer Lösung anzureichern. Dazu werden Nanopartikel aus superparamagnetischem $Fe_3O_4$ mit einer kovalent gebundenen Schicht aus Polyacrylsäure beschichtet. Das Polyacrylat weist eine Vielzahl ionischer Gruppen auf, welche mit dem interessierenden Protein in Wechselwirkung treten können, wodurch das Protein an die Oberfläche des Nanopartikels gebunden wird. Die Polyacrylatketten werden über Aktivierung mit Carbodiimid an das $Fe_3O_4$-Nanopartikel gebunden. Im Mittel werden zwei Polyacrylatmoleküle pro magnetisches Nanopartikel gebunden.

X.-D. Tong, B. Xue, Y. Sun, Biotechnol. Prog. 2001, 17, 134 - 139 beschreiben einen magnetisierbaren Träger, welcher für die Adsorption von Proteinen sowie deren Anreicherung eingesetzt werden kann. Die magnetisierbaren Partikel besitzen einen Kern aus einem magnetisierbaren Material, beispielsweise $Fe_3O_4$, der mit einer Schale aus vernetztem Polyvinylalkohol beschichtet ist. Um die Affinität der Partikel zu Proteinen zu erhöhen, kann die Oberfläche modifiziert werden, indem entsprechende Gruppen an die Oberfläche des Partikels gebunden werden.

Neben den oben beschriebenen magnetisierbaren Partikeln sind für die Durchführung von Fermentationen oder die Anreicherung von Proteinen eine ganze Reihe anderer Träger bekannt. So beschreiben A. Suarez, B. Guieysse, B. Mattiasson, Biotechnology Letters 25, 927 - 933, 2003 den biologischen Abbau von Nonylphenol in einem kontinuierlich betriebenen Festbettbioreaktor. Das Bett besteht aus einem Granulat aus geschäumtem Glas, wobei auf der Oberfläche der Granulatkörner ein Biofilm aus Mikroorganismen immobilisiert ist. Das mit Nonylphenol belastete Substrat wird über das Bett geleitet, wobei im aus dem Reaktor abgeführten Eluat ein deutlicher Abbau des Nonylphenol nachgewiesen werden kann.

H. Zilouei, B. Guieysse, B. Matthiasson, Process Biochemistry 41 (2006), 1083 - 1089 beschreiben den biologischen Abbau von Chlorphenolen mit Hilfe von Bakterien, welche als Biofilm in einem gepackten Bett aus Schaumglasgranulat immobilisiert sind. Auch hier konnte gezeigt werden, dass ein deutlicher Abbau der Chlorphenole erreicht werden kann, wenn ein mit Chlorphenol belastetes Substrat durch den gepackten Reaktor geleitet wird.

Schaumglasgranulate werden in verschiedener Form auf dem Markt angeboten. Die Schaumglasgranulate können eine offenporige oder eine geschlossenporige Struktur aufweisen. Außerdem können die Granulate aus verschiedenen Glassorten hergestellt werden. Beispielsweise kann durch Zusatz von $Al_2O_3$ die Festigkeit und die Temperaturbeständigkeit des Glasgranulats erhöht werden.

In der DE 195 31 801 A1 wird die Herstellung eines offenporigen Blähglasgranulats beschrieben. Dazu wird ein Glaspulver mit einer siliziumorganischen Verbindung sowie Wachskügelchen vermischt. Diese Mischung wird zu einem Granulat verarbeitet, welches zunächst bei niedriger Temperatur vorgehärtet wird. Dabei wird durch teilweise Zersetzung der siliziumorganischen Verbindung ein Zusammenhalt zwischen den Partikeln des Glaspulvers erzeugt. Anschließend wird durch weitere Temperatursteigerung das Wachs ausgeschmolzen, sodass im Granulat offene Poren zurückbleiben. Nach dem Ausschmelzen des Wachses wird das Glasgranulat bei höherer Temperatur, vorzugsweise im Bereich von 600 bis 800 °C ausgehärtet. Das offenporige Glasgranulat eignet sich zur Immobilisierung von Mikroorganismen, die beispielsweise bei der aeroben Abwasserbehandlung eingesetzt werden.

In der DE 197 34 791 A1 wird ebenfalls ein offenporiges Blähglasgranulat beschrieben, welches aus einer Glasmehlmischung mit einer niedrigschmelzenden und einer hochschmelzenden Komponente hergestellt wird. Die Glasmehlmischung wird mit einem Blähmittel vermischt und dann über die Blähtemperatur der niedrigschmelzenden Glaskomponente erhitzt. Beim Blähen öffnen sich die Wände der Poren, die aus der niedrigschmelzenden Komponente aufgebaut sind, sodass eine offenporige Struktur erhalten wird.

Dieses offenporige Blähglasgranulat kann auch als Träger von Mikroorganismen für die aerobe Behandlung von Abwässern verwendet werden, wie in der DE 198 17 268 A1 beschrieben wird. Dazu wird das offenporige Blähglasgranulat zunächst mit etwa 5 Masse-% $Fe_2O_3$ beschichtet, indem das Granulat in eine Eisensalzlösung eingetaucht und anschließend getempert wird. Das Granulat kann dann einem Belebtschlammreaktor zugegeben werden. Durch das Eisenoxid werden schwer abbaubare Verbindungen unter Zusatz von Wasserstoffperoxid anoxidiert, während die weitere Mineralisation von Mikroorganismen bewirkt wird.

Es wurden auch bereits magnetisierbare Schaumglasgranulate beschrieben. So sind aus der EP 1 900 698 A1 und der EP 1 900 697 A1 Schaumgläser bekannt, bei welchen ferromagnetische Stoffe, wie zum Beispiel Eisen, Nickel oder Kobalt homogen im Glasschaum verteilt sind. Als Schaumglas wird ein erstarrter Glasschaum bezeichnet, welcher luftdicht geschlossene Zellen umfasst. Allerdings werden die Herstellung und die Struktur des magnetisierbaren Glasschaums nicht beschrieben. Das magnetisierbare Schaumglas wird zur Herstellung von Filtern verwendet. Diese umfassen ein fluiddichtes Gehäuse mit darin enthaltenen Formkörpern aus Schaumglas. Die Formkörper können beispielsweise ein Granulat mit einer vorgegebenen bzw. weitgehend beliebigen Körnung sein.

Ein weiteres offenporiges magnetisierbares anorganisches Material wird in der US 5,734,020 beschrieben. Zur Herstellung des Materials wird das poröse Glas mit einer Suspension metallischer Partikel imprägniert. Überschüssige Suspension wird entfernt und die imprägnierten Partikel anschließend getrocknet. Da die magnetisierbaren Partikel in den Poren des Glasschaums abgelagert werden, nimmt das Porenvolumen des Glases durch die Imprägnierung ab.

Das Granulat weist eine Korngröße im Bereich von ungefähr 1 bis 200 μm auf. Es kann als Träger für chromatographische Verfahren, in Immunoassays, für Synthesen, beispielsweise von Oligonucleotiden, sowie andere Trenn- und Reinigungsverfahren eingesetzt werden.

In der WO 2004/113245 wird ein Granulat aus Bruchstücken eines aus zermahlenem Hüttenglas gesinterten Sinterkörpers beschrieben, in welches Eisenpartikel eingelagert sind. Der Sinterkörper wird zu einem Granulat gebrochen, sodass eine große Oberfläche erhalten wird, wobei die Eisenpartikel aus der Oberfläche herausragen. Die Herstellung des Granulats erfolgt gemäß einer speziellen Ausführungsform indem zunächst Glasmehl mit einem Blähmittel und feinteiligem Eisen vermischt und die Mischung dann gebläht wird, wobei ein Körper, beispielsweise in Form einer Platte erhalten wird. Die Platte wird dann zu einem Granulat gebrochen. Ab einem Eisengehalt von 6 Gew.-% kann das Granulat magnetisch beeinflusst werden. Dies kann dazu benutzt werden, um beispielsweise Feinteile des Schaumglases aus einer Suspension von Schmutzstoffen abzutrennen. Das Granulat kann insbesondere als Schüttung für die Wasserreinigung verwendet werden.

In der DE 10 2004 012 598 A1 wird ein Verfahren zur Herstellung von Schaumglasgranulat beschrieben, welches sich beispielsweise als Zuschlagstoff für Baumaterialien eignet, um die Isolierwirkung von beispielsweise Wänden zu erhöhen. Zur Herstellung des Schaumglasgranulats wird zunächst vorgemahlenes Glas mit Wasserglas und Blähmittel unter Zugabe von Wasser zu einem Rohansatz vermischt. Der Rohansatz wird dann über mehrere Stunden zu einem Schlicker nassvermahlen. Der Schlicker wird zu Granulatgrünkörpern granuliert und diese werden in Drehrohrofen bei einer Temperatur von üblicherweise 800 bis 900 °C verschäumt.

In der EP 2 022 768 A2 wird ein poröses Material beschrieben, welches einen Gehalt von Silizumoxid in einem Bereich von 60 Gew.-% bis 85 Gew.-%, Aluminiumoxid in einem Bereich von 6 Gew.-% bis 20 Gew.-% und Alkali- und/oder Erdalkalioxid und/oder Alkali- und/oder Erdalkalihydroxid in einem Bereich von 0 bis 15 Gew.-% aufweist. Das poröse Material ist oberflächlich von einer Haut umgeben, die vorzugsweise wasserdicht ist. Das Wasseraufnahmevermögen gemäß DIN EN 1609 beträgt zwischen 0 bis 5 Gew.-%, vorzugsweise 0,2 bis 3 Gew.-%, insbesondere bevorzugt 0,5 bis 1 Gew.-%. Das Schaumglas wird hergestellt, indem Ton, Siliziumoxid sowie gegebenenfalls Alkali- und/oder Erdalkalioxid bzw. Alkali- und/oder Erdalkalihydroxid mit Wasser zu einer Suspension vermengt werden. Die Suspension wird granuliert und das Granulat gegebenenfalls getrocknet. Anschließend wird das Granulat erhitzt, wobei ein geschlossenporiges poröses Material erhalten wird. Um das Aufschäumen zu unterstützen, kann der Suspension gegebenenfalls auch ein Blähmittel zugegeben werden. Es werden verschiedene Anwendungsbereiche für das Schaumglasgranulat vorgeschlagen, beispielsweise als Verdickungsmittel, Wärmeschutzmaterial, Schalldämmmaterial, Füllstoff, Baumaterial, Brandschutzmittel, Feuerfestmaterial, Chromatographiematerial und/oder Trägermaterial.

In der WO 2006/092153 A1 wird ein weiteres Verfahren zur Herstellung von Schaumglasgranulat beschrieben. Dabei wird zunächst bei Raumtemperatur aus Wasser, einem Treibmittel und einem Glasbinder eine Glasbinderschlempe hergestellt. Zur Glasbinderschlempe wird dann Glasmehl zudosiert, sodass ein feuchter, rührfähiger Glasansatz erhalten wird. Der Glasansatz wird homogenisiert und anschließend für 2 bis 6 Stunden gerührt um die Glasbestandteile zumindest teilweise aufzuschließen. Es wird dann weiteres Glasmehl zudosiert und die Mischung granuliert. Das Granulat wird zunächst getrocknet und anschließend bei einer Temperatur von etwa 790 °C aufgeschäumt.

Aus WO 93/10162 A1 ist schließlich ein magnetisierbares Aggregat bekannt, das einen partikulären magnetisierbaren Träger und eine auf dessen Oberfläche immobilisierte aktive Komponente umfasst. Als partikulärer magnetisierbarer Träger wird hierbei ein poröses Glas (Controlled Pore Glass, kurz CPG) herangezogen. Das an sich nicht magnetisierbare Glas wird mit magnetisierbaren Bereichen versehen, indem die porösen Glaspartikel mit einer kolloidalen Suspension von magnetischen Partikeln gemischt werden,
so dass sich die magnetischen Partikel in den Poren des porösen Glases absetzen. Als aktive Komponente kann auf der Oberfläche der magnetisierbar gemachten Glaspartikel eine Beschichtung aus organischen Silanen aufgebracht werden.

Wie bereits erläutert, können Fermentationen für die Herstellung einer Vielzahl von Verbindungen genutzt werden.

Dies können relativ einfache Verbindungen sein, wie Methan, aber auch Verbindungen mit sehr komplexer Struktur, wie beispielsweise bei der Herstellung von Arzneimitteln. Die bei den Fermentationen eingesetzten Biokatalysatoren, wie Enzyme oder Mikroorganismen, können recht wertvoll sein, sei es, dass sie nur aufwändig zur Verfügung gestellt werden können, sei es, dass sie nur in einer begrenzten Menge in einem Reaktor bereitgestellt werden können, beispielsweise bedingt durch die langsame Wachstumsrate eines Mikroorganismus.

Ein weiterer Bereich, in welchem große Vorteile erwartet werden können, wenn biologisch aktive Moleküle, die nur schwer zugänglich sind, mehrfach verwendet werden können, sind Separationsprozesse. Dabei können Fängermoleküle eingesetzt werden, um bestimmte Komponenten aus einer Lösung heraus anzureichern. Als Fängermoleküle können beispielsweise Antikörper, DNA-Fängersonden, RNA-Fängersonden, Protein A, Avidin, Streptavidin oder Proteine mit langen Histidinketten eingesetzt werden. Bisher geht man in der Weise vor, dass man die Lösung, welche die interessierenden Moleküle enthält, über eine Säule leitet, in welcher geeignete Fängermoleküle immobilisiert sind. Enthält die Lösung jedoch beispielsweise Mikroorganismen, so müssen diese zuvor von der Lösung abgetrennt werden.

Der Erfindung lag daher die Aufgabe zugrunde, ein Verfahren zur Behandlung eines organischen Substrats zur Verfügung zu stellen, welches die Rückgewinnung wertvoller Komponenten aus dem behandelten organischen Substrat ermöglicht. Die wertvolle Komponente kann dann erneut verwendet werden oder beispielsweise auch zurückgeführt werden, um beispielsweise die Konzentration an biologisch aktiver Komponente in einem Bioreaktor zu erhöhen.

Diese Aufgabe wird mit einem Verfahren mit den Merkmalen des Patentanspruchs 1 gelöst. Vorteilhafte Ausführungsformen des erfindungsgemäßen Verfahrens sind Gegenstand der abhängigen Ansprüche.

Beim erfindungsgemäßen Verfahren wird ein spezieller magnetisierbarer partikulärer Träger verwendet, welcher aus einem festen Schaum aufgebaut ist und auf welchem eine aktive Komponente immobilisiert ist. In der festen Phase des Schaums sind magnetisierbare Partikel verteilt, wobei diese in die kontinuierliche Phase des festen Schaums eingeschlossen sind. Da die magnetisierbaren Partikel in der kontinuierlichen Phase eingeschlossen sind, wird verhindert, dass die magnetisierbaren Partikel korrodieren und dadurch ihre Magnetisierbarkeit verlieren bzw. aus dem magnetisierbaren partikulären Träger entfernt werden, indem die Partikel beispielsweise herausgelöst werden. Vorzugsweise sind die magnetisierbaren Partikel homogen in der festen Phase verteilt. Durch die Menge des im Träger enthaltenen magnetisierbaren Materials kann die Kraft eingestellt werden, welche ein gegebenes Magnetfeld auf ein Trägerpartikel ausübt und damit die Geschwindigkeit beeinflusst werden, mit welcher sich das Trägerpartikel in einem gegebenen Magnetfeld und einem gegebenen Medium bewegt. Es ist daher möglich, die Abscheiderate der magnetisierbaren Träger zu erhöhen, indem die Menge an magnetisierbarem Material im Träger erhöht wird. Durch die Steigerung der Menge an magnetisierbarem Material erhöht sich die Dichte des ungeschäumten Granulats, da im Allgemeinen das spezifische Gewicht des magnetisierbaren Materials, aus welchem die magnetisierbaren Partikel aufgebaut sind, höher ist, als die Dichte des festen Materials der Hauptphase, eines Netzbildners bzw. eines Glases, welches die kontinuierliche feste Phase des Trägers bilden. Damit würde sich die Sedimentationsgeschwindigkeit des Trägers im organischen Substrat, das im Bioreaktor bereitgestellt ist, erhöhen. Der Sedimentation kann jedoch durch die Porenstruktur des festen Schaums entgegengewirkt werden. Der feste Schaum, aus welchem der magnetisierbare Träger aufgebaut ist, besitzt zumindest im Kern des Trägers eine geschlossenporige Struktur. In die geschlossenen Poren dringt kein Wasser bzw. Lösungsmittel ein, sodass sie in Wasser oder einem anderen Lösungsmittel wie ein Auftriebskörper für den magnetischen Träger wirken. Durch die Porengröße bzw. den Anteil der Poren und die Größe des geschlossenporigen Kerns lässt sich die Dichte des Trägers einstellen. Durch den Grad des Aufschäumens kann daher gesteuert werden, ob der Träger beispielsweise in Wasser absinkt, aufschwimmt oder, wenn der Träger eine vergleichbare Dichte wie Wasser aufweist, in Wasser annähernd in der Schwebe gehalten wird.

Auf dem magnetisierbaren Träger ist eine aktive Komponente immobilisiert. Unter einer aktiven Komponente wird eine Komponente verstanden, die mit dem organischen und/oder anorganischen Substrat in Wechselwirkung treten kann.

Die Wechselwirkung kann darin bestehen, dass das organische und/oder anorganische Substrat an der immobilisierten aktiven Komponente umgesetzt wird. Die aktive Komponente kann bei dieser Ausführungsform beispielsweise als Katalysator wirken, an welchem das Substrat zu einem Produkt umgesetzt wird. Nach der Umsetzung kann das magnetisierbare Aggregat aus der Produktmischung entfernt werden und bei einem kontinuierlich betriebenen Prozess beispielsweise wieder frischer Substratmischung zugesetzt werden. Nach der Umsetzung kann das magnetisierbare Aggregat aber auch gewaschen und konditioniert werden, um danach erneut verwendet zu werden. Alternativ kann es auch bis zu einer erneuten Verwendung aufbewahrt werden.

Gemäß einer bevorzugten Ausführungsform wird als aktive Komponente ein biokatalytisch aktives System eingesetzt. Unter einem biokatalytisch aktiven System wird ein System biologischen Ursprungs verstanden, welches eine Umsetzung eines organischen und/oder anorganischen Substrats zu einem Produkt bewirken kann. Ein System ist biologischen Ursprungs, wenn es in der lebenden Natur vorkommt bzw. aus dieser abgeleitet ist, beispielsweise mit Hilfe gentechnologischer Methoden. Unter einem biologisch aktiven System werden insbesondere lebende Zellen, Teile von Zellen, beispielsweise Vesikel, sowie Enzyme verstanden.

Lebende Zellen können gemäß einer Ausführungsform Zellen aus Zellkulturen sowie Mikroorganismen sein. Unter Mikroorganismen werden allgemein mikroskopisch kleine Organismen verstanden, die bevorzugt als Einzeller oder auch als Mehrzeller vorliegen können. Mikroorganismen sind gemäß einer bevorzugten Ausführungsform ausgewählt aus Archaeobakterien, Eubakterien, Hefen, sowie anderen Pilzen. Die Mikroorganismen können als Reinkulturen, als definierte Mischkulturen oder auch als komplexe Biozönosen verwendet werden. Die Mikroorganismen können natürlich vorkommende Mikroorganismen oder auch mit bekannten Methoden genetisch manipulierte Mikroorganismen oder Zellen sein.

Eine Wechselwirkung zwischen magnetisierbarem Aggregat und organischem und/oder anorganischem Substrat kann aber beispielsweise auch die Ausbildung einer, vorzugsweise nicht kovalenten, Bindung zwischen dem Substrat und der auf dem magnetisierbaren Träger immobilisierten aktiven Komponente sein. Bei dieser Ausführungsform kann das magnetisierbare Aggregat also das Substrat adsorbieren. Dies ermöglicht eine Ausführungsform, bei welcher das erfindungsgemäße Verfahren beispielsweise dazu verwendet wird, das Substrat aus einer, vorzugsweise wässrigen, Mischung heraus anzureichern.

Gemäß einer solchen Ausführungsform wird die aktive Komponente von einem Fängermolekül gebildet. Unter einem Fängermolekül wird ein Molekül verstanden, welches ein, vorzugsweise organisches, Substrat durch zumindest eine nicht-kovalente Bindung binden kann. Die nicht-kovalente Bindung kann durch eine ionische Bindung, eine dipolare Wechselwirkung oder durch van-der-Waals Bindungen gebildet werden. Als Fängermoleküle können kleine Moleküle eingesetzt werden, beispielsweise DNA-Sonden, aber auch größere biologisch aktive Aggregate, beispielsweise Antikörper. Es können aber auch Polymere eingesetzt werden, welche Gruppen umfassen, die nicht-kovalente Bindungen zu einem organischen Substrat ausbilden können, beispielsweise Polyethylenimin.

Das aus dem magnetisierbaren Träger und der darauf immobilisierten aktiven Komponente gebildete magnetisierbare Aggregat lässt sich sehr gut in flüssigen Medien verteilen. Es lässt sich daher sehr gut in kontinuierlichen Prozessen einsetzen, in welchen das magnetisierbare Aggregat aus der Produktmischung abgetrennt und, ggf. nach einer Zwischenbehandlung, wieder in den Prozess zurückgeführt wird. Ist die aktive Komponente beispielsweise als Fängermolekül ausgestaltet, so kann der Träger beispielsweise in ein Kulturmedium gegeben werden, in welchem in einer biologisch katalysierten Reaktion eine interessierende Verbindung erzeugt wird. Diese Verbindung wird dann über das Fängermolekül am magnetisierbaren Aggregat gebunden. Das magnetisierbare Aggregat mit der daran gebundenen interessierenden Verbindung kann dann mit einer magnetischen Abscheidevorrichtung aus dem Kulturmedium abgetrennt und anschließend die Verbindung wieder vom magnetisierbaren Aggregat bzw. dem Fängermolekül verdrängt und damit isoliert werden. Das von der interessierenden Verbindung abgereicherte magnetisierbare Aggregat kann dann wieder in das Kulturmedium gegeben werden. Dieses Verfahren ermöglicht es, kontinuierlich aus einer biologisch katalysierten Reaktion, beispielsweise einer Fermentation, eine bestimmte Verbindung zu isolieren, ohne dass dazu der Prozess unterbrochen oder zuvor der biologische Katalysator, z.B. ein Mikroorganismus, abgetrennt werden müsste.

Gemäß einer bereits weiter oben genannten Ausführungsform ist die aktive Komponente als biokatalytisch aktives System ausgebildet. Unter einem biokatalytisch aktiven System wird im Sinne der Erfindung insbesondere eine lebende Zelle oder ein Teil einer Zelle oder ein Enzym verstanden, welche die Umsetzung eines organischen und/oder anorganischen Substrats zu einem Produkt bewirkt. Substrat und Produkt unterscheiden sich bei dieser Ausführungsform also in ihrer Struktur. Die Umsetzung des Substrats kann beispielsweise bei Verwendung von Enzymen die Anwesenheit von Cofaktoren erfordern. Bei Verwendung von Zellen, insbesondere Mikroorganismen, kann die Anwesenheit von beispielsweise Nährstoffen, Spurenelementen und ähnlichem erforderlich sein. Die Bedingungen für derartige Systeme sind dem Fachmann an sich bekannt.

Besonders vorteilhafte Ergebnisse werden erzielt, wenn die aktive Komponente bzw. das biokatalytisch aktive System durch lebende Zellen und insbesondere durch zumindest einen Mikroorganismus gebildet wird. Der zumindest eine Mikroorganismus kann einer einzelnen Mikroorganismenart angehören. Es ist aber auch möglich, dass mehrere Mikroorganismenarten gleichzeitig als aktive Komponente wirken.

Da der Träger im Vergleich zu den bisher in biologischen Prozessen eingesetzten magnetisierbaren Trägern relativ groß ist, wird dieser nicht lediglich in die Schleimschicht von Mikroorganismen eingebaut, sondern kann von Zellen oder Mikroorganismen besiedelt werden. Die Mikroorganismen bzw. Zellen befestigen sich dabei selbst aktiv mit Hilfe von extrazellulären polymeren Substanzen auf der Oberfläche des Trägers. Die Mikroorganismen können auf der Oberfläche des Trägers zunächst Kolonien ausbilden, die sich bei weiterem Wachstum zu einem Biofilm vereinigen können. Dadurch wird die Dichte an Mikroorganismen im Substrat relativ hoch, sodass hohe Umsatzraten des Substrats verwirklicht werden können. Analoges gilt bei der Verwendung von Zellen, beispielsweise Zellen aus Zellkulturen.

Der Träger wird im erfindungsgemäßen Verfahren in partikulärer Form eingesetzt, d.h. in Form eines schüttbaren Granulats.

Der partikuläre magnetisierbare Träger mit der darauf immobilisierten aktiven Komponente kann homogen im Substrat verteilt werden, was sich ebenfalls vorteilhaft auf die Umsatzrate bzw. Abscheiderate auswirkt, da keine lokalen Ungleichgewichte auftreten und beispielsweise eine lokale Übersäuerung vermieden werden kann. Im Gegensatz zu starren Festbetten besteht bei Verwendung partikulärer Träger auch keine Verstopfungsgefahr, wenn das Substrat beispielsweise feste Bestandteile umfasst.

Durch die feste Schaumstruktur, welche zumindest im Kern des Trägers geschlossenporig ist, ist der Träger mechanisch stabil. Durch mechanische Einwirkung während des Gebrauchs des magnetisierbaren Aggregats erleidet der Träger daher kaum Abnutzungen, sodass das aus dem magnetisierbaren Träger und der darauf immobilisierten aktiven Komponente gebildete magnetisierbare Aggregat wiederholt aus der Substrat- bzw. Produktmischung abgetrennt und erneut eingesetzt werden kann, ohne dass ein wesentlicher Aktivitätsverlust in Kauf genommen werden muss. Gemäß einer Ausführungsform umfasst der Kern des Trägers zumindest 50 % des gesamten Volumens des Trägers. Details zum Träger werden weiter unten erläutert.

Erfindungsgemäß wird daher ein Verfahren zur Behandlung eines organischen und/oder anorganischen Substrats zur Verfügung gestellt, wobei

- in einem Reaktionsraum eine Substratmischung bereitgestellt wird, welche das organische und/oder anorganische Substrat enthält,

- die Substratmischung mit einem magnetisierbaren Aggregat versetzt wird, wobei das magnetisierbare Aggregat einen magnetisierbaren Träger und eine auf dem magnetisierbaren Träger immobilisierte aktive Komponente umfasst;

- die Substratmischung mit dem magnetisierbaren Aggregat zu einer Produktmischung umgesetzt wird, und

- das magnetisierbare Aggregat mit einer magnetischen Abscheidevorrichtung von der Produktmischung abgetrennt wird,

wobei erfindungsgemäß der magnetisierbare Träger in Form eines partikulären magnetisierbaren Trägers vorliegt, wobei der partikuläre magnetisierbare Träger aus einem festen Schaum mit einer kontinuierlichen Phase aufgebaut ist, welche Poren des festen Schaums umgibt, wobei in der kontinuierlichen Phase magnetisierbare Bereiche angeordnet sind, die eine diskontinuierliche Phase bilden, und wobei zumindest im Kern des partikulären magnetisierbaren Trägers der feste Schaum geschlossenporig ist.

Das erfindungsgemäße Verfahren kann an sich mit üblichen Reaktoren durchgeführt werden, welche einen Reaktionsraum aufweisen. Der Reaktor kann batchweise oder auch kontinuierlich betrieben werden. Unter einem batchweisen Betrieb wird verstanden, dass der Reaktionsraum mit einer bestimmten Menge an Substrat gefüllt wird, die Substratmischung zu einer Produktmischung umgesetzt wird, die Produktmischung ggf. aus dem Reaktionsraum entnommen wird, und die Produktmischung aufgearbeitet wird. Das Substrat kann dabei zu Beginn der Umsetzung in der gesamten Menge vorgelegt werden. Es ist aber auch möglich, zu Beginn der Umsetzung nur eine Teilmenge des Substrats vorzulegen und in zeitlichem Abstand weitere Teilmengen an Substrat in den Reaktor zu geben ("fed-batch"). Die Konzentration an Substrat und Produkt ändert sich dabei mit dem zeitlichen Ablauf der Fermentation. Werden als aktive Komponente Mikroorganismen bzw. Zellen eingesetzt, verändert sich deren Konzentration durch ihre Vermehrung ebenfalls während der Umsetzung.

Bei einer kontinuierlichen Prozessführung wird eine annähernd konstante Konzentration des Substrats, des Produkts sowie des magnetisierbaren Aggregats im Reaktionsraum eingestellt. Das organische Substrat wird, ggf. nach einer Anfahrphase des Reaktors, kontinuierlich, d.h. in einem beständigen Zustrom, oder quasi-kontinuierlich, d.h. in mehreren kleinen Portionen dem Reaktionsraum zugeführt und eine entsprechende Produktmenge aus dem Reaktionsraum ausgeschleust. Es stellt sich also ein stationäres Gleichgewicht ein, bei welchem die Konzentration an Substrat, Produkt und magnetisierbarem Aggregat nur innerhalb enger Grenzen schwankt. Die kontinuierliche bzw. quasi-kontinuierliche Fahrweise ist beim erfindungsgemäßen Verfahren bevorzugt.

Die Betriebsweise des Reaktors ist an sich keinen Beschränkungen unterworfen. Es ist beispielsweise möglich, einen kesseiförmigen Reaktor einzusetzen, zum Beispiel einen Rührkesselreaktor, bei welchem der Reaktorinhalt ggf. kontinuierlich oder phasenweise bewegt wird, um den Reaktorinhalt zu homogenisieren. Die Intensität, mit welcher der Reaktorinhalt bewegt wird, hängt von der konkreten Ausgestaltung der Umsetzung ab, die mit dem erfindungsgemäßen Verfahren durchgeführt wird. Werden Mikroorganismen oder lebende Zellen als aktive Komponente des magnetisierbaren Aggregats eingesetzt, wird der Reaktorinhalt vorzugsweise möglichst schonend bewegt, um zu verhindern, dass ein auf dem magnetisierbaren Träger immobilisierter Biofilm durch Scherkräfte abgelöst wird. Es kann dabei ausreichend sein, dass der Reaktorinhalt beispielsweise nur einmal am Tag kurzzeitig bewegt wird.

Es ist aber auch möglich, einen rohrförmigen Reaktortyp zu verwenden, beispielsweise einen Rohrreaktor, bei welchem das Substrat in einer pfropfenförmigen oder turbulenten Strömung durch den Reaktionsraum geführt wird.

Der Reaktor kann mit üblichen Einrichtungen versehen sein. Beispielsweise kann vorgesehen sein, dass der Reaktor thermostatisiert werden kann, also eine Kühlung oder eine Heizung vorgesehen ist. Am Reaktor können Zuführungen und Abführungen vorgesehen sein, mit welchen ein Substrat zugeführt bzw. ein Produkt abgeführt werden kann. Es kann ein Rührwerk vorgesehen sein, mit welchem der Reaktorinhalt bewegt und vermischt werden kann. Weiter können Vorrichtungen vorgesehen sein, um Temperatur, Druck oder verschiedene andere Prozessparameter, wie beispielsweise den pH-Wert zu messen. An sich herrschen bei der Gestaltung des Bioreaktors bzw. des in diesem angeordneten Reaktionsraums keine Beschränkungen. Insbesondere bei Umsetzungen, die unter anaeroben Bedingungen ablaufen, beispielsweise bei der Biogasproduktion, ist der Reaktionsraum bevorzugt geschlossen ausgeführt.

In dem Reaktionsraum wird eine Substratmischung bereitgestellt, welche das Substrat enthält. Die Substratmischung kann eine Lösung sein. Es ist aber auch möglich, die Substratmischung als Suspension auszuführen, wobei auch größere feste Bestandteile, beispielsweise Pflanzenreste, in der Substratmischung enthalten sein können. Die Substratmischung enthält vorzugsweise Wasser als flüssige Phase. Die Substratmischung kann übliche Komponenten enthalten, beispielsweise Puffersysteme, Spurenelemente, Cofaktoren oder Salze. Die Konzentration der einzelnen Bestandteile der Substratmischung wird in Abhängigkeit von der durchgeführten Reaktion innerhalb üblicher Bereiche gewählt.

Enthält die Substratmischung Feststoffe, wird der Feststoffanteil gemäß einer Ausführungsform geringer als 50 Gew.-%, gemäß einer weiteren Ausführungsform geringer als 30 Gew.-% und gemäß einer weiteren Ausführungsform geringer als 5 Gew.-% eingestellt, berechnet als Trockengewicht der festen Bestandteile und bezogen auf das Gewicht der Substratmischung. Der Feststoffgehalt der Suspension wird gemäß einer Ausführungsform so gewählt, dass die Viskosität der Suspension bevorzugt so gering ist, dass eine effiziente Abtrennung der magnetisierbaren Aggregate möglich ist. Wird beispielsweise Getreideschlempe als Substrat eingesetzt, wird wegen der hohen Viskosität der Feststoffgehalt bevorzugt geringer als 3 Gew.-% gewählt.

Gemäß einer Ausführungsform beträgt der Feststoffgehalt der Suspension mehr als 0,1 Gew.-%, gemäß einer weiteren Ausführungsform mehr als 1 Gew.-% und gemäß noch einer weiteren Ausführungsform mehr als 1,5 Gew.-%.

Der pH-Wert der Substratmischung kann in Abhängigkeit von der durchgeführten Reaktion eingestellt werden. Werden gemäß einer Ausführungsform Mikroorganismen für eine Fermentation verwendet, wird der pH-Wert bevorzugt im neutralen Bereich, bevorzugt im Bereich von 1 bis 10, gemäß einer weiteren Ausführungsform im Bereich von 4 bis 9 und gemäß einer besonders bevorzugten Ausführungsform im Bereich von 6 bis 8 gewählt. Werden Enzyme als Biokatalysatoren gewählt, wird der pH-Wert der Substratmischung entsprechend zu deren Aktivitätsprofil eingestellt. Die Konzentration von Salzen, Spurenelementen oder Puffern wird ebenfalls innerhalb üblicher Bereiche gewählt. Analoges gilt für eine Ausführungsform, bei welcher Fängermoleküle als aktive Komponente eingesetzt werden.

Die Substratmischung enthält zumindest ein organisches und/oder anorganisches Substrat, welches mit dem magnetisierbaren Aggregat zu einer Produktmischung umgesetzt wird. Als Substrat kann gemäß einer Ausführungsform ein Substrat eingesetzt werden, welches durch das magnetisierbare Aggregat im Weg einer Fermentation zu einem Produkt umgesetzt wird. Die aktive Komponente wird bei dieser Ausführungsform bevorzugt durch eine Zelle bzw. einen Mikroorganismus gebildet. Organisches und/oder anorganisches Substrat und Produkt haben bei dieser Ausführungsform also eine unterschiedliche Struktur. An sich kann dabei jedes Substrat gewählt werden, welches einer Fermentation zugänglich ist. Geeignete organische Substrate sind beispielsweise Zucker, wie zum Beispiel Glukose, oder Kohlenhydrate, wie Stärke oder Cellulose, Fette, Fettsäuren, Glycerin, stickstoffhaltige Substrate, wie Proteine, Proteinhydrolysate, Peptide, Aminosäuren bzw. Aminosäuregemische. In Abhängigkeit von der durchgeführten Fermentation können als organisches Substrat aber auch komplexe Mischungen eingesetzt werden, wie beispielsweise organische Abfälle, wie Jauche, Gülle, Mist, gehäckselte Pflanzen, Silage, überaltertes Saatgut, oder auch Abwässer oder Klärschlamm, sowie Fermentationsreste, Brennereirückstände, Molke, Algensuspensionen, industrielle organische Rückstände, Speisereste, Lebensmittel mit überschrittenem Datum der Mindesthaltbarkeit, Flotatfette usw.

Das organische und/oder anorganische Substrat kann aber auch ein, vorzugsweise biologisch aktives, Molekül sein, welches aus einer Substratmischung, beispielsweise einem Kulturmedium, mit Hilfe des erfindungsgemäßen Verfahrens abgetrennt wird. Das Substratmolekül wird dabei über ein Fängermolekül an den magnetisierbaren partikulären Träger gebunden, ohne dass eine chemische Reaktion eintritt. Die Struktur des organischen Substrats bleibt bei dieser Ausführungsform des Verfahrens beim Übergang zu einer Produktmischung unverändert. pH-Wert, Salzkonzentration und andere Parameter werden geeignet so gewählt, dass eine feste Bindung zwischen organischem Substrat und magnetisierbarem Aggregat bzw. Fängermolekül erreicht wird.

Die aktive Komponente ist auf einem speziellen magnetisierbaren Träger immobilisiert. Die Immobilisierung kann mit üblichen Verfahren erreicht werden. Beispielsweise kann ein Enzym oder ein Enzymkomplex bzw. ein Fängermolekül auf der Oberfläche des Trägers adsorbiert sein oder auch mit Hilfe eines Spacers kovalent an die Oberfläche des Trägers gebunden sein. Sofern Mikroorganismen als aktive Komponente eingesetzt werden, siedeln diese gemäß einer bevorzugten Ausführungsform auf der Oberfläche des magnetisierbaren Trägers und bilden dort Kolonien bzw. einen Biofilm aus. Gemäß einer anderen Ausführungsform können die Mikroorganismen auch künstlich mit Hilfe von mehr oder weniger gut biologisch abbaubaren Polymeren wie z.B. Polycaprolacton oder Poly-3-Hydroxybuturat oder z.B. Alginat o.ä. auf der Oberfläche des magnetisierbaren Trägers fixiert werden, indem sie in die Polymermatrix eingeschlossen werden. Die Oberfläche des magnetisierbaren Trägers kann zuvor auch chemisch modifiziert / beschichtet worden sein, um die Anheftung von Enzymen, Mikroorganismen oder Fängermolekülen zu erleichtern. Werden Mikroorganismen oder Zellen als aktive Komponente des magnetisierbaren Aggregats eingesetzt, kann der magnetisierbare Träger beispielsweise mit einer Nährlösung, Spurenelementen oder auch Verbindungen beschichtet werden, die ein Aufwachsen von Mikroorganismen oder Zellen auf den magnetisierbaren Träger erleichtern. In der Beschichtung können auch verschiedene Komponenten enthalten sein. Eine beispielhafte Beschichtung ist eine Beschichtung mit Xanthan, welche mit Spurenelementen oder einer Nährstoffkombination kombiniert wird. Als Spurenelemente oder Nährstoffe werden übliche Verbindungen eingesetzt, wie sie aus der Mikrobiologie bekannt sind.

Bei diskontinuierlicher, also batchweiser Fahrweise, wird der Substratmischung bevorzugt bereits zu Beginn der Umsetzung das magnetisierbare Aggregat zugegeben. Werden geträgerte Enzyme als biokatalytisch aktives System verwendet, wird geeignet die gesamte Enzymmenge in geträgerter Form bereitgestellt. Das gleiche gilt wenn Fängermoleküle als aktive Komponente eingesetzt werden. Bei Verwendung von Mikroorganismen als aktive Komponente kann auch nur ein Teil der erforderlichen Mikroorganismen in geträgerter Form zur Substratmischung gegeben werden. Die Substratmischung wird also bei dieser Ausführungsform mit den Mikroorganismen angeimpft, wobei die Mikroorganismen auch bereits in geträgerter Form vorliegen können. Während der Fermentation können sich die Mikroorganismen vermehren und ggf. auch frischen magnetisierbaren Träger besiedeln, welcher der Substratmischung zugegeben wird.

Es erfolgt dann in üblicher Weise eine Umsetzung zu einer Produktmischung, die dann, ggf. nach vorheriger Entnahme aus dem Reaktionsraum, aufgearbeitet wird.

Bei einer kontinuierlichen Fahrweise, welche insbesondere bei Verwendung von Mikroorganismen bevorzugt ist, muss zunächst der Betriebszustand des Reaktors erreicht werden, also ein stationäres Gleichgewicht im Reaktionsraum eingestellt werden.

Gemäß einer ersten Ausführungsform wird mit dem erfindungsgemäßen Verfahren kontinuierlich ein organisches und/oder anorganisches Substrat aus einer Substrat- bzw. Produktmischung entnommen. Die Entnahme kann in einem kontinuierlichen Strom oder aber auch portionsweise erfolgen. Eine kontinuierliche Entnahme ermöglicht eine kontinuierliche Prozessführung im Reaktionsraum eines Reaktors, d.h. im Reaktionsraum kann sich ein Fließgleichgewicht einstellen, bei welchem Substrat dem Reaktionsraum zugeführt und Produkt aus dem Reaktionsraum entnommen wird, wobei die Bedingungen im Reaktionsraum im wesentlichen unverändert bleiben.

Die Substratmischung kann beispielsweise ein Kulturmedium sein, in welchem mit Hilfe eines Mikroorganismus biotechnologisch ein bestimmter Wirkstoff hergestellt wird. Der Wirkstoff kann selbst das organische Substrat sein, welches mit dem erfindungsgemäßen Verfahren aus dem Kulturmedium entfernt wird. Das organische Substrat kann aber auch zu einer anderen Stoffgruppe gehören, z.B. Stoffe welche sich hemmend auf den biotechnologischen Prozess auswirken (Inhibitoren). Gemäß dieser Ausführungsform kann also kontinuierlich eine Komponente aus dem Kulturmedium entfernt werden, die sich beispielsweise hemmend auf die im Kulturmedium ablaufende Umsetzung auswirkt, beispielsweise weil sie hemmend für das Wachstum von Zellen wirkt.

Als geträgerte aktive Komponente würde bei dieser Ausführungsform ein Fängermolekül eingesetzt werden. Das Fängermolekül könnte dann das organische und/oder anorganische Substrat binden, sodass dieses mit Hilfe des magnetisierbaren Aggregats über eine magnetische Abscheidevorrichtung aus dem Substratgemisch entfernt werden könnte. Bei dieser Ausführungsform können Mikroorganismen im Kulturmedium enthalten, aber im Wesentlichen nicht an dem magnetisierbaren Träger gebunden sein.

Mikroorganismen und magnetisierbares Aggregat können gemeinsam im Kulturmedium enthalten sein. Es ist aber auch eine Ausführungsform möglich, bei welcher in einem ersten Schritt die Mikroorganismen von dem Kulturmedium abgetrennt werden und in einem weiteren Schritt mit Hilfe des magnetisierbaren Aggregats das organische Substrat aus dem verbleibenden Medium abgetrennt wird.

Die Entfernung eines organischen und/oder anorganischen Substrats aus einer Substratmischung wurde unter Bezugnahme auf die Isolierung eines organischen Substrats aus einem Kulturmedium erläutert, wobei das organische Substrat in einem biotechnologischen Prozess mit Hilfe von Mikroorganismen erzeugt wurde. Das Substrat kann aber auch auf andere Weise erzeugt worden sein. An sich bestehen hier keine Beschränkungen. Das organische und/oder anorganische Substrat kann an sich auch in einer chemischen Reaktion erzeugt worden sein. Das Substrat wird durch nicht-kovalente Bindungen über das Fängermolekül an das magnetisierbare Aggregat gebunden welches dann mittels eines magnetischen Abscheiders aus der Substrat- bzw. Produktmischung abgetrennt wird.

Gemäß einer weiteren bevorzugten Ausführungsform wird das magnetisierbare Aggregat von einem magnetisierbaren Träger und einem darauf immobilisierten Mikroorganismus gebildet. Die Umsetzung des Substrats wird von dem Mikroorganismus bewirkt. Bei dieser Ausführungsform wird das magnetisierbare Aggregat bevorzugt mit der Produktmischung aus dem Bioreaktor ausgeschleust und einer magnetischen Abscheidevorrichtung zugeführt. In der magnetischen Abscheidevorrichtung wird das magnetisierbare Aggregat dann von der Produktmischung abgetrennt und kann ggf. wieder in den Reaktor zurückgeführt werden.

Allgemein wird beim erfindungsgemäßen Verfahren der Reaktionsraum mit der Substratmischung befüllt und das organische und/oder anorganische Substrat mit dem magnetisierbaren Aggregat zu einer Produktmischung umgesetzt, bis sich ein Reaktionsgleichgewicht eingestellt hat. Das Gleichgewicht kann stationär oder auch ein Fließgleichgewicht sein. Während der Umsetzung können ggf. weitere Reaktionskomponenten, beispielsweise Nährstoffe, zugesetzt werden.

Zu Beginn der Umsetzung, wenn also der Reaktor erstmalig bzw. neu mit organischem und/oder anorganischem Substrat beschickt wird, kann bereits das magnetisierbare Aggregat zur Substratmischung gegeben werden. Dies ist beispielsweise bei enzymatisch durchgeführten Fermentationen bevorzugt, bei welchen bereits zu Beginn der Fermentation das geträgerte Enzym zur Substratmischung gegeben wird. Diese Ausführungsform ist auch bevorzugt, wenn mit dem erfindungsgemäßen Verfahren ein organisches Substrat aus einer Produktmischung abgetrennt werden soll, also das magnetisierbare Aggregat einen magnetisierbaren Träger sowie ein darauf immobilisiertes Fängermolekül umfasst. Werden Mikroorganismen als Biokatalysatoren eingesetzt, können diese in einer Form zugegeben werden, bei welcher die betreffenden Mikroorganismen bereits den Träger besiedelt haben oder auch auf künstliche Weise auf dem Träger immobilisiert wurden. Dies kann beispielsweise durch Einschlussimmobilisierung der Mikroorganismen erfolgen. Dabei werden die Mikroorganismen in einer Schicht aus Polymer eingeschlossen, welche auf der Oberfläche des magnetischen Trägers abgeschieden wird. Gemäß einer Ausführungsform wird für die Immobilisierung der Mikroorganismen ein Polymer verwendet.

Gemäß einer anderen Ausführungsform wird für die Immobilisierung der Mikroorganismen ein Gemisch aus zumindest zwei Polymeren verwendet. Eines der Polymere kann dabei leicht von den Mikroorganismen abgebaut werden. Dieses Polymer dient den Mikroorganismen in der Ansiedlungsphase auch als Nährstoff. Sein Anteil an dem Polymergemisch wird vorzugsweise im Bereich von 10 bis 40 Gew.-% gewählt, bezogen auf das Gesamtgewicht der Polymermischung. Ein geeignetes Polymer ist beispielsweise Poly-3-hydroxy-butyrat. Das zweite Polymer ist biologisch schwer abbaubar. Es dient vor allem als Gerüstsubstanz. Sein Anteil wird bevorzugt im Bereich von 60 bis 90 Gew.-% gewählt, bezogen auf das Gesamtgewicht der Polymermischung. Ein geeignetes biologisch schwer abbaubares Polymer ist beispielsweise Polycaprolacton.

Gemäß einer anderen Ausführungsform wird jedoch in der Weise vorgegangen, dass der magnetisierbare Träger ohne eine vorherige Besiedlung durch Mikroorganismen zur Substratmischung gegeben wird und die Substratmischung mit dem betreffenden Mikroorganismus bzw. mit einer betreffenden Mikroorganismenmischung angeimpft wird. Dazu können in der Anlaufphase Mikroorganismen zum Beispiel in Form von flüssigem oder festem Impfmaterial aus anderen Fermentationsanlagen hinzugefügt werden.

Während einer Anfahrphase vermehren sich die Mikroorganismen, wobei auch der der Substratmischung zugegebene magnetisierbare Träger von den Mikroorganismen besiedelt wird, sodass sich erfindungsgemäß ein magnetisierbares Aggregat ausbildet. Gegebenenfalls kann während der Anfahrphase frisches organisches Substrat zugesetzt und verbrauchtes Substrat bzw. gegebenenfalls bereits Produktmischung aus dem Reaktionsraum entnommen werden. Während der Anfahrphase werden geeignete Prozessparameter regelmäßig überwacht, wie pH-Wert, Temperatur, Substratkonzentration, Intermediatkonzentration, Endproduktkonzentration oder Besiedelungsgrad des magnetisierbaren Trägers durch Mikroorganismen, um die Stabilität der Fermentation zu überwachen. Steht eine ausreichende Menge an Mikroorganismen zur Verfügung und hat sich ein stationäres Gleichgewicht im Reaktionsraum eingestellt, ist der Betriebszustand des Bioreaktors erreicht. Bei einer kontinuierlichen Fahrweise wird dann beständig oder in zeitlichem Abstand frisches organisches Substrat zugegeben bzw. verbrauchtes Substrat, d.h. Produktmischung aus dem Reaktionsraum ausgeschleust.

Nachdem sich bei kontinuierlicher Fahrweise des Reaktors der Betriebszustand eingestellt hat, wird die Substratmischung an dem geträgerten biokatalytisch aktiven System, insbesondere dem zumindest einen Mikroorganismus kontinuierlich zu einer Produktmischung umgesetzt. Dazu wird dem Reaktionsraum kontinuierlich frische Substratmischung zugeführt und eine entsprechende Menge an Produktmischung aus dem Reaktionsraum entnommen. Die Produktmischung kann dann weiter verarbeitet werden.

Die Produktmischung kann feste, flüssige oder auch gasförmige Komponenten enthalten. Eine Produktmischung kann beispielsweise eine Lösung oder Suspension sein, in welcher sich das Produkt angereichert hat.

Gemäß einer Ausführungsform kann die Produktmischung jedoch auch aus einer Lösung oder Suspension sowie einer gasförmigen Komponente bestehen. Die gasförmige Komponente wird gesammelt und regelmäßig aus dem Reaktionsraum ausgeschleust. Die flüssigen bzw. festen Komponenten können ggf. getrennt über einen separaten Ausgang aus dem Reaktionsraum ausgeschleust werden. Bei dieser Ausführungsform kann die flüssige bzw. feste Komponente beispielsweise bei der Biogaserzeugung einen Gärrest bilden, während die gasförmige Komponente, beispielsweise ein methanhaltiges Gas, als weitere Produktkomponente erhalten wird.

Bei der Aufarbeitung der Produktmischung wird das magnetisierbare Aggregat erfindungsgemäß mit einer magnetischen Abscheidevorrichtung von der Produktmischung abgetrennt. Als magnetische Abscheidevorrichtung können an sich alle bekannten magnetischen Abscheidevorrichtungen eingesetzt werden. Geeignet sind beispielsweise Permanentmagneten, die stationär oder beweglich in oder an der Abscheidevorrichtung angeordnet sein können. Es können in gleicher Weise jedoch auch beispielsweise Elektromagneten eingesetzt werden. Stationäre Magnete sammeln das magnetisierbare Aggregat an einer bestimmten Stelle in der Abscheidevorrichtung an, während beweglich angeordnete magnetische Abscheidevorrichtungen das magnetisierbare Aggregat nach der Abtrennung beispielsweise zu einer bestimmten Stelle transportieren und dort sammeln können. Dazu kann beispielsweise ein Permanentmagnet entlang der Außenwand eines Abscheiderohres verschoben werden. Nachdem das magnetisierbare Aggregat mit Hilfe der magnetischen Abscheidevorrichtung von der Produktmischung abgetrennt worden ist, kann dieses gesammelt werden, indem beispielsweise die magnetische Abscheidevorrichtung abgeschaltet wird, sodass sich das magnetisierbare Aggregat beispielsweise wieder von einer Wand der magnetischen Abscheidevorrichtung löst und beispielsweise abgespült und gesammelt werden kann.

Die magnetische Abscheidevorrichtung kann im Reaktionsraum selbst angeordnet sein, beispielsweise als ummantelter stabförmiger Magnet. Es ist aber auch möglich, die Abscheidevorrichtung außerhalb des Reaktionsraums vorzusehen und die Produktmischung, beispielsweise einen Gärrest, aus dem Reaktionsraum in die magnetische Abscheidevorrichtung zu überführen. Bevorzugt befindet sich die magnetische Abscheidevorrichtung im Ablauf des Reaktors.

Erfahrungsgemäß werden beim Durchgang durch den magnetischen Abscheider nicht alle magnetisierbaren Aggregate aus der Produktmischung entfernt, sodass Verluste hingenommen werden müssen. Insbesondere beim Anfahren des Prozesses werden magnetisierbare Aggregate, die nur schwach magnetisierbar sind, nicht vom magnetischen Abscheider aus der Produktmischung abgetrennt. Dies kann beispielsweise daran liegen, dass die Partikel des magnetisierbaren Trägers zu klein sind, sodass sie bei einer gegebenen Viskosität der Produktmischung und einem gegebenen Produktstrom durch den magnetischen Abscheider vom magnetischen Abscheider nicht erfasst werden. Während des Anfahrens der Umsetzung kann daher eine Selektion innerhalb der magnetisierbaren Träger bzw. Aggregaten stattfinden, sodass sich die Größenverteilung der Träger gegenüber dem ursprünglichen Zustand verändert.

Beim erfindungsgemäßen Verfahren wird ein spezieller Träger eingesetzt, auf welchem die aktive Komponente immobilisiert ist um erfindungsgemäß ein magnetisierbares Aggregat zu bilden. Durch die Verwendung eines partikulären Trägers kann das magnetisierbare Aggregat homogen in der Substratmischung verteilt werden. Dies ermöglicht eine effiziente Durchführung des Verfahrens, da beispielsweise eine Fermentation des organischen Substrats gleichmäßig im gesamten Reaktionsraum ablaufen kann.

Der partikuläre Träger ist aus einem festen Schaum aufgebaut. Durch den festen Schaum erhält der Träger eine hohe mechanische Stabilität, sodass er auch höheren mechanischen Belastungen standhalten kann, wie sie beispielsweise auftreten, wenn das erfindungsgemäße Verfahren zur Biogaserzeugung eingesetzt wird. Zumindest im Kern des Trägers weist der feste Schaum eine geschlossenporige Struktur auf. Durch die geschlossenen Poren, welche gasgefüllt sind, erhält der magnetisierbare Träger Auftrieb, wenn er in ein flüssiges, insbesondere wässriges Medium eingebracht wird.

Durch den Kern aus einem geschlossenporigen Schaum kann der partikuläre Träger relativ groß ausgeführt werden, ohne dass der magnetisierbare Träger ein hohes spezifisches Gewicht und damit eine hohe Sedimentationsgeschwindigkeit aufweist. Gemäß einer Ausführungsform weist der magnetisierbare Träger einen Durchmesser von bis zu 10 mm, gemäß einer weiteren Ausführungsform einen Durchmesser von bis zu 5 mm, gemäß einer weiteren Ausführungsform einen Durchmesser von bis zu 2 mm auf. Die Größe der magnetisierbaren Träger lässt sich mit üblichen Verfahren einstellen, beispielsweise Sieben oder Windsichten. Um den magnetisierbaren Träger gut handhaben und insbesondere innerhalb technisch interessanter Prozesszeiten wieder von der Prozessmischung abtrennen zu können, hat es sich als vorteilhaft erwiesen, wenn der Durchmesser des Träger größer als 50 $\mu$m, gemäß einer weiteren Ausführungsform größer als 100 $\mu$m gewählt wird. Insbesondere wenn Mikroorganismen als biologisch aktive Komponente verwendet werden, wird der Durchmesser des magnetisierbaren Trägers bevorzugt zumindest so groß gewählt, dass die Mikroorganismen auf dem Träger aufwachsen können. Gemäß einer Ausführungsform beträgt der Durchmesser des magnetisierbaren Trägers zumindest 150 $\mu$m, gemäß einer weiteren Ausführungsform zumindest 200 $\mu$m. Die Größe des magnetisierbaren Trägers lässt sich beispielsweise durch Sieben bestimmen.

Wird das erfindungsgemäße Verfahren dazu verwendet, ein organisches und/oder anorganisches Substrat durch Adsorption an dem magnetisierbaren Aggregat aus einer Substratmischung zu isolieren, ist bevorzugt, dass der magnetisierbare Träger einen relativ kleinen Durchmesser aufweist, da dadurch eine hohe Oberfläche erzeugt wird, auf welcher die aktive Komponente in Form eines Fängermoleküls bereitgestellt wird, das wiederum mit dem Substrat eine nicht-kovalente Bindung eingehen kann, um das Substrat am magnetisierbaren Aggregat zu binden. Bei dieser Ausführungsform wird der mittlere Durchmesser $D_{50}$ des magnetisierbaren Trägers bevorzugt kleiner als 500 $\mu$m, gemäß einer weiteren Ausführungsform kleiner als 300 $\mu$m gewählt. Um eine effiziente Abtrennung des magnetisierbaren Aggregats aus der Substrat- bzw. Produktmischung zu erreichen, wird der Durchmesser des magnetisierbaren Trägers bevorzugt größer als 0,5 $\mu$m, gemäß einer Ausführungsform größer als 1 $\mu$m gewählt. Der Durchmesser des Trägers beträgt gemäß einer Ausführungsform weniger als 2 mm, gemäß einer Ausführungsform weniger als 1 mm.

Der partikuläre Träger kann eine Kugelform aufweisen. Es ist aber auch möglich, den Träger in anderer geometrischer Form auszubilden, beispielsweise als unregelmäßig geformter dreidimensionaler Körper.

Der Träger weist zumindest in seinem Kern im festen Schaum geschlossene Poren auf. Durch den Anteil der geschlossenen Poren am Gesamtvolumen des magnetisierbaren Trägers kann die Dichte des Trägers eingestellt, insbesondere erniedrigt werden. Es ist daher nicht wie bei den aus dem Stand der Technik bekannten Verfahren erforderlich, die Abmessungen der magnetisierbaren Partikel klein zu wählen, um eine Sedimentation des Trägers zu unterbinden oder diese zumindest zu verlangsamen. Durch die geschlossenen Poren kann sich die Dichte des Trägers während seines Einsatzes in der Fermentation allenfalls geringfügig durch eindringendes Wasser erhöhen. Vorzugsweise wird die Dichte des magnetisierbaren Trägers im Bereich von 0,8 bis 1,2 g/ml gewählt. Die Kornrohdichte des magnetisierbaren Trägers kann nach DIN V18004,5.3 (EN1097-6:2000) bestimmt werden.

Die Größe der Poren kann an sich beliebig gewählt werden. Bevorzugt wird die Porengröße so gewählt, dass der Träger mit den darauf immobilisierten Biokatalysator in der Substratmischung keine wesentliche Neigung zur Sedimentation oder zum Aufschwimmen zeigt und die mechanische Stabilität des Trägers gewährleistet ist.

Der Träger kann über sein gesamtes Volumen hinweg eine geschlossenporige Schaumstruktur aufweisen. Der magnetisierbare Träger weist in diesem Fall auf seiner äußeren Fläche eine relativ glatte Struktur mit einer im Wesentlichen durchgehenden Haut auf. Gemäß einer anderen Ausführungsform weist der magnetisierbare Träger jedoch nur in seinem Kern bzw. nur in einem Teil seines Volumens eine geschlossenporige Struktur auf. Der Kern bildet also im Inneren des magnetisierbaren Trägers einen Abschnitt, in welchem der feste Schaum eine geschlossenporige Struktur aufweist.

Durch die Schaumstruktur erhält der magnetisierbare Träger trotz seines geringen Gewichts eine hohe mechanische Stabilität. Der Träger widersteht auch mechanischen Belastungen, die beispielsweise durch Scherkräfte beim Bewegen der Substratmischung oder bei der magnetischen Abscheidung auf den Träger einwirken. Der Träger lässt sich daher über einen langen Zeitraum im Prozess einsetzen ohne dass eine wesentliche Abnutzung eintritt. Um einen kontinuierlichen Prozess mit Rückführung des magnetisierbaren Aggregats in den Reaktionsraum aufrecht zu erhalten genügt es, lediglich den Anteil des Trägers bzw. des magnetisierbaren Aggregats in der Substratmischung zu ergänzen, der durch die unvollständige magnetische Abscheidung unvermeidlich verloren geht.

Der feste Schaum des Trägers umfasst eine kontinuierliche Phase. Die kontinuierliche Phase wird aus einem Netzbildner gebildet. Als Netzbildner wird ein anorganische Material, nämlich ein Glas, verwendet, das eine kontinuierliche Struktur ausbilden kann.

Der Netzbildner ist bei den Bedingungen, unter welchen die Fermentation durchgeführt wird, fest. Er zeigt bevorzugt keine Löslichkeit in der flüssigen Phase, insbesondere Wasser, welche in der Substratmischung enthalten ist. In der kontinuierlichen Phase sind magnetisierbare Bereiche angeordnet, die gemäß einer Ausführungsform eine diskontinuierliche Phase bilden. Die magnetisierbaren Bereiche können beispielsweise durch magnetisierbare Partikel gebildet werden, die in der kontinuierlichen Phase angeordnet sind. Die magnetisierbaren Bereiche werden vorzugsweise von der kontinuierlichen Phase umschlossen, sodass die magnetisierbaren Bereiche innerhalb der kontinuierlichen Phase angeordnet sind. Dadurch lässt sich verhindern, dass die magnetisierbaren Bereiche durch Korrosionsprozesse beeinflusst und beispielsweise herausgelöst werden. Der Träger soll bevorzugt nicht mit der Substratmischung oder der aktiven Komponente in eine Wechselwirkung treten, welche das Verfahren nachteilig beeinflusst, beispielsweise wenn das Verfahren für eine Fermentation eingesetzt wird und die aktive Komponente durch einen Mikroorganismus oder ein Enzym gebildet wird.

Die magnetisierbaren Bereiche bzw. die magnetisierbaren Partikel sind vorzugsweise homogen in der kontinuierlichen Phase des Netzbildners verteilt. Die Ausdehnung der magnetisierbaren Bereiche wird vorzugsweise klein gewählt. Gemäß einer Ausführungsform weisen die magnetisierbaren Bereiche einen Durchmesser von weniger als 500 $\mu$m auf. Gemäß einer Ausführungsform liegt der Durchmesser der magnetisierbaren Bereiche im Bereich von 100 nm bis 400 $\mu$m, gemäß einer weiteren Ausführungsform im Bereich von 200 nm bis 100 $\mu$m. Bei der praktischen Anwendung hat sich als vorteilhaft herausgestellt, wenn die Größe der magnetischen Partikel in einem Bereich von 0,3 bis 25 $\mu$m gewählt wird.

Die magnetisierbaren Bereiche können auch durch Nanopartikel gebildet werden, welche vorzugsweise superparamagnetische Eigenschaften aufweisen. Gemäß einer Ausführungsform liegt der Durchmesser der magnetisierbaren Bereiche innerhalb eines Bereichs von 1 nm bis 500 nm, vorzugsweise 1 nm bis 100 nm. Um superparamagnetische Eigenschaften zu erhalten, wird die Größe der magnetisierbaren Partikel vorzugsweise in einem Bereich von 1 bis 10 nm gewählt. Die Partikelgröße der magnetisierbaren Bereiche lässt sich geeignet nach dem Vermahlen der Ausgangsstoffe bestimmen. Nach dem Einschmelzen in den Netzbildner aus Glas, lässt sich der Durchmesser der magnetisierbaren Partikel nur noch sehr schwer bestimmen.

Durch die homogene Verteilung der magnetisierbaren Bereiche im partikulären Träger können größere Mengen an magnetisierbaren Partikeln im Träger vorgesehen werden. Im magnetischen Abscheider kann daher bei einer vorgegebenen Feldstärke eine höhere Kraft auf den partikulären Träger wirken, sodass der geträgerte Biokatalysator bzw. allgemein das magnetisierbare Aggregat schneller und damit vollständiger von der Produktmischung abgetrennt werden kann.

Der Anteil der magnetisierbaren Partikel, durch welche magnetisierbare Bereiche in der kontinuierlichen Phase des festen Schaums gebildet werden, wird vorzugsweise in einem Bereich von 8 bis 40 Gew.-% gemäß einer weiteren Ausführungsform im Bereich von 15 bis 30 Gew.-% und gemäß einer weiteren Ausführungsform im Bereich von 18 bis 25 Gew.-% gewählt, bezogen auf das Gewicht des Trägers. Der Anteil der kontinuierlichen Phase wird vorzugsweise im Bereich von 92 bis 60 Gew.-%, gemäß einer weiteren Ausführungsform im Bereich von 85 bis 70 Gew.-% und gemäß einer weiteren Ausführungsform im Bereich von 82 bis 75 Gew.-% gewählt. Die prozentualen Anteile beziehen sich auf das Gewicht des partikulären Trägers und entsprechen im Wesentlichen den Anteilen von magnetisierbaren Partikeln und dem Material der kontinuierlichen Phase, wie sie bei der Herstellung des Trägers eingesetzt werden.

Die Menge des in der Substratmischung bereitgestellten magnetisierbaren Aggregats wird in Abhängigkeit von den Bedingungen gewählt, unter welchen das erfindungsgemäße Verfahren durchgeführt wird. Bezogen auf das Gewicht des trockenen organischen Substrats wird die Menge des magnetisierbaren Aggregats gemäß einer Ausführungsform im Bereich von 1 bis 70 Gew.-%, gemäß einer weiteren Ausführungsform im Gereich von 1 bis 20 Gew.-% und gemäß noch einer weiteren Ausführungsform im Bereich von 2 bis 5 Gew.-% gewählt.

Wie bereits erläutert, kann die Abtrennung des magnetisierbaren Aggregats von der Produktmischung bereits im Reaktionsraum erfolgen. Dieses Verfahren kann beispielsweise bei diskontinuierlich geführten Verfahren, beispielsweise bei absatzweisen Fermentationen angewendet werden. Dazu kann beispielsweise eine magnetische Abscheidevorrichtung, zum Beispiel ein ummantelter stabförmiger Magnet, in die Produktmischung eingetaucht werden. Nachdem sich das magnetisierbare Aggregat an der magnetischen Abscheidevorrichtung gesammelt hat, wird die Abscheidevorrichtung mit dem anhaftenden magnetisierbaren Aggregat wieder aus der Produktmischung entnommen.

Gemäß einer bevorzugten Ausführungsform ist jedoch vorgesehen, dass die Produktmischung aus dem Reaktionsraum in eine magnetische Abscheidevorrichtung überführt wird, in welcher das magnetisierbare Aggregat dann von der Produktmischung abgetrennt wird. Eine solche Ausführungsform ist insbesondere vorteilhaft, wenn das Verfahren in einer kontinuierlichen Fahrweise durchgeführt wird. Dabei wird eine bestimmte Menge der Produktmischung aus dem Reaktionsraum ausgeschleust und der magnetischen Abscheidevorrichtung zugeführt. Das Ausschleusen kann portionsweise oder in einem beständigen Produktstrom erfolgen. Als magnetische Abscheidevorrichtung kann beispielsweise ein Rohr vorgesehen werden, durch welches die Produktmischung geleitet wird. Innerhalb des Rohres kann beispielsweise ein stabförmiger Magnet vorgesehen sein, an welchem sich das magnetisierbare Aggregat anreichert. Es ist aber auch möglich, beispielsweise an der Außenwand des Rohres Magneten anzuordnen, sodass sich das magnetisierbare Aggregat an der Innenwand der magnetischen Abscheidevorrichtung niederschlägt. Die Form der magnetischen Abscheidevorrichtung sowie die Stärke der eingesetzten Magneten wird so gewählt, dass eine möglichst hohe Abscheiderate des magnetisierbaren Aggregats erreicht wird. Gemäß einer Ausführungsform werden mehr als 60 Gew.-%, gemäß einer weiteren Ausführungsform mehr als 80 Gew.-% und gemäß noch einer weiteren Ausführungsform mehr als 90 Gew.-% und gemäß noch einer weiteren Ausführungsform mehr als 98 % des in der Produktmischung enthaltenen magnetisierbaren Aggregats in der magnetischen Abscheidevorrichtung abgetrennt. Die prozentualen Angaben beziehen sich auf einen einmaligen Durchgang des Substrats durch die magnetische Abscheidevorrichtung. Eine vollständige Abtrennung des magnetisierbaren Aggregats bzw. des geträgerten Biokatalysators ist bei der technischen Durchführung des Verfahrens nur schwer zu erreichen. Nach dem Durchlaufen der magnetischen Abscheidevorrichtung sind in der Produktmischung vorzugsweise weniger als 1 Gew.-%, gemäß einer weiteren Ausführungsform weniger als 0,5 Gew.-% und gemäß noch einer weiteren Ausführungsform weniger als 0,1 Gew.-% des ursprünglich nach dem Ausschleusen in der Produktmischung enthaltenen magnetisierbaren Aggregats verblieben.

Das aus der Produktmischung abgetrennte magnetisierbare Aggregat kann zurückgewonnen und gegebenenfalls in einem weiteren Verfahren, beispielsweise einer weiteren Fermentation eingesetzt werden.

Gemäß einer bevorzugten Ausführungsform wird das aus der Produktmischung abgetrennte magnetisierbare Aggregat in den Reaktionsraum zurückgeführt. Auf diese Weise lässt sich insbesondere bei kontinuierlich geführten Fermentationen die stationäre Menge an biokatalytisch aktivem System, insbesondere Mikroorganismen, im Reaktionsraum des Reaktors erhöhen. Dadurch kann auch die Belastung des Reaktors, das heißt der Durchsatz an organischem Substrat und damit die Ausbeute an Produkt erhöht werden. Dies ist insbesondere vorteilhaft, wenn als aktive Komponente des magnetisierbaren Aggregats sehr langsam wachsende Mikroorganismen eingesetzt werden.

Gemäß einer Ausführungsform weisen die als biologisch aktive Komponente eingesetzten Mikroorganismen eine Generationszeit von mehr als 8 Stunden, gemäß einer weiteren Ausführungsform von mehr als 24 Stunden auf. Unter der Generationszeit wird der Zeitraum verstanden, innerhalb dessen die Zellen sich unter optimalen Wachstumsbedingungen einmal teilen, d.h. innerhalb dessen sich die Zellzahl verdoppelt. Die Generationszeiten sind sehr stark von den Reaktionsbedingungen abhängig und können sich unter ungünstigen Bedingungen vervielfachen. Schnell wachsende Mikroorganismen wie z.B. *E.Coli* haben unter optimalen Wachstumsbedingungen eine Generationszeit von nur 20 min. Langsam wachsende Mikroorganismen, z.B. manche Nitrifikanten, weisen unter optimalen Wachstumsbedingungen Generationszeiten von 8 - 24 h auf. Unter nicht optimalen Bedingungen, z.B. bei winterlichen Temperaturen in Abwasserreinigungsanlagen können sich deren Generationszeiten auf > 18 d verlängern. Sehr langsam wachsende Mikroorganismen wie die meisten Methangasbakterien oder die sog. Anamox-Bakterien können noch längere Generationszeiten besitzen. So hat z.B. das Methangasbakterium *Methanosaeta sp.* selbst bei optimalen Wachstumsbedingungen Generationszeiten von 1 - 3 d. Bei suboptimalen Bedingungen können sich diese Generationszeiten auf 20 oder noch mehr Tage verlängern.

Für die Rückführung des abgetrennten magnetisierbaren Aggregats in den Reaktionsraum können alle zur Verfügung stehenden Verfahren eingesetzt werden. Die Rückführung kann kontinuierlich oder portionsweise durchgeführt werden, indem beispielsweise zunächst eine bestimmte Menge an magnetisierbarem Aggregat gesammelt und diese dann in den Reaktionsraum zurückgeführt wird. Die Rückführung kann automatisiert werden. Es ist aber auch möglich, die Rückführung manuell durchzuführen.

Die Vorteile des erfindungsgemäßen Verfahrens zeigen sich insbesondere bei Fermentationen mit langsam wachsenden Mikroorganismen.

Gemäß einer bevorzugten Ausführungsform werden als aktive Komponente des magnetisierbaren Aggregats Mikroorganismen eingesetzt. Die Mikroorganismen sind bevorzugt in Form eines Biofilms auf dem magnetisierbaren Träger bereitgestellt. Ein Biofilm bildet sich während der Anfahrphase des Reaktors auf dem Träger aus. Beim Anfahren des Reaktors erfolgt zunächst eine Besiedelung des magnetisierbaren Trägers durch Mikroorganismen, welche dann durch weiteres Wachstum zunächst einzelne Kolonien ausbilden, welche im weiteren Verlauf zusammenwachsen können und einen kontinuierlichen Biofilm auf dem Träger ausbilden können. Durch die hohe Dichte an Mikroorganismen sind Biofilme, insbesondere wenn sie auf partikulären Trägern angeordnet sind, sehr effiziente Biokatalysatoren für Fermentationen.

Beim erfindungsgemäßen Verfahren wird ein spezieller magnetisierbarer Träger eingesetzt, welcher zumindest in seinem Kern aus einem geschlossenporigen festen Schaum aufgebaut ist. Wie bereits erläutert, können als kontinuierliche Phase des festen Schaums an sich alle Materialien eingesetzt werden, die zur Ausbildung einer kontinuierlichen, glasartigen Netzstruktur befähigt sind. Wegen ihrer hohen mechanischen Stabilität und der meist günstigen Verfügbarkeit der Rohstoffe ist die kontinuierliche Phase des partikulären Trägers aus einem anorganischen Material, nämlich ein Glas, insbesondere ein Silikatglas, gebildet.

Gläser sind in verschiedenen Zusammensetzungen und Qualitäten erhältlich. Da beispielsweise bei Fermentationen jedoch meist keine aggressiven Reaktionsbedingungen herrschen, können günstige Gläser verwendet werden, die beispielsweise einen hohen Gehalt an Alkalimetall, insbesondere Natrium aufweisen. Solche Gläser werden auch als Natrongläser bezeichnet und werden beispielsweise für die Herstellung von Glasbehältern und Flachglas verwendet. Insbesondere kann gemäß einer Ausführungsform der Träger eine kontinuierliche Glasphase umfassen, welche aus Altglas hergestellt ist. Dadurch können im Glas neben den üblichen Bestandteilen, wie $SiO_2$, $Na_2O$, $K_2O$, $CaO$ auch noch andere Bestandteile enthalten sein, die beispielsweise über das Altglas eingetragen werden. Beispielhafte Komponenten sind $B_2O_3$, $Al_2O_3$ oder Eisen- und Manganoxide. Diese weiteren Bestandteile sind vorzugsweise in einem Anteil von weniger als 10 Gew.-%, gemäß einer weiteren Ausführungsform in einem Anteil von weniger als 8 Gew.-%, bezogen auf die Glasphase, in der kontinuierlichen Phase enthalten.

Beim erfindungsgemäßen Verfahren wird das magnetisierbare Aggregat mittels einer magnetischen Abscheidevorrichtung aus der Produktmischung abgetrennt. Um eine für technische Prozesse geeignete Durchführung zu ermöglichen, sollte die Abtrennung innerhalb eines geeignet kurzen Zeitraums erfolgen können. Dazu sollte die magnetische Abscheidevorrichtung eine ausreichend hohe Kraft auf den Träger ausüben können, sodass dieser mit einer ausreichend hohen Geschwindigkeit in der Produktmischung zum gewünschten Ort transportiert werden kann. Gemäß einer Ausführungsform ist vorgesehen, dass der partikuläre Träger eine magnetische Massensuszeptibilität im Bereich von 5 x 10$^{-9}$ bis 3,7 x 10$^{-7}$ m$^3$/kg aufweist.

Um eine Agglomeration des magnetisierbaren Aggregats ohne Einwirkung eines äußeren Magnetfelds zu vermeiden, werden für die magnetisierbaren Bereiche bevorzugt Materialien eingesetzt, welche nach Abschalten eines äußeren Magnetfeldes nur eine geringe Remanenz zeigen. Die Remanenz lässt sich aus der sog. Magnetisierungskurve ablesen. Dabei wird ein Material einem äußeren Magnetfeld ausgesetzt und wird dabei selber mehr oder weniger stark magnetisiert. Durch Absenkung der Feldstärke ausgehend von der magnetischen Sättigung auf den Wert Null bleibt eine Restmagnetisierung übrig. Diese wird in milliTesla (mT) gemessen. Die Remanenz verschwindet erst dann vollständig, wenn eine Magnetkraft, die entgegengesetzt zum ursprünglichen Feld gepolt ist, mit der Feldstärke Hc (Koerzitivfeldstärke) auf das Material einwirkt.

Gemäß einer ersten Ausführungsform werden die magnetisierbaren Bereiche im partikulären magnetisierbaren Träger durch einen superparamagnetischen Stoff gebildet. Ein superparamagnetischer Stoff zeigt nach Abschalten eines äußeren Magnetfelds keine Restmagnetisierung mehr. Ein geeigneter superparamagnetischer Stoff ist beispielsweise Magnetit in Form von Nanopartikeln.

Gemäß einer weiteren Ausführungsform werden die magnetisierbaren Bereiche von einem ferrimagnetischen Material gebildet. Ferrimagnetische Materialien zeigen nach Abschalten eines äußeren Magnetfelds nur eine geringe Remanenz. Geeignete ferrimagnetische Materialien sind beispielsweise Ferrit, Ferrite und Maghemit.

Das erfindungsgemäße Verfahren zeigt seine Vorteile insbesondere, wenn als aktive Komponente des magnetisierbaren Aggregats langsam wachsende Mikroorganismen eingesetzt werden. Anaerobe Mikroorganismen zeigen aus thermodynamischen Gründen nur ein relativ langsames Wachstum. Gemäß einer Ausführungsform wird die aktive Komponente des magnetisierbaren Aggregats daher durch eine Reinkultur einer Mikroorganismenart, durch eine definierte Mischkultur mehrerer Mikrooganismenarten bzw. durch eine gemischte Biozönose von anaerob wachsenden Mikroorganismen gebildet. Gemäß einer Ausführungsform ist dann vorgesehen, dass die Umsetzung der Substratmischung an dem magnetisierbaren Aggregat, welches als aktive Komponente anaerob wachsende Mikroorganismen umfasst, zu einer Produktmischung unter anaeroben Bedingungen durchgeführt wird.

Besonders gute Ergebnisse, das heißt eine hohe Ausbeute an Produkt sowie ein hoher Substratdurchsatz wird erzielt, wenn das abgetrennte magnetisierbare Aggregat, wobei die aktive Komponente durch zumindest einen anaerob wachsenden Mikroorganismus gebildet wird, wieder in den Reaktionsraum zurückgeführt wird, da auf diese Weise bei anaeroben Mikroorganismen die stationäre Konzentration im Reaktor deutlich erhöht werden kann.

Gemäß einer bevorzugten Ausführungsform sind die Mikroorganismen methanogene Bakterien. Beispielhafte methanogene Bakterien sind Methanosaeta concilii, Methanosarcina barkeri, Methanosphaera stadtmanae, Methanobrevibacter ruminantium, Methanobacterium formicicum, Methanococcus voltae, sowie Methanoculleus palmolei. Die Aufzählung soll nicht als abschließend angesehen werden.

Methanogene Bakterien werden beispielsweise zur Biogasproduktion eingesetzt. Gemäß einer besonders bevorzugten Ausführungsform ist das erfindungsgemäße Verfahren daher als Verfahren zur Erzeugung von Biogas ausgebildet.

Wird das erfindungsgemäße Verfahren zur Biogaserzeugung eingesetzt, können an sich bekannte Reaktoren zur Biogaserzeugung herangezogen werden, wobei diese Reaktoren mit einer magnetischen Abscheidevorrichtung versehen werden, mit welcher die auf dem magnetisierbaren Träger immobilisierten Mikroorganismen aus dem Gärrest entfernt werden können. Ein geeigneter Fermenter ist beispielsweise in der DE 10 2005 024 886 B3 oder der WO 2007/093138 A2 beschrieben.

Als organisches Substrat können übliche Materialien verwendet werden, zum Beispiel Bioabfälle, Grünschnitt, Gewerbeabfälle, Lebensmittelabfälle, landwirtschaftliche Abfälle, Küchenabfälle, organische Abfälle oder nachwachsende Rohstoffe und ähnliche Stoffe.

Die Substratmischung kann einen hohen Wassergehalt aufweisen. Gemäß einer Ausführungsform wird der Wassergehalt der Substratmischung im Bereich von 50 bis 99 Gew.-% gewählt, gemäß einer weiteren Ausführungsform im Bereich von 60 bis 95 Ges.-%.

Die Verweildauer des organischen Substrats im Reaktionsraum ist abhängig von der Art des eingesetzten organischen Substrats und den Reaktionsbedingungen im Fermenter und kann zwischen wenigen Stunden, beispielsweise 2 Stunden, und mehreren Tagen, beispielsweise bis zu 40 Tagen, und in manchen Fällen auch bis zu mehreren Monaten, beispielsweise bis zu drei Monaten betragen.

Ist das Verfahren beispielsweise als Verfahren zur Erzeugung von Biogas ausgestaltet, kann gemäß einer Ausführungsform die Verweildauer des organischen Substrats im Reaktionsraum im Bereich von 3 bis 28 Tagen gewählt werden.

Der Durchsatz an organischem Substrat im Reaktionsraum wird gemäß einer Ausführungsform im Bereich von 1 bis 10 kg organische Trockensubstanz/$m^3$ Tag eingestellt. Der Durchsatz an organischem Substrat wird an sich nur durch anlagentechnische Bedingungen begrenzt, sodass auch höhere Durchsätze möglich sind.

Das Verfahren kann in einem einzelnen Reaktor durchgeführt werden. Es ist aber auch möglich, mehrere Reaktoren hintereinander zu schalten und das Verfahren mehrstufig zu betreiben. Die verschiedenen Stufen des Verfahrens können dann auf bestimmte Parameter hin optimiert werden. So ist bei der Biogaserzeugung eine Ausführungsform möglich, bei welcher das organische Substrat in einer ersten Reaktionsstufe aufgeschlossen wird und das aufgeschlossene organische Substrat dann in einer zweiten Stufe vergoren wird, wobei Biogas erzeugt wird.

Das bei der Biogaserzeugung anfallende Fermentationsgas enthält im Wesentlichen Kohlendioxid, Methan, Wasserstoff und Schwefelwasserstoff. Sauerstoff wird, wenn er in den Reaktionsraum gelangt, zügig verbraucht.

Wird das erfindungsgemäße Verfahren als Fermentationsverfahren zur Erzeugung von Biogas ausgestaltet, wird die Prozesstemperatur im Reaktionsraum gemäß einer Ausführungsform zwischen 30 und 70 °C, gemäß einer weiteren Ausführungsform im Bereich von 35 bis 42 °C oder 50 bis 65 °C gewählt. In diesen Bereichen haben mesophile bzw. thermophile methanogene Bakterien ihren Präferenzbereich.

Der pH-Wert im Reaktionsraum kann lokal zwischen 3,5 und 8,5 liegen. Um eine hohe Rate an Methanbildung zu erreichen, ist es günstig, bei der Biogasproduktion im Reaktionsraum einen neutralen pH-Wert einzustellen. Bevorzugt wird der pH-Wert der Substratmischung im Reaktionsraum in einem Bereich von 6,5 bis 8,0 eingestellt.

Das C/N-Verhältnis des bei der Biogaserzeugung eingesetzten organischen Substrats wird gemäß einer Ausführungsform größer als 20:1, gemäß einer Ausführungsform größer als 30:1 und gemäß einer weiteren Ausführungsform größer als 40:1 gewählt.

Das organische Substrat kann mit üblichen Vorrichtungen in den Reaktionsraum eingebracht werden. Beispielsweise kann das organische Substrat mit einer flüssigkeitsdichten Fördereinrichtung in den Reaktionsraum eingebracht werden. Hierbei kann das organische Substrat beispielsweise mit einer flüssigkeitsdichten Förderschnecke aus einem Substratspeicher durch einen Kanal in den Reaktionsraum eingebracht werden. Die Zuführung des organischen Substrats kann aber auch mit nicht flüssigkeitsdichten Fördereinrichtungen erfolgen.

Um das magnetisierbare Aggregat aus der Produktmischung, die im Fall einer Biogaserzeugung durch einen Gärrest gebildet wird, möglichst vollständig abtrennen zu können, ist bevorzugt, dass die Produktmischung einen hohen Wassergehalt aufweist. Dies ermöglicht eine ausreichende Beweglichkeit des magnetisierbaren Aggregats in der Produktmischung und damit eine rasche Abscheidung. Bevorzugt weist die Produktmischung einen Wassergehalt von mehr als 50 Gew.-%, gemäß einer Ausführungsform einen Wassergehalt im Bereich von 70 bis 99 Gew.-% auf. Ggf. kann die aus dem Reaktionsraum ausgeschleuste Produktmischung mit Wasser verdünnt werden.

Die Produktmischung kann mit üblichen Methoden und Vorrichtungen aus dem Reaktionsraum ausgeschleust werden. Eine geeignete Vorrichtung ist beispielsweise eine flüssigkeitsdichte Förderschnecke. Nach Abtrennung des magnetisierbaren Aggregats kann die verbliebene Produktmischung abgepresst und die flüssige Phase wieder ganz oder teilweise zurück in den Reaktionsraum geführt werden. Dadurch kann die stationäre Konzentration an Mikroorganismen weiter erhöht werden.

Die Fermentationsgase, welche bei der Biogasproduktion entstehen, können am Kopf des Reaktionsraums abgezogen werden. Anschließend kann das Biogas in üblicher Weise verarbeitet werden, beispielsweise durch Abtrennung schwefelhaltiger Verbindungen.

Um den Aufschluss des organischen Substrats zu erleichtern, können beispielsweise Enzyme zur Substratmischung gegeben werden. Beispielhafte Enzyme sind Cellulasen, Hemicellulasen, Lipasen, Proteasen und/oder Amylasen.

Bei der Biogaserzeugung werden bevorzugt kostengünstige Materialien eingesetzt. Als Säurepuffer können zum Beispiel Puffer auf Kalk- oder Ammoniumbasis verwendet werden.

Als Spurenelemente können beispielsweise Nickel, Kobalt, Molybdän, Selen und/oder Wolfram zur Substratmischung gegeben werden. Insbesondere Methanbakterien haben einen hohen Bedarf an Spurenelementen.

Das im erfindungsgemäßen Verfahren eingesetzte magnetisierbare Aggregat kann in vielfältigen Anwendungen eingesetzt werden. Es kann, wie oben geschildert, verwendet werden, um ein biokatalytisch aktives System zu immobilisieren, beispielsweise ein Enzym oder einen Mikroorganismus, wie z.B. eine Reinkultur, eine definierte Mischkultur oder eine Mischbiozönose von Bakterienarten, Pilzarten oder Zellkultur-Zellen. Das magnetisierbare Aggregat kann aber auch eingesetzt werden, um beispielsweise bestimmte Substanzen aus einem Gemisch abzutrennen, indem die betreffende Verbindung durch die aktive Komponente am magnetisierbaren Aggregat adsorbiert wird. Dabei kann es sich um eine direkte Adsorption handeln oder um eine indirekte Bindung. Der magnetisierbare partikuläre Träger kann vorab z.B. mit folgenden Substanzen als aktive Komponente beschichtet worden sein: Streptavidin, Avidin, Protein A, Immunglobuline (IgGs) oder die Partikeloberfläche kann zuvor mit -$NH_2$- bzw. -COOH-Gruppen modifiziert worden sein. Ebenso können Gensonden oder andere Fänger-Moleküle an der Partikeloberfläche angebracht worden sein.

Gegenstand der Erfindung ist daher auch ein magnetisierbares Aggregat, umfassend einen partikulären magnetisierbaren Träger aus einem festen Schaum mit einer kontinuierlichen Phase, welche vorzugsweise aus einem Netzbildner aufgebaut ist und welche Poren des festen Schaums umgibt, wobei in der kontinuierlichen Phase magnetisierbare Bereiche angeordnet sind, wobei zumindest im Kern des magnetisierbaren Trägers der Schaum geschlossenporig ist, und wobei weiter auf der Oberfläche des partikulären magnetisierbaren Trägers zumindest eine aktive Komponente immobilisiert ist.

Die Eigenschaften und die Struktur des partikulären magnetisierbaren Trägers wurden zum Teil bereits weiter oben bei der Beschreibung des Verfahrens erläutert.

Der partikuläre magnetisierbare Träger besteht aus einem festen Schaum, bei welchem die Poren des festen Schaums von einer kontinuierlichen Phase umgeben sind. Die kontinuierliche Phase wird aus einem Netzbildner gebildet. Der Netzbildner wird durch ein Glas, insbesondere ein silikatisches Glas bereitgestellt. Als Glas kann beispielsweise Quarzglas, Borglas oder auch ein aluminiumoxidhaltiges Glas verwendet werden. Insbesondere für eine Verwendung in biotechnologischen Systemen, beispielsweise der oben beschriebenen Fermentation, können jedoch relativ kostengünstige Gläser eingesetzt werden, beispielsweise Natronglas oder vermahlenes Altglas. Die Gläser enthalten als Hauptbestandteil bevorzugt Siliziumdioxid. Ein beispielhaftes Glas weist eine Zusammensetzung von 68,5 bis 75 Gew.-% $SiO_2$, 10 bis 14 Gew.-% $Na_2O$, 6 bis 11 Gew.-% CaO auf. Als weitere Bestandteile können beispielsweise $Al_2O_3$ in einem Anteil von 1,5 bis 3 Gew.-%, $K_2O$ in einem Anteil bis zu 2,5 Gew.-%, MgO in einem Anteil von 0,5 bis 4 Gew.-%, sowie BaO in einem Anteil von bis zu 3 Gew.-% enthalten sein. Weitere Bestandteile, die beispielsweise als Verunreinigungen durch das Altglas eingeschleppt werden können, sind gemäß einer Ausführungsform in einem Anteil von weniger als 2 Gew.-%, gemäß einer weiteren Ausführungsform in einem Anteil von weniger als 1 Gew.-% enthalten. Solche Verunreinigungen sind beispielsweise Eisenoxid oder Titanoxid. Der Anteil des Glases bzw. der kontinuierlichen Phase am partikulären magnetisierbaren Träger wird bevorzugt im Bereich von 70 bis 85 Gew.-% gewählt. Der Anteil der magnetisierbaren Partikel, aus welchen die magnetisierbaren Bereiche des partikulären Trägers gebildet werden, ist bevorzugt im Bereich von 15 bis 30 Gew.-% gewählt.

Der magnetisierbare Träger weist einen Kern aus einem geschlossenporigen Schaum auf. Der Kern nimmt vorzugsweise zumindest die Hälfte, gemäß einer weiteren Ausführungsform zumindest zwei Drittel des gesamten Volumens des magnetisierbaren Trägers ein. Der Kern kann zentral im Mittelpunkt des magnetisierbaren Trägers angeordnet sein. Es ist aber auch möglich, dass der Mittelpunkt des Kerns relativ zum Mittelpunkt des magnetisierbaren Trägers verschoben ist.

Der partikuläre Träger weist gemäß einer Ausführungsform an seiner Oberfläche eine durchgehende Haut auf, sodass die im Inneren des partikulären Trägers angeordneten Poren abgeschlossen sind.

Der partikuläre Träger weist vorzugsweise eine niedrige Wasseraufnahmefähigkeit auf. Das Wasseraufnahmevermögen des partikulären Trägers, bestimmt gemäß DIN EN 13055 / DIN V 18004 5.3 und zusätzlich EN1097-6:2000 beträgt vorzugsweise 0 bis 50 Gew.-%, gemäß einer Ausführungsform weniger als 35 Gew.-% und gemäß einer weiteren Ausführungsform weniger als 10 Ges.-%.

Der erfindungsgemäße partikuläre Träger liegt gemäß einer Ausführungsform in Form annähernd sphärischer Partikel vor. Angaben zum Durchmesser des magnetisierbaren Trögers wurden bereits weiter oben gemacht. Gemäß einer weiteren Ausführungsform liegt der Durchmesser des Trägers, gemessen als mittlere Korngröße, im Bereich von 0,5 $\mu$m bis 5 mm, gemäß einer Ausführungsform im Bereich von 100 $\mu$m bis 2 mm. Unter einer mittleren Korngröße wird die volumenbezogene Korngröße $D_{50}$ verstanden, wobei 50 % der Partikel einen größeren Durchmesser als $D_{50}$ und 50 % der Partikel einen kleineren Durchmesser als $D_{50}$ aufweisen. Die mittlere Korngröße $D_{50}$ lässt sich beispielsweise durch Siebanalyse ermitteln. Geeignete Verfahren sind beispielsweise in der DIN 66165-1 und -2 angegeben. Alternativ kann die mittlere Korngröße mittels Laserdiffraktion gemessen werden. Geeignete Verfahren sind in der ISO 13320:2009 beschrieben.

Der partikuläre Träger weist vorzugsweise zumindest abschnittsweise eine unregelmäßig geformte Oberfläche auf. Dies erleichtert es Mikroorganismen, auf der Oberfläche des partikulären magnetisierbaren Trägers anzuwachsen. Eine derartige unregelmäßige Oberfläche kann beispielsweise erzeugt werden, indem der partikuläre Träger nach dem Aufschäumen noch vorsichtig vermahlen wird, sodass der geschäumte magnetisierbare Träger gebrochen wird. An den Bruchflächen werden dann aufgebrochene Poren erhalten. Bei einer solchen Ausführungsform weist der partikuläre magnetisierbare Träger auf zumindest Abschnitten seiner Oberfläche scharfkantige Grate sowie Vertiefungen auf, die aus den aufgebrochenen Poren entstehen. Eine solche Struktur lässt sich beispielsweise mit Hilfe eines Auflichtmikroskops oder eines Rasterelektronenmikroskops feststellen.

Der im erfindungsgemäßen magnetisierbaren Aggregat enthaltene partikuläre magnetisierbare Träger weist vorzugsweise eine Schüttdichte im Bereich von 100 bis 1000 kg/m$^3$, gemäß einer Ausführungsform eine Schüttdichte im Bereich von 200 bis 800 kg/m$^3$ und gemäß einer weiteren Ausführungsform eine Schüttdichte von 300 bis 600 kg/m$^3$ auf. Die Schüttdichte kann nach DIN EN 13055-1 ermittelt werden.

Gemäß einer weiteren bevorzugten Ausführungsform weist der partikuläre Träger eine Kornrohdichte von weniger als 2000 kg/m$^3$, vorzugsweise eine Kornrohdichte im Bereich 300 bis 1500 kg/ m$^3$ und gemäß einer weiteren Ausführungsform eine Kornrohdichte im Bereich von 600 bis 1000 kg/ m$^3$ auf. Die Kornrohdichte ist der Quotient aus der Masse des trockenen partikulären magnetisierbaren Trägers und dem Volumen des wassergesättigten partikulären Trägers. Sie wird nach DIN EN 13055-1 sowie DIN V 18004,5.2 bestimmt.

Wird die Kornrohdichte in diesem Bereich eingestellt, kann vorteilhaft ein Sedimentieren bzw. ein Aufschwimmen des partikulären magnetisierbaren Trägers, welcher ggf. mit einem auf diesem immobilisierten Biofilm versehen ist, wirksam zurückgedrängt werden.

Gemäß einer Ausführungsform weist der erfindungsgemäße Träger eine Transformationstemperatur von weniger als 800 °C, insbesondere einen Bereich von 500 bis 750 °C auf. Die Transformationstemperatur ist die Temperatur, bei der das Glas während der Abkühlung aus dem plastischen Bereich in den starren Zustand übergeht.

In die aus dem Netzbildner gebildete kontinuierliche Phase des partikulären magnetisierbaren Trägers sind kleine, vorzugsweise überwiegend kristalline Partikel eines magnetisierbaren Materials eingeschlossen. Die magnetisierbaren Partikel weisen vorzugsweise einen Durchmesser von weniger als 500 $\mu$m, gemäß einer Ausführungsform einen Durchmesser im Bereich 1 nm bis 400 $\mu$m auf, gemäß einer weiteren Ausführungsform einen Durchmesser im Bereich 10 nm bis 100 $\mu$m auf.

Die magnetisierbaren Partikel sind bevorzugt homogen in der kontinuierlichen Phase des festen Schaums verteilt.

Gemäß einer weiteren Ausführungsform werden sehr kleine Partikel des magnetisierbaren Materials verwendet. Durch die Verwendung sehr kleiner Partikel mit einem Durchmesser von weniger als 500 nm, gemäß einer Ausführungsform von weniger als 100 nm, gemäß einer weiteren Ausführungsform von weniger als 10 nm, lassen sich beispielsweise auch superparamagnetische Teilchen erzeugen. Gemäß einer Ausführungsform werden Nanopartikel für die Bereitstellung der magnetisierbaren Bereiche eingesetzt. Die Partikel für die magnetisierbaren Bereiche weisen bei dieser Ausführungsform bevorzugt einen Partikeldurchmesser im Bereich von 1 nm bis 10 nm auf.

Die Angaben zur Korngröße beziehen sich auf den mittleren Korngrößendurchmesser $D_{50}$. An sich hat die Breite der Korngrößenverteilung keinen bedeutenden Einfluss auf die Eigenschaften des magnetisierbaren Trägers bzw. des magnetisierbaren Aggregats. Bevorzugt wird die Korngrößenverteilung so eingestellt, dass die magnetisierbaren Partikel vollständig von der kontinuierlichen Phase des magnetisierbaren Trägers eingeschlossen sind.

Die Korngrößenverteilung lässt sich für größere Partikel (> 100 $\mu$m) beispielsweise mittels Siebanalyse ermitteln. Bei kleineren Partikeln kann der mittlere Partikeldurchmesser durch Laserdiffraktometrie gemessen werden. Für die Bestimmung des Partikeldurchmessers wird davon ausgegangen, dass sich die Partikelgröße der magnetisierbaren Partikel beim Aufschäumen des Granulats nicht mehr ändert.

Bevorzugt sind die Partikel des magnetisierbaren Materials vollständig in der kontinuierlichen Phase eingeschlossen, sodass Korrosion der magnetisierbaren Partikel sowie Wechselwirkungen zwischen den magnetisierbaren Partikeln und einem Umgebungsmedium, beispielsweise einem Biofilm, zurückgedrängt werden.

Als magnetisierbare Materialien werden gemäß einer Ausführungsform superparamagnetische Metalloxide oder ferrimagnetische Metalloxide verwendet. Geeignete Materialien für die magnetisierbaren Partikel sind beispielsweise Magnetit, Maghemit und Ferrit.

Bei ferrimagnetischen Materialien, die nach Abschalten eines äußeren Magnetfeldes ihre Magnetisierung behalten, handelt es sich um magnetisch harte Materialien. Ferrimagnetismus ist auf atomarer Ebene durch zwei unterschiedlich große entgegengesetzte Magnetvektoren gekennzeichnet, die aus der Spinellstruktur dieser oxidischen Materialien herrührt.

Sowohl Magnetit ($Fe_3O_4$), als auch $\gamma$-$Fe_2O_3$ sind ferrimagnetische Materialien. Von ferromagnetischen Materialien können diese durch die Spinellstruktur und den oxidischen Chemismus abgegrenzt werden.

Besonders bevorzugt werden weiche ferrimagnetische Materialien für die Herstellung des partikulären magnetisierbaren Trägers verwendet, da diese nach Abschalten des Magnetfeldes ihre Magnetisierung weitgehend verlieren und damit keine Agglomerate bilden.

Gemäß einer weiteren Ausführungsform werden superparamagnetische Materialien, wie beispielsweise $\gamma$-$Fe_2O_3$, das bis in den Nanobereich hinab vermahlen wurde, verwendet. Superparamagnetische Materialien zeigen keine Hysterese mehr.

Erfindungsgemäß ist auf dem partikulären magnetisierbaren Träger eine aktive Komponente immobilisiert. Was unter einer aktiven Komponente verstanden wird, wurde bereits weiter oben erläutert.

Gemäß einer bevorzugten Ausführungsform ist die aktive Komponente ein biokatalytisch aktives System. Ein solches biokatalytisch aktives System kann beispielsweise ein Enzym, ein Mikroorganismus oder eine Zellkulturzelle sein oder auch durch Bestandteile von Zellen, beispielsweise Vesikel, gebildet werden.

Geeignete Mikroorganismen können beispielsweise Eubakterien, Archaeobakterien oder Pilze sein. Die Mikroorganismen können jeweils als Reinkulturen, definierte Mischkulturen oder Mischbiozönosen bereitgestellt werden. Es kann sich auch um Zellkulturzellen handeln. Gemäß einer besonders bevorzugten Ausführungsform sind die Mikroorganismen in Form eines Biofilms auf dem partikulären magnetisierbaren Trägermaterial immobilisiert. Insbesondere bevorzugt umfasst der Biofilm mehrere Mikroorganismenarten.

Gemäß einer weiteren Ausführungsform können auf der Oberfläche des magnetisierbaren Trägers als aktive Komponente des magnetisierbaren Aggregats Fängermoleküle angeordnet sein. Unter einem Fängermolekül werden Verbindungen verstanden, welche andere Verbindungen, welche beispielsweise Gruppen einer bestimmten Struktur aufweisen, selektiv adsorbieren können. Fängermoleküle können dabei Verbindungen sein, welche ein relativ niedriges Molekülgewicht aufweisen, bevorzugt ein Molekülgewicht von weniger als 100.000 g/mol, gemäß einer weiteren Ausführungsform von weniger als 10.000 g/mol, gemäß einer weiteren Ausführungsform von weniger als 5.000 g/mol, gemäß noch einer weiteren Ausführungsform von weniger als 1.000 g/mol. Beispielhafte Fängermoleküle mit einem relativ niedrigen Molekülgewicht sind beispielsweise DNA-Sonden oder RNA-Sonden.

Als Fängermolekül können jedoch auch Verbindungen oder Aggregate mit einem höheren Molekülgewicht eingesetzt werden. Geeignet sind beispielsweise polymere Moleküle mit Gruppen, welche ionische oder dipolare Bindungen ausbilden können. Ein beispielhaftes Polymer ist Polyethylenimin.

Als Fängermolekül können aber auch Peptide oder Proteine mit höherem Molekülgewicht verwendet werden, beispielsweise Antikörper, die mit üblichen Methoden gegen ein bestimmtes Antigen erzeugt worden sind.

Die Bindung zwischen dem Fängermolekül und dem adsorbierten organischen /und oder anorganischen Substrat kann beispielsweise durch ionische oder dipolare Wechselwirkungen erfolgen. Am Fängermolekül können dazu geeignete Affinitätsgruppen vorgesehen sein, die ionische oder dipolare Bindungen ausbilden können. Beispielhafte Affinitätsgruppen sind Aminogruppen, Hydroxygruppen, Carboxygruppen, Carbamidgruppen oder auch heteroaromatische Gruppen.

Als Fängermolekül sind beispielsweise geeignet: DNA-Fängersonden, RNA-Fängersonden, Antikörper (IgG), Protein A, Avidin, Streptavidin oder Proteine mit langen Histidinketten.

Die Fängermoleküle sind geeignet, beispielsweise DNA, RNA, Aminosäuren, Peptide, Proteine, Kohlenhydrate, Fette oder komplex aufgebaute Substanzen wie z.B. Lipoproteine zu adsorbieren. Solche Verbindungen können dann mit dem erfindungsgemäßen magnetisierbaren Aggregat beispielsweise aus biologischen Flüssigkeiten angereichert werden. Ebenso kann der magnetisierbare Träger, welcher mit geeigneten Fängermolekülen oberflächenmodifiziert wurde, dazu verwendet werden, selektiv bestimmte Verbindungen, beispielsweise Proteine, Peptide, Antibiotika, pharmazeutisch wirksame Substanzen, spezifische Ziel-DNA-Moleküle, spezifische Ziel-RNA-Moleküle, Signalsubstanzen, Hormone, Wachstumsfaktoren etc. aus biotechnologisch hergestellten Produktmischungen abzutrennen.

Gemäß einem weiteren Aspekt betrifft die Erfindung ein Verfahren zur Herstellung eines solchen magnetisierbaren Aggregats, wobei ein Netzbildner, ein magnetisierbares Material sowie ein Blähmittel zu einem Granulat verarbeitet werden, das Granulat zu einem partikulären magnetisierbaren Träger gebläht wird und auf dem partikulären magnetisierbaren Träger eine aktive Komponente immobilisiert wird.

Die Herstellung des partikulären magnetisierbaren Trägers erfolgt an sich nach Verfahren wie sie bereits für die Herstellung von Schaumglasgranulat bekannt sind. Im Wesentlichen kann bei der Herstellung des partikulären magnetisierbaren Trägers Verfahren gefolgt werden, wie sie beispielhaft in den Druckschriften DE 10 2004 012 598 A1, EP 2 022 768 A2 und WO 2006/092153 A1 beschrieben sind, wobei zusätzlich magnetisierbare Partikel eingearbeitet werden.

Überraschend hat sich gezeigt, dass trotz der beim Schäumen des Granulats herrschenden Temperaturen sowie Prozessschritten, wie beispielsweise einem Vermahlen der Ausgangsprodukte, die magnetischen Eigenschaften des magnetisierbaren Materials erhalten bleiben, sodass auch nach dem Schäumen der partikuläre Träger magnetische Eigenschaften aufweist und beispielsweise in einem Magnetfeld angezogen wird.

Als Ausgangsmaterial wird vorzugsweise von einem Netzbildner, insbesondere Glasmehl, und einem magnetisierbaren Material, beispielsweise einem Magnetpigment ausgegangen, die in Form von Pulvern vorgelegt werden. Bei Verwendung von beispielsweise Glasmehl als Netzbildner können das Glasmehl bzw. das magnetisierbare Material entsprechend fein vermahlen werden. Das Vermahlen der Komponenten kann einzeln oder gemeinsam erfolgen. Nach dem Vermahlen weist das Glasmehl und ggf. das magnetisierbare Material bevorzugt eine mittlere Partikelgröße $D_{50}$ von weniger als 500 μm, gemäß einer Ausführungsform einen Durchmesser im Bereich von 1 nm bis 400 μm, gemäß einer weiteren Ausführungsform einen Durchmesser im Bereich von 10 nm bis 100 μm auf.

Bevorzugt wird zumindest ein Teil des Glasmehls nass vermahlen. Das magnetisierbare Material kann trocken oder nass vermahlen werden. Dies ermöglicht es, sehr kleine Partikelgrößen zu erhalten. Nach dem Vermahlen weisen die Partikel des Glasmehls bzw. das magnetisierbare Material bevorzugt einen mittleren Durchmesser $D_{50}$ im Bereich von 1 nm bis 200 μm, gemäß einer Ausführungsform in einem Bereich von 10 nm bis 100 μm auf.

Das Vermahlen des Glasmehls und des magnetisierbaren Materials kann gemeinsam oder getrennt erfolgen.

Bevorzugt wird das Glas, gegebenenfalls unter Zusatz des magnetisierbaren Materials, über mehrere Stunden hinweg nassvermahlen. Es kann die gesamte Menge des Glases oder nur ein Anteil des Glases fein vermahlen werden. Wird nur ein Teil des Glases fein vermahlen, wird das feinvermahlene Glasmehl vor dem Granulieren mit gröber vermahlenem Glasmehl vermischt. Durch das Nassvermahlen wird die Oberfläche des Glases aufgeschlossen, was einen besseren mechanischen Zusammenhalt des geblähten Granulats ermöglicht. Vorzugsweise wird das Nassvermahlen über einen Zeitraum von mindestens 3 Stunden, vorzugsweise 4 bis 8 Stunden vorgenommen.

Das magnetisierbare Material kann dem Glasmehl bereits vor dem Nassvermahlen zugesetzt werden. Es ist aber auch möglich, zunächst das Glasmehl bis zu einer bestimmten Partikelgröße vorzumahlen, dann das, ggf. ebenfalls vorvermahlene, magnetisierbare Material zuzusetzen und dann das Gemisch bis zur gewünschten Feinheit weiter zu vermahlen.

Gemäß einer Ausführungsform des Verfahrens kann dem Glasmehl während des Nassvermahlens Wasserglas zugesetzt werden. Dadurch wird die Oberfläche des Glasmehls weiter angelöst, sodass eine bessere Verbindung der Glaspartikel und damit eine Erhöhung der mechanischen Stabilität des Granulats erreicht werden.

Glasmehl und magnetisierbares Materiale werden vorzugsweise in einem Anteil von 60 bis 90 Gew.-% Glasmehl sowie 10 bis 40 Gew.-% magnetisierbares Material, gemäß einer weiteren Ausführungsform in einem Anteil von 70 bis 85 Gew.-% Glasmehl sowie 15 bis 30 Gew.-% magnetisierbares Material, sowie gemäß noch einer weiteren Ausführungsform in einem Anteil von 75 bis 82 Gew.-% Glasmehl sowie 18 bis 25 Gew.-% magnetisierbares Material eingesetzt, bezogen auf das Gewicht der Mischung aus Glasmehl und magnetisierbarem Material.

Der Mischung aus Glasmehl und magnetisierbarem Material werden vorzugsweise ein Blähmittel sowie gegebenenfalls ein Bindemittel zugegeben. Geeignete Blähmittel sind beispielsweise Zucker, Braunstein, Soda, Natriumnitrat oder Siliziumnitrid. Die Menge des Blähmittels wird entsprechend zum gewünschten Grad des Aufschäumens gewählt. Als Bindemittel können anorganische Bindemittel, zum Beispiel Wasserglas, und/oder organische Bindemittel, zum Beispiel Zucker oder Harze verwendet werden. Durch das Bindemittel kann die mechanische Stabilität des ungeschäumten Granulats erhöht werden.

Diese Mischung wird, ggf. nach Zusatz von gröber vermahlenem Glasmehl, dann mit üblichen Verfahren zu einem Granulat verarbeitet. Dazu kann die Mischung beispielsweise in einem Rührer oder auf einem Granulierteller granuliert werden. Es ist aber auch möglich die Mischung in einem Sprühtrockner zu einem feinen Granulat zu verarbeiten, welches dann, beispielsweise unter Zugabe von Wasserglas, zu einem gröberen Granulat verarbeitet wird. Das Granulat wird gegebenenfalls noch getrocknet und anschließend gebläht.

Das Blähen erfolgt vorzugsweise bei Temperaturen von weniger als 1.000 °C, gemäß einer Ausführungsform bei einer Temperatur von weniger als 900 °C und gemäß noch einer weiteren Ausführungsform in einem Temperaturbereich von 550 bis 800°C. Das Granulat kann mit üblichen Verfahren gebläht werden. Vorzugsweise wird das Granulat in einem Drehrohrofen gebläht. Um ein Agglomerieren des Granulats beim Blähen zu verhindern, kann das ungeblähte Granulat gegebenenfalls vorher mit einem geeigneten Trennmittel, beispielsweise mit einer Schicht aus Talkum, gepudert werden. Die Dauer für das Blähen wird gemäß einer Ausführungsform im Bereich von 5 Minuten bis 45 Minuten gewählt.

Das Aufschäumen des Granulats wird vorzugsweise in der Weise durchgeführt, dass der partikuläre magnetisierbare Träger eine Dichte von weniger als 1,2, vorzugsweise im Bereich von 0,8 bis 1,1 kg/l aufweist. Die Dichte des erhaltenen magnetisierbaren partikulären Trägers kann durch die Reaktionsbedingungen oder auch durch beispielsweise den Anteil an Blähmittel oder Wasser im ungeschäumten Granulat eingestellt werden.

Als magnetisierbares Material können superparamagnetische oder ferrimagnetische Materialen eingesetzt werden, beispielsweise Magnetit ($Fe_3O_4$) oder $\gamma\text{-}Fe_2O_3$. Ein Beispiel für superparamagnetische Teilchen ist Nano-$\gamma\text{-}Fe_2O_3$.

Gemäß einer Ausführungsform wird die Herstellung des partikulären magnetisierbaren Trägers unter Inertgas durchgeführt, wobei insbesondere bevorzugt das Aufschäumen unter Inertgas erfolgt. Dies schützt das magnetisierbare Material, beispielsweise Magnetit, vor Oxidation, durch welche dessen magnetische Eigenschaften verlorengehen würden. Wird $\gamma\text{-}Fe_2O_3$ als magnetisierbares Material verwendet, wurde überraschend gefunden, dass eine Schutzgasatmosphäre nicht notwendig ist, um noch signifikante Magnetisierbarkeit des partikulären Trägers zu erreichen. Dies ist umso überraschender, da reines $\gamma\text{-}Fe_2O_3$ bei einer Temperatur von mehr als 300 °C in $\alpha\text{-}Fe_2O_3$ umgewandelt wird, wodurch der Ferromagnetismus verloren geht.

Gemäß einer Ausführungsform des erfindungsgemäßen Verfahrens wird in der folgenden Weise vorgegangen:

- Vermischen von Glasmehl und magnetisierbarem Material, welches vorzugsweise ein oder mehrere ferrimagnetische Materialien umfasst,

- Dispergieren der Mischung zu einer ersten homogenen Vormischung,

- Vermischen zumindest eines Teils dieser ersten Vormischung mit Blähmittel, gegebenenfalls Bindemittel und Wasser zu einem homogenen Schlicker,

- Granulieren des Schlickers unter Verwendung des gegebenenfalls verbleibenden Restes der ersten Vormischung zu magnetischen Blähglas-Granulat-Grünkörpern, und

- Verschäumen der Blähglas-Granulat-Grünkörper zu magnetischen Blähglas-Granulat-Partikeln bei Temperaturen von 750 °C bis 1000 °C.

Gemäß einer bevorzugten Ausführungsform wird die Mischung aus Glasmehl und magnetisierbarem Material nass dispergiert, um eine erste homogene Vormischung zu erhalten. Insbesondere bevorzugt wird die Mischung aus Glasmehl und magnetisierbarem Material nass vermahlen, sodass eine sehr kleine Partikelgröße erhalten wird und die Oberfläche des Glases aufgeschlossen wird. Für das Nassvermahlen können übliche Mühlen verwendet werden, beispielsweise Kugelmühlen.

Gemäß einer bevorzugten Ausführungsform wird bei der Herstellung der ersten Vormischung das Glasmehl in einem Anteil von 65 bis 92 Gew.-% und das magnetisierbare Material in einem Anteil von 8 bis 35 Gew.-% eingesetzt, bezogen auf die Gesamtmenge an Glasmehl und magnetisierbarem Material. Eine besonders bevorzugte Vormischung enthält etwa 80 Gew.-% Glasmehl und 20 Gew.-% magnetisierbares Material.

Die Anteilsbereiche der Komponenten der zweiten Vormischung liegen vorzugsweise im Bereich zwischen 30 und 99 Gew.-% für Wasserglas, 0 bis 70 Gew.-% für Wasser und 1 bis 10 Gew.-% Blähmittel, bevorzugt in Form von Natronsalpeter. Besonders bevorzugte Bereiche sind 54 bis 56 Gew.-% für Wasserglas, 43 bis 44 Gew.-% für Wasser und 2 bis 2,5 Gew.-% für das Blähmittel.

Als Wasserglas wird vorzugsweise ein Wasserglas mit einem $SiO_2/Na_2O$ Verhältnis von 1,1 bis 4 eingesetzt. Das Wasserglas hat vorzugsweise einen Feststoffgehalt im Bereich von 40 bis 100 Gew.-%.

Das Massenverhältnis zwischen der ersten und zweiten Vormischung wird vorzugsweise im Bereich von 2:1 bis 4:1, weiter bevorzugt 2,7:1 bis 3,0:1 gewählt.

Vorzugsweise besitzen innerhalb des ansonsten zumindest überwiegend amorphen Glasgefüges die kristallinen magnetisierbaren Bereiche des partikulären magnetisierbaren Trägers folgende Basis-Phasenzusammensetzungen:

- 25 bis 100 Masse-% $\gamma$-$Fe_2O_3$ und $Fe_3O_4$ in Summe, und
- 0 bis 75 Masse-% $\alpha$-$Fe_2O_3$.

Besonders bevorzugt sind folgende Anteilsbereiche der kristallinen Bestandteile insgesamt:

- 0 bis 100, vorzugsweise 44 Masse-% $\gamma$-$Fe_2O_3$ (Maghemit)
- 0 bis 100, vorzugsweise 34 Masse-% $Fe_3O_4$ (Magnetit)
- 0 bis 50, vorzugsweise 21 Masse-% $\alpha$-$Fe_2O_3$ (Hematit)

In an sich bekannter Weise kann das Granulat vor dem Verschäumen getrocknet und klassiergesiebt werden.

Gemäß einer Ausführungsform kann der partikuläre magnetisierbare Träger auch noch mit einer Beschichtung versehen werden, welche beispielsweise das Wachstum von Mikroorganismen befördert. Eine solche Beschichtung kann beispielsweise aus Xanthan gebildet sein. Bevorzugt werden für die Beschichtung Verbindungen verwendet, welche beispielsweise das Anhaften von Zellen oder Mikroorganismen auf dem partikulären magnetisierbaren Träger erleichtern oder die als Nähr- oder Wachstumsstoffe wirken. Geeignet sind beispielsweise Spurenelemente.

Die Erfindung wird im Weiteren anhand von Beispielen sowie unter Bezugnahme auf die beigefügten Figuren näher erläutert. Dabei zeigt:

Fig. 1:   eine schematische Darstellung eines Schnitts durch verschiedene Ausführungsformen eines magnetisierbaren Trägers;

Fig. 2:   eine schematische Darstellung von verschiedenen Stadien, welche bei der Ausbildung eines Biofilms auf einem magnetisierbaren Träger durchlaufen werden;

Fig. 3: eine schematische Darstellung verschiedener Ausführungsformen des erfindungsgemäßen magnetisierbaren Aggregats;

Fig. 4: eine schematische Darstellung einer Versuchsanordnung zur Untersuchung der Abscheidung von magnetisierbaren Trägerpartikeln aus einer Modellflüssigkeit;

Fig. 5: ein Diagramm, in welchem die Scheinviskosität $\nu$ verschiedener Modellflüssigkeiten in Abhängigkeit von der Schergeschwindigkeit $\gamma_s$ aufgetragen ist;

Fig. 6: ein Diagramm, in welchem der Abscheidegrad A von magnetisierbaren Trägerpartikeln in Abhängigkeit von der Häufigkeit N der Durchleitung durch eine magnetische Abscheidevorrichtung dargestellt ist, wobei als Modellflüssigkeit Wasser verwendet wurde;

Fig. 7: ein Diagramm, in welchem der Abscheidegrad A von magnetisierbaren Trägerpartikeln in Abhängigkeit von der Häufigkeit N der Durchleitung durch eine magnetische Abscheidevorrichtung dargestellt ist, wobei als Modellflüssigkeit eine 0,25 %-ige Lösung von Xanthan in Wasser verwendet wurde;

Fig. 8: ein Diagramm, in welchem der Abscheidegrad A von magnetisierbaren Trägerpartikeln bei einmaligem Durchleiten durch eine magnetische Abscheidevorrichtung dargestellt ist, wobei der Anteil der magnetisierbaren Trägerpartikel in der Probe sowie die Viskosität der Modellflüssigkeit variiert wurden;

Fig. 9: ein Diagramm, in welchem die relative magnetische Suszeptibilität $\chi_r$ der magnetisierbaren Trägerpartikel dargestellt ist, wobei nur die in der magnetischen Abscheidevorrichtung abgeschiedenen Trägerpartikel vermessen wurden;

Fig. 10: ein Diagramm, in welchem der Abscheidegrad A in Abhängigkeit vom Volumenstrom in der magnetischen Abscheidevorrichtung, angegeben als Anzahl n der Durchleitungen durch die Abscheidevorrichtung dargestellt ist;

Fig. 11: ein Diagramm, in welchem die magnetische Suszeptibilität $\chi$ der in der magnetischen Abscheidevorrichtung abgeschiedenen magnetisierbaren Trägerpartikel in Abhängigkeit vom Volumenstrom, angegeben als Anzahl n der Durchleitungen durch den Abscheider dargestellt ist;

Fig. 12: ein Diagramm, in welchem der Einfluss der Partikelgröße und der Viskosität der Testflüssigkeit auf den Abscheidegrad dargestellt ist;

Fig. 13: ein Diagramm, in welchem der Einfluss der Partikelgröße auf die magnetische Suszeptibilität $\chi$ dargestellt ist;

Fig. 14: eine schematische Darstellung des Versuchsaufbaus zur Fermentierung von Rübensilage;

Fig. 15: ein Diagramm, in welchem die Methanbildungsrate $r(CH_4)$ sowie die Raumbelastung oS mit organischer Substanz der Reaktoren MF und MFK in Abhängigkeit vom zeitlichen Verlauf des Versuchs aufgetragen sind. Die Substratzugabe beim Kontrollreaktor MFK wurde ca. 3 Monate nach Beginn der 2. Versuchsphase eingestellt;

Fig. 16: ein Diagramm, in welchem die Methanausbeute $y(CH_4)$ sowie die Raumbelastung oS mit organischer Substanz der Reaktoren MF und MFK in Abhängigkeit vom zeitlichen Verlauf des Versuchs aufgetragen sind. Die Substratzugabe beim Kontrollreaktor MFK wurde ca. 3 Monate nach Beginn der 2. Versuchsphase eingestellt ;

Fig. 17: ein Diagramm, in welchem der pH-Wert des Substrats im Reaktor sowie die Raumbelastung oS mit organischer Substanz der Reaktoren MF und MFK in Abhängigkeit vom zeitlichen Verlauf des Versuchs aufgetragen sind. Die Substratzugabe beim Kontrollreaktor MFK wurde ca. 3 Monate nach Beginn der 2. Versuchsphase eingestellt;

Fig. 18: Lichtmikroskopische Aufnahmen eines magnetisierbaren partikulären Trägerpartikels, welches mit einem Biofilm bedeckt ist; dabei zeigt (a) eine Phasenkontrastaufnahme, (b) eine Auflicht-Fluoreszenz mit UV-Anregung und Emission über das gesamte visuelle Spektrum; (c) eine Auflicht-Fluoreszenz mit Blau-Anregung und Emission im Grünen und (d) ein Mischbild der Aufnahmen (a) bis (c).

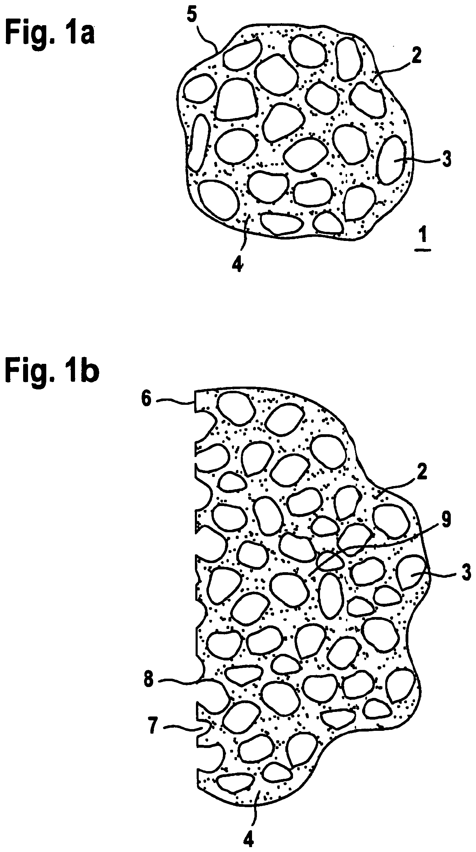

Fig. 1a zeigt schematisch einen Schnitt durch einen magnetisierbaren Träger 1, wie er im erfindungsgemäßen magnetisierbaren Aggregat enthalten ist. Der magnetisierbare Träger umfasst eine kontinuierliche Glasphase 2, welche geschlossene Poren 3 umschließt. Die geschlossenen Poren sind mit Gas gefüllt. Durch die Größe und die Anzahl der geschlossenen Poren 3 lässt sich die Dichte des magnetisierbaren Trägers 1 einstellen. In der kontinuierlichen Glasphase 2 sind magnetisierbare Partikel 4 verteilt, welche magnetisierbare Bereiche bilden. Die magnetisierbaren Partikel 4 sind in der kontinuierlichen Glasphase eingelagert und in dieser homogen verteilt. Bei der in Fig. 1 a gezeigten Ausführungsform ist die Außenseite des magnetisierbaren Trägers 1 von einer durchgehenden Haut gebildet, sodass im Wesentlichen alle Poren 3 geschlossen sind.

Fig. 1b zeigt schematisch einen Schnitt durch einen magnetisierbaren Träger 1, welcher aus dem in Figur 1a gezeigten magnetisierbaren Träger erhalten wird, indem dieser beispielsweise in einer Mühle gebrochen wird. Durch den Bruch wird eine Bruchfläche 6 erhalten, auf welcher Vertiefungen 7 sowie Grate 8 angeordnet sind. Bei der in der Figur 1b gewählten Darstellung verläuft die Bruchfläche 6 senkrecht zur Schnittebene, sodass nur die Kante zwischen Bruchfläche 6 und Schnittebene dargestellt ist. Vertiefungen 7 sowie Grate 8 werden erhalten, indem geschlossene Poren 3 durch den Bruch des magnetisierbaren Trägers 1 geöffnet werden. Wie auch beim in Fig. 1a gezeigten magnetisierbaren Träger weist der in Fig. 1b gezeigte magnetisierbare Träger in seinem Kern 9 eine geschlossenporige Schaumstruktur auf, welche geschlossene Poren 3 umfasst, die von der kontinuierlichen Glasphase 2 umschlossen werden. In der kontinuierlichen Glasphase 2 sind magnetisierbare Partikel 4 angeordnet, beispielsweise Ferritpartikel.

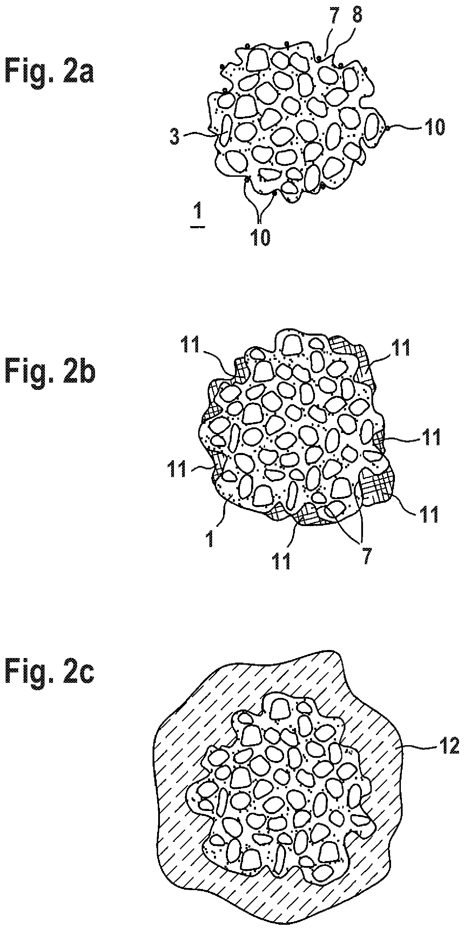

Fig. 2 zeigt verschiedene Stadien, die bei der Ausbildung eines aus Mikroorganismen gebildeten Biofilms auf einem magnetisierbaren Träger durchlaufen werden. Der magnetisierbare Träger 1 ist jeweils im Schnitt dargestellt.

Fig. 2a zeigt ein sehr frühes Stadium des Bewuchses. An einzelnen Stellen auf der Oberfläche des magnetisierbaren Trägers 1 haben sich Mikroorganismen 10 angesiedelt, wobei bevorzugt die Vertiefungen 7 als geschützte Bereiche besiedelt werden.

In Fig. 2b ist die Besiedlung weiter fortgeschritten. Insbesondere in den Vertiefungen 7 haben sich größere Kolonien 11 von Mikroorganismen ausgebildet.

Fig. 2c zeigt den Zustand nach Ausbildung eines Biofilms 12. Die in Fig. 2b gezeigten Kolonien 11 sind zusammengewachsen und bilden einen durchgehenden Biofilm 12 aus. Der Biofilm 12 umfasst mehrere Mikroorganismenarten.

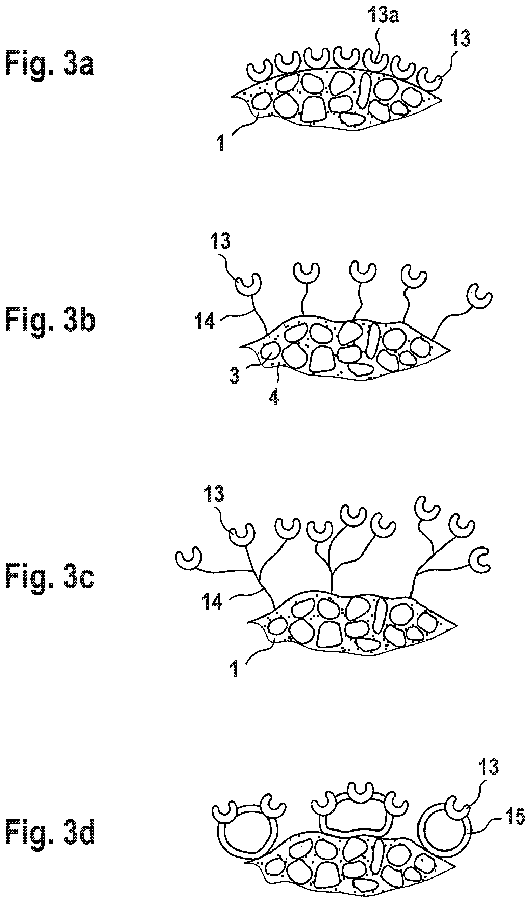

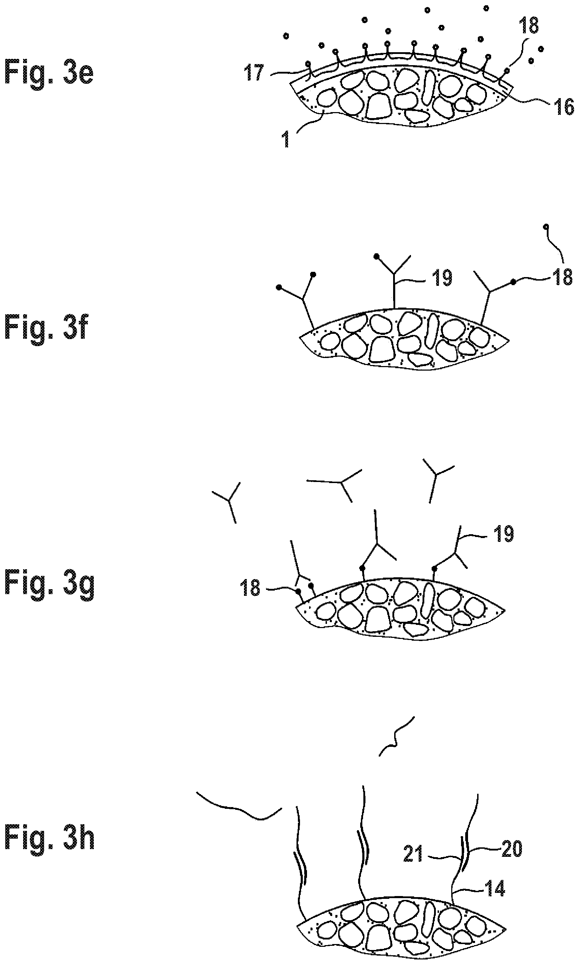

Fig. 3 zeigt schematisch verschiedene Ausführungsformen des erfindungsgemäßen magnetisierbaren Aggregats. Dargestellt ist jeweils ein Schnitt durch ein magnetisierbares Aggregat, wobei der Übersichtlichkeit halber jeweils nur ein Ausschnitt des Schnittbilds dargestellt ist. Die magnetisierbaren Aggregate umfassen jeweils einen magnetisierbaren Träger 1, der zumindest in seinem Kern von einem geschlossenporigen festen Schaum gebildet wird. Auf der Oberfläche des magnetisierbaren Trägers sind verschiedene aktive Komponenten angeordnet.

So wird in Fig. 3a eine Ausführungsform gezeigt, bei welcher ein Enzym 13 auf der Oberfläche des magnetisierbaren Trägers 1 gebunden ist. Das Enzym weist ein aktives Zentrum 13a auf. Das Enzym 13 kann auch über einen Spacer 14 an die Oberfläche des magnetisierbaren Trägers 1 gebunden sein, wie in Fig. 3 b gezeigt. Der Spacer 14 kann, wie in Fig. 3b gezeigt, linear sein oder auch um die Anzahl der Bindungsstellen zu erhöhen, verzweigt sein, wie dies in Fig. 3c gezeigt ist.

Bei der in Fig. 3d gezeigten Ausführungsform sind Vesikel 15 als Zellbestandteile auf der Oberfläche des magnetisierbaren Trägers 1 gebunden. In den Membranen der Vesikel 15 können dann beispielsweise membranständige Enzyme 13 eingeschlossen sein.

Fig. 3e zeigt eine Ausführungsform, bei welcher auf der Oberfläche des magnetisierbaren Trägers 1 ein Polymerfilm 16 angeordnet ist. Der Polymerfilm 16 wird aus Polymeren gebildet, welche Affinitätsgrupen 17 aufweisen, beispielsweise $NH_3^+$-Gruppen, an welche Zielmoleküle 18 gebunden werden können. Mit der in Fig. 3e gezeigten Ausführungsform ist es also möglich, Zielmoleküle 18 aus einer flüssigen Phase an das magnetisierbare Aggregat zu binden und dadurch anzureichern.

Fig. 3f zeigt eine Ausführungsform, bei welcher Antikörper 19 als Fängermoleküle auf der Oberfläche des magnetisierbaren Trägers 1 immobilisiert sind. Die Antikörper 19 binden Antigene 18, welche dadurch aus einer Lösung angereichert werden können.

Fig. 3g zeigt eine Ausführungsform, bei welcher Antigene 18 als aktive Komponente auf der Oberfläche des magnetisierbaren Trägers immobilisiert sind. Die Antigene 18 wirken als Fängermoleküle, um Antikörper 19 aus einer Lösung heraus an das magnetisierbare Aggregat zu binden.

Fig. 3h zeigt schließlich eine Ausführungsform, bei welcher eine DNA-Sonde 21 über einen Spacer 14 an die Oberfläche des magnetisierbaren Trägers 1 gebunden ist. Ziel-DNA-Moleküle 20, welche eine zur DNA-Sonde komplementäre Sequenz aufweisen, können von der DNA-Sonde gebunden werden und somit aus der Lösung an der Oberfläche des magnetisierbaren Trägers angereichert werden.

Zur Charakterisierung der magnetisierbaren Träger werden folgende Methoden eingesetzt.

Wassergehalt:

Der Wassergehalt der Produkte bei 105°C wird unter Verwendung der Methode DIN/ISO-787/2 ermittelt.

Bestimmung des Schüttgewichts

Ein bei der 1000 ml Markierung abgeschnittener Messzylinder wird gewogen. Dann wird die zu untersuchende Probe mittels eines Pulvertrichters so in einem Zug in den Messzylinder eingefüllt, dass sich oberhalb des Abschlusses des Messzylinders ein Schüttkegel ausbildet. Der Schüttkegel wird mit Hilfe eines Lineals, das über die Öffnung des Messzylinders geführt wird, abgestreift und der gefüllte Messzylinder erneut gewogen. Die Differenz entspricht dem Schüttgewicht.

Bestimmung der Kornrohdichte

Die Kornrohdichte wurde auf Grundlage der DIN EN 13055-1 nach DIN V 18004 5.2 bzw. nach der EN 1097-6:2000 bestimmt.

Bestimmung der magnetischen Suszeptibilität

Die magnetische Volumensuszeptibilität $c_v$ beschreibt eine Proportionalitätskonstante, definiert durch das Verhältnis:

$$M = \chi_V \cdot H$$

von Magnetisierung *M* und magnetischer Feldstärke *H.* Als Verhältnisgröße ist die Suszeptibilität einheitenlos.

Die Bestimmung von $c_v$ erfolgte mit einem Analysator FMA 5000 der Firma Forgenta (Forgenta Forschungstechnik- und Geräte-Entwicklung Adlershof GmbH, 6, Rudower Chaussee, 12489 Berlin). In dem Gerät befindet sich eine Spule mit einem Eisenkern. Sie wird mit einer Wechselspannung von 400 Hz durchflossen. Findet eine Veränderung der magnetischen Suszeptibilität durch das Einbringen des Versuchsmaterials statt, so ändern sich die Induktivität und damit der Wechselstromwiderstand in der Spule. Eine Brückenschaltung ermöglicht die Messung der Frequenzänderung $\Delta f$. Die Umrechnung auf $c_v$ erfolgte nach Klose et al. (2003) [Klose, S; Tölle, R; Bäucker, E; Makeschin, F:"Stratigraphic distribution of lignite-derived atmospheric deposits in forest soils of the upper Lusatian Region, East Germany" Water, Air and Soil Pollution 142:3-25 (2003)] anhand folgender Beziehung:

$$\chi_V = 0{,}70 \cdot 10^{-5} \cdot \Delta f$$

Da magnetisierbare Trägerpartikel mit unterschiedlicher Dichte verglichen werden sollen, wird anstelle von $c_v$ die magnetische Massensuszeptibilität $c_{mass}$ bestimmt. Die Umrechnung von $c_v$ auf $c_{mass}$ in der SI-Einheit $m^3 kg^{-1}$ erfolgt anhand der Probendichte $\rho$ ($kg\ m^{-3}$) durch die Gleichung

$$\chi_{mass} = \frac{\chi_V}{\rho} \ .$$

Sofern Proben untersucht werden, welche aus dem Fermenter entnommen worden waren wurden diese durch Zusatz von 0,5% Xanthan verdickt, um ein Absinken der magnetisierbaren Trägerpartikel aus der Spule während der Messung zu verhindern.

Bestimmung der Viskosität

Die Erfassung der Fließkurven erfolgte mit Hilfe des beheizbaren Rotationsviskosimeters MC1/RM300 mit dem Zylindersystem Z10 der Firma Haake. Mit diesem Gerät ist es möglich, die scheinbare dynamische Viskosität von Newtonschen und Nicht-Newtonschen Substanzen zu messen. Die Substanz befindet sich hierbei in einem Ringspalt zwischen zwei koaxialen Zylindern. Der äußere Zylinder steht fest, während der innere Zylinder angetrieben wird. Durch vorgegebene Programme kann das entstehende Schergefälle sehr genau eingestellt werden. Dadurch wird ein Fließwiderstand in der Probe hervorgerufen, der ein Maß für die dynamische Viskosität der Modellflüssigkeit darstellt. Durch einen hochgenauen Drehmomentaufnehmer am inneren Zylinder kann die scheinbare Viskosität in Abhängigkeit von der Schergeschwindigkeit aufgezeichnet werden.

Beispiel 1: Herstellung magnetisierbarer Glaspartikel

1. Verfahrensvariante

Eine erste Verfahrensvariante für die Herstellung magnetischer Glaspartikel sieht folgende Produktionsroutine im Labormaßstab vor:
Als Ausgangsstoffe werden verwendet:

| | |
|---|---|
| Glasmehl aus gemahlenem Altglas: | 8028 g |
| $\gamma$-$Fe_2O_3$-Pigment Bayoxide® EAB 21: | 1972 g. |

Diese Ausgangsstoffe werden in einem Mischer trocken vorgemischt und anschließend vermahlen.
Solche Pigmente werden beispielsweise von der Fa. Lanxess vertrieben. Ein Beispiel für das angegebene $\gamma$-Eisenoxidpigment stellt das Material Bayoxide® EAB21 dar. Ein Beispiel für ein Magnetitpigment stellt das Material Bayoxide® E 7810 dar.
Als zweite Vormischung werden nass gemischt:

| | |
|---|---|
| Wasserglas: | 1864 g |
| Wasser: | 1480 g |
| Natronsalpeter (Blähmittel): | 76 g. |

Die beiden Vormischungen werden vollständig in einem Lödige-Pflugscharmischer für 60 Sekunden granuliert. Die hergestellten Blähglas-Granulat-Grünkörper werden anschließend für mehrere Stunden bei einer Temperatur von 105° C in einem Ofen getrocknet.
Die getrockneten Grünkörper werden anschließend klassiergesiebt, große Bestandteile mechanisch zerkleinert und abermals klassiergesiebt. Eine Siebgrenze ist 0,25 mm.
Die erhaltenen Grünkörper werden mit einem Zusatz von 30 Volumen-% Kaolin als Trennmittel in einem Drehrohrofen bei Temperaturen zwischen 780° C und 815° C über einen Zeitraum von 15 Minuten verschäumt. Je nach Verschäumungstemperatur ergeben sich Schüttdichten im Bereich zwischen 100 g/l und 1200 g/l, wobei in der Regel mit steigender Verschäumungstemperatur die Schüttdichte abnimmt.
Die geschäumten Partikel des so hergestellten magnetischen Blähglasgranulats werden anschließend wiederum klassiergesiebt und in einer Größe von 0,1 mm bis 0,3 mm für die weiter unten beschriebenen Verwendungsversuche eingesetzt.
Mittels Röntgenbeugungsanalyse in einem Diffraktometer (Philips X-PERT) können die Phasen dieses Materials halbquantitativ bestimmt werden zu:

| | |
|---|---|
| 40% | $\gamma$-$Fe_2O_3$ (Maghemit) |
| 24% | $Fe_3O_4$ (Magnetit) |
| 10% | $\alpha$-$Fe_2O_3$ |
| 17% | $SiO_2$ (Cristobalit) |
| 9% | $SiO_2$ (Quarz). |

Bezogen auf Eisenoxid ergibt sich:

| | |
|---|---|
| 54% | $\gamma$-$Fe_2O_3$ (Maghemit) |
| 32% | $Fe_3O_4$ (Magnetit) |
| 14% | $\alpha$-$Fe_2O_3$ |

Als magnetische Suszeptibilität wurde bei diesem Material eine dimensionslose volumenbezogene Suszeptibilität von 1,21*10$^{-5}$ und eine massebezogene magnetische Suszeptibilität mit 2*10$^{-8}$ m$^3$/kg gemessen.

Die Werte des Ausgangsoxides Bayoxide$^®$ EAB21 liegen für die volumenbezogene magnetische Suszeptibilität bei 2,41*10$^{-4}$ und die massebezogene magnetische Suszeptibilität bei 2,77*10$^{-7}$ m$^3$/kg. Die Phasenzusammensetzung liegt bei 97% $\gamma$-Fe$_2$O$_3$ (Maghemit) und 3% $\alpha$-Fe$_2$O$_3$.

2. Verfahrensvariante

Eine zweite Verfahrensvariante für die Herstellung von magnetischem Blähglasgranulat sieht die Erstellung einer ersten Vormischung aus folgenden Bestandteilen vor:

| | |
|---|---|
| Glasmehl: | 138,3 g |
| $\gamma$-Fe$_2$O$_3$-Magnetpartikel Bayoxide$^®$ EAB 21: | 34,6 g. |

Diese Bestandteile werden in einem Mischer trockendispergiert.

Eine zweite Vormischung wird erstellt aus:

| | |
|---|---|
| Wasserglas: | 137,0 g |
| Wasser: | 108,7 g |
| Natronsalpeter (Blähmittel): | 5,9 g. |

Diese Bestandteile werden in einem Mischer zu einem homogenen Schlicker dispergiert, der erwärmt wird.

Eine weitere Charge der ersten Vormischung wird hergestellt aus:

| | |
|---|---|
| Glasmehl: | 443,2 g |
| $\gamma$-Fe$_2$O$_3$-Magnetpartikel Bayoxide$^®$ EAB 21: | 118,8 g. |

Auch diese zweite Charge wird in einem Mischer trocken dispergiert.

Die beiden Chargen der Vormischung 1 und die Vormischung 2 werden in einem Hobart-Mischer mit den Stufen 1-2-1 granuliert. Die so hergestellten Blähglas-Granulat-Grünkörper werden über mehrere Stunden bei einer Temperatur von 105° C getrocknet.

Zur Herstellung der eigentlichen magnetisierbaren Blähglasgranulat-Partikel werden die vorgenannten Grünkörper mit 30 Volumen-% Kaolin als Trennmittel in einem Drehrohrofen bei 740° C über eine Zeit von 0,5 Stunden verschäumt. Es ergibt sich ein magnetisches Blähglasgranulat mit einer Schüttdichte von 505 g/l.

3. Verfahrensvariante

In einer dritten Verfahrensvariante für die Herstellung von magnetisierbarem Blähglasgranulat kommt ein Sprühturm zur Aufgranulierung einer Schlickersuspension vor.

Zu deren Herstellung wird eine erste Vormischung aus folgenden Bestandteilen hergestellt:

| | |
|---|---|
| Glasmehl: | 1690 g |
| $\gamma$-Fe$_2$O$_3$-Magnetpartikel: | 450 g. |

Diese Bestandteile werden in einer Kugelmühle für ca. 20 min gemahlen und homogenisiert.

Danach wird die Schlickersuspension erstellt aus:

| | |
|---|---|
| der vorstehenden Vormischung | 1900 g |
| Wasserglas | 600 g |
| Wasser | 1300 g |
| Natronsalpeter (Blähmittel) | 66 g. |

Diese Bestandteile werden in einem Mischer zu einem homogenen Schlicker dispergiert, der in einem Sprühturm mittels einer Ringdüse versprüht wird.

Beispiel 2: Abscheideversuche mit unbesiedelten magnetisierbaren Trägerpartikeln

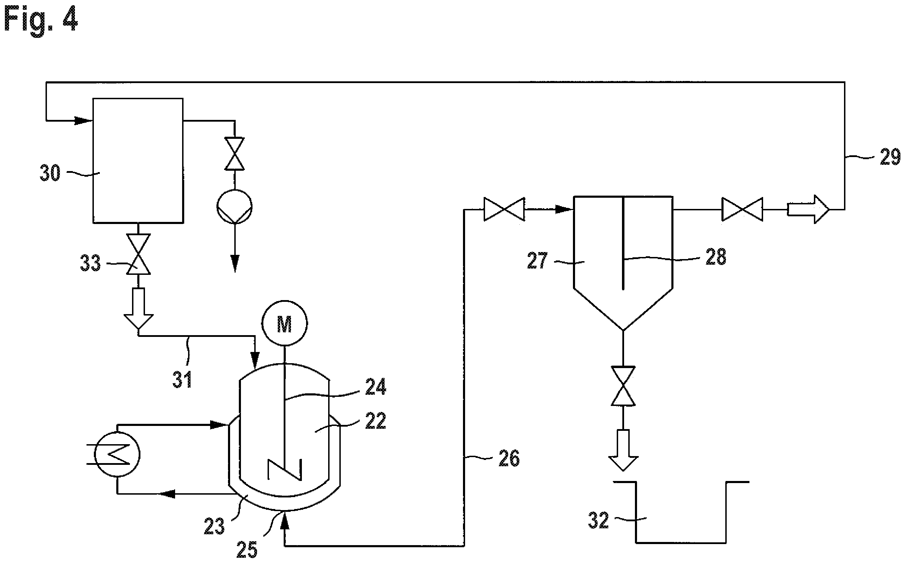

Zur Durchführung der Abscheideversuche wurde die in Fig. 4 dargestellte Vorrichtung verwendet.

In einem Rührkesselreaktor 22, welcher mit einer Heizung 23 sowie einem Rührwerk 24 ausgestattet ist, wird eine Substratmischung vorgelegt. In der Substratmischung sind magnetisierbare Träger suspendiert, auf welchen Mikroorganismen aufgewachsen sind. Über einen Ablauf 25 wird die Substratmischung aus dem Reaktor ausgeschleust und über Leitung 26 in einen magnetischen Abscheider 27 überführt. Im magnetischen Abscheider 27 ist eine magnetische Abscheidevorrichtung 28, beispielsweise ein stabförmiger Magnet, angeordnet. Während die Substratmischung durch den magnetischen Abscheider 27 geleitet wird, scheiden sich die magnetisierbaren Träger an der magnetischen Abscheidevorrichtung 28 ab. Das an den magnetisierbaren Trägerpartikeln abgereicherte Substrat wird dann über eine Leitung 29 einem Vorratsbehälter 30 zu geführt. Aus dem Vorratsbehälter 30 lässt sich das Substrat über Leitung 31 wieder dem Rührkesselreaktor 22 zuführen.

Die an der magnetischen Abscheidevorrichtung abgeschiedenen magnetisierbaren Träger können herausgespült werden und in einem Ablaufbecken 32 aufgefangen werden.

Die Modellflüssigkeit und die darin suspendierten magnetisierbaren Träger werden in den Vorratsbehälter 30 eingefüllt. Durch Öffnen des Ventils 33 werden die Modellflüssigkeit und die darin suspendierten magnetisierbaren Träger in den Rührkesselreaktor 22 überführt. Anschließend wird das Ventil 33 wieder geschlossen. Die Modellflüssigkeit und die darin suspendierten magnetisierbaren Träger werden dann aus dem Rührkesselreaktor durch den Magnetabscheider 27 gepumpt, wo eine Abtrennung der magnetisierbaren Träger an der magnetischen Abscheidevorrichtung 28 erfolgt. Die an magnetisierbaren Trägerpartikeln abgereicherte Modellflüssigkeit wird dann wieder in den Vorratsbehälter 30 zurückgepumpt.

Magnetisierbare Trägerpartikel

Als magnetisierbare Trägerpartikel wurden die in Tabelle 1 zusammengefassten Schaumglasgranulate eingesetzt:

Tabelle 1: verwendete Schaumglasgranulate

| Versuchsprodukt-Bezeichnung | Durchmesser | magnetische Massensuszeptibilität $\chi_{mass}$ |
|---|---|---|
| V1 | 0,1 bis 0,3 mm | $2,00 * 10\text{-}8\ m^3\ kg^{-1}$ |
| V2 | 1 bis 2 mm | $1,05 * 10\text{-}8\ m^3\ kg^{-1}$ |

Modellflüssigkeiten

Als Modellflüssigkeiten wurden Wasser bzw. Gemische aus Wasser und Xanthan eingesetzt, welche 0,1% bzw. 0,25% Xanthan enthielten. Xanthan ist ein bakterielles Heteropolysaccharid, das als Verdickungs- und Geliermittel für die Lebensmittelherstellung zugelassen ist. Damit wurden wässrige Flüssigkeiten mit verschiedener Viskosität hergestellt. Somit konnte der Einfluss der Viskosität auf die Rückgewinnung der magnetisierbaren Trägerpartikel untersucht werden.

Wasser hat die geringste Viskosität. 0,1% Xanthan-Lösung besitzt eine mittlere Viskosität (etwa im Mittelfeld von stark / mittel / wenig viskosem Reaktorablauf). Und 0,25% Xanthan-Lösung weist die höchste Viskosität auf.

Als Beispiele für reale Substrate, wie sie bei der praktischen Durchführung der Biogaserzeugung anfallen, wurden auch verschiedene Abläufe aus Laborbiogasreaktoren untersucht.

Der Impfablauf "meso" dient als Beispiel für einen Ablauf mit hoher Viskosität. Dieser Ablauf weist den höchsten Trockensubstanzgehalt der untersuchten Abläufe auf.

Der Ablauf "Rübensilage" wurde als Beispiel für einen Biogasreaktorablauf mit mittlerer Viskosität untersucht.

Der Ablauf "AFR Ablauf thermo" weist nur einen Trockensubstanzgehalt von 0,36 % auf und dient als Beispiel für einen sehr dünnflüssigen Ablauf.

Bestimmung der Viskosität der Modellflüssigkeiten

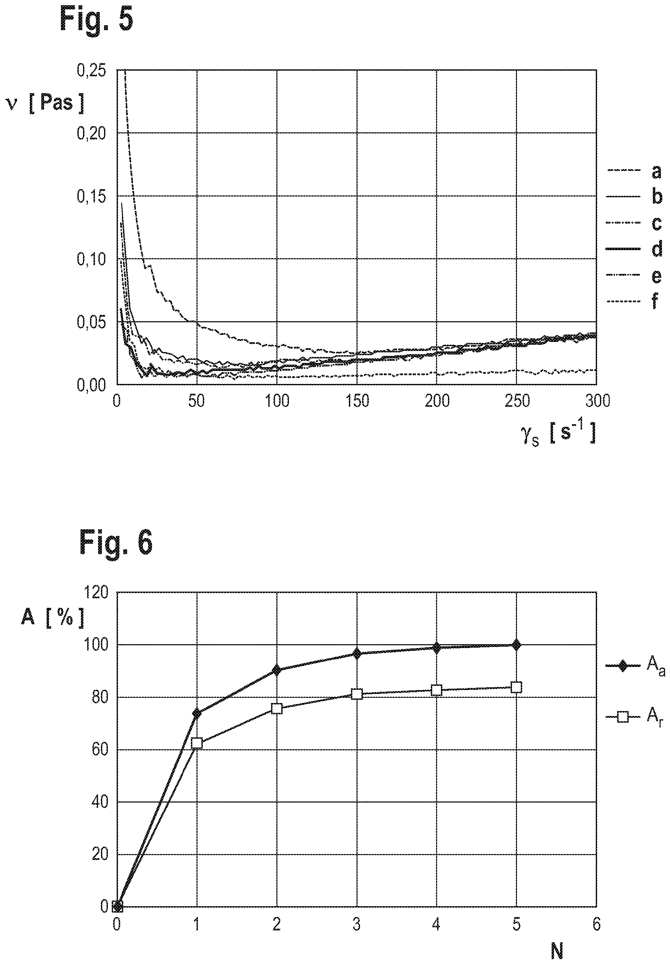

Da die Viskosität einen wesentlichen Einfluss hat, erfolgte eine genaue Einstellung und Charakterisierung der in Tabelle 2 beschriebenen Modellflüssigkeiten a bis f. Es wurde jeweils die Scheinviskosität $\nu$ in Pas in Abhängigkeit von der Schergeschwindigkeit $\gamma_s$ in $s^{-1}$ bestimmt. Diese Daten werden in Fig. 5 dargestellt. Zur besseren Bewertung wurde auch die Viskosität realer Reaktorabläufe bestimmt und ebenfalls in Figur 5 aufgenommen. Die Indices "raum", "meso" und "thermo" beziehen sich auf die Temperatur der untersuchten Modellflüssigkeiten.

raum = Raumtemperatur (20°C),
meso = mesophil (35°C),
thermo = thermophil (55°C).

Es wurde die Viskosität der in Tabelle 2 zusammengefassten Modellflüssigkeiten bestimmt.

Tabelle 2: Modellflüssigkeiten zur Bestimmung der Viskosität

| | Kurzbezeichnung Modellflüssigkeit | Beschreibung |
|---|---|---|
| a | Xanthan 2,5:1000 raum | 0,25% Xanthanlösung in Wasser |
| b | Impfablauf meso | Laborbiogasanlagenablauf aus der mesophilen Vergärung von Gülle (Rind und Schwein) sowie Silagen aus nachwachsenden Rohstoffen (Gras-, Mais- und Getreideganzpflanzen) |
| c | Xanthan 1:1000 raum | 0,1% Xanthanlösung in Wasser |
| d | Gehaltsrübensilage thermo | Laborbiogasanlagenablauf aus thermophil vergorener Gehaltsrübensilage mit 5% Masseanteil an Roggenschrot |
| e | AFR Ablauf thermo | Laborbiogasanlagenablauf aus der thermophilen Vergärung von überwiegend Maissilage und geringen Mengen von Schweinegülle. |
| f | Wasser | Wasser |

Versuchsaufbau

Als magnetische Abscheidevorrichtung wurde ein Magnetseparator Liquimag LM9-E-050-7 der Firma S+S Separation and Sorting Technology GmbH, Regener Straße 130, 94513 Schönberg (2009) verwendet. Bei diesem Gerät handelt es sich um einen handelsüblichen Filtermagneten, der in der Lebensmittelindustrie zur Abtrennung von metallischen Verunreinigungen eingesetzt wird. Das Funktionsprinzip basiert darauf, dass die hintereinander angeordneten Magnetstäbe von der magnetpartikelhaltigen Flüssigkeit umströmt werden und die magnetisierbaren Trägerpartikel an diesen Stäben anhaften. Flüssigkeitsstrom und Magnetstäbe kreuzen sich in diesem Fall, da die Magnetstäbe senkrecht zur Fließrichtung angeordnet sind.

Dieser Magnetseparator muss in bestimmten Zeitabständen manuell gereinigt werden. Dafür sind eine Unterbrechung des Flüssigkeitskreislaufes und eine Entnahme der Magnetstäbe notwendig.

Der Magnetseparator wurde wie in Fig. 4 dargestellt mit einem Vorratsbehälter sowie einem Auffangbehälter verbunden, um die Proben durch den Magnetseparator zu führen. Die Förderung des Probenmaterials erfolgte über Unterdruck, den eine am Auffangbehälter angeschlossene Pumpe erzeugte. Zusätzlich wurden die Probentemperatur, die Masse der in den Auffangbehälter gepumpten Probe und die Durchflusszeit bestimmt.

Versuchsdurchführung

Aus der jeweiligen Modellflüssigkeit a bis f und einer bestimmten Menge an magnetisierbaren Trägerpartikeln wurde für jeden Versuch ein Gemisch hergestellt. Dabei wurden die Viskosität der Modellflüssigkeit und deren Gehalt an magnetisierbaren Trägerpartikeln systematisch variiert.

Dieses Gemisch wurde dann in den Vorratsbehälter gegeben und mit einer ebenfalls variierten Fließgeschwindigkeit durch den Magnetseparator gepumpt. Der Pumpvorgang erfolgte mittels Unterdruck am Auffangbehälter. Abschließend wurde die Masse der im Magnetseparator zurückgehaltenen magnetisierbaren Trägerpartikel bestimmt und daraus der prozentuale Rückgewinnungsgrad, bezogen auf die ursprünglich der Probe zugesetzten Masse an magnetisierbaren Trägerpartikeln, errechnet.

Dazu wurden nach jedem Trennversuch die Edelstahlhülsen der Magnetstäbe des Magnetseparators manuell gereinigt. Das heißt, die an den Edelstahlhülsen anhaftenden magnetisierbaren Trägerpartikel wurden mit deionisiertem Wasser abgespült und in Eindampfschalen aufgefangen.

Anschließend wurde die Flüssigkeit über eine Zeitdauer von 24 h in einem Trockenschrank bei 105 °C eingedampft. Dadurch konnte nach Subtraktion des Leergewichts der Eindampfschale das Gewicht der abgeschiedenen magnetisierbaren Trägerpartikel bestimmt werden.

Versuchsergebnisse

## 1. Abscheidegrad der magnetisierbaren Trägerpartikel im Magnetseparator

### 1.1. Wasser

Es wurden 1% (w/w) magnetisierbare Trägerpartikel Durchmesser 0,1-0,3 mm (V1) in Wasser suspendiert und 20x durch den Magnetseparator geleitet. Nach dem 1., 2., 5., 10. und 20. Durchgang wurden die im Magnetseparator abgeschiedenen magnetisierbaren Trägerpartikel aufgefangen, getrocknet und gewogen. Die abgeschiedene Menge an magnetisierbarem Träger ist in Tabelle 3 angegeben. Aus den Werten ergibt sich die in Fig. 6 dargestellte Abtrennkurve, wobei für jede Probennummer N der Abtrenngrad A angegeben ist. Angegeben ist jeweils der Abtrenngrad $A_a$ bezogen auf die eingesetzte Menge an magnetischem Träger sowie der Abtrenngrad $A_r$, welcher auf die Gesamtmenge an magnetisch abgeschiedenem Träger bezogen ist.

Tabelle 3: Menge der aus Wasser abgeschiedenen magnetisierbaren Trägerpartikel; Eingesetzte Menge an magnetischem Träger 70,00g; maximal abgeschiedene Menge 58,87 g; 84,1 %)

| Durchlauf | Probennr. N | Menge pro Durchlauf (g) | Summe (g) | Anteil an abgeschiedener Menge $A_r$ (%) | Anteil an eingesetzter Menge $A_a$ (%) |
|---|---|---|---|---|---|
| 0 | 0 | 0 | 0 | 0,0 | 0,0 |
| 1 | 1 | 43,68 | 43,68 | 74,20 | 62,40 |
| 2 | 2 | 9,46 | 53,14 | 90,27 | 75,91 |
| 2 - 5 | 3 | 3,77 | 56,91 | 96,66 | 81,29 |
| 6 - 10 | 4 | 1,44 | 58,34 | 99,10 | 83,35 |
| 11 - 20 | 5 | 0,52 | 58,87 | 100,00 | 84,10 |

Nach 20 Durchläufen zeigte die Kurve ein Sättigungsverhalten an, d.h. nach 20 Durchläufen waren praktisch alle abtrennbaren magnetisierbaren Trägerpartikel aus der Modellflüssigkeit abgetrennt. Von den zu Versuchsbeginn dosierten 70,00 g magnetisierbare Trägerpartikel konnten 58,87 g abgeschieden werden. Das entspricht 84,10%. Beim verbleibenden Anteil von 15,9% handelt es sich um schlecht bzw. nicht abscheidbares Material mit nur schwachen magnetischen Eigenschaften. Die abscheidbaren 84,10% der ursprünglich eingesetzten Menge an magnetisierbaren Trägerpartikeln wurden als maximal abtrennbarer Anteil der magnetisierbaren Trägerpartikel und damit als Bezugsgröße für 100 % gesetzt. So gerechnet, konnten nach dem 1. Durchgang 74,20% des abscheidbaren Materials abgetrennt werden, nach dem 2. Durchgang weitere 16,07% und für die Abtrennung der restlichen 9,63% wurden weitere 18 Durchläufe benötigt

### Wässrige Xanthanlösung (0,25 %)

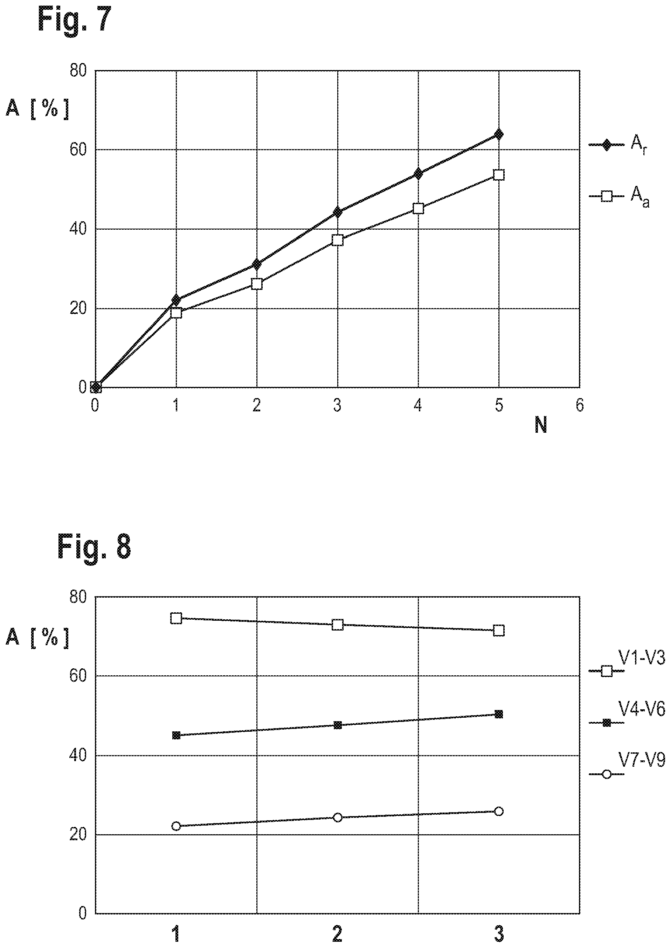

Der Versuch wurde mit 0,25 %-iger wässriger Xanthanlösung wiederholt. Die Menge an abgeschiedenen magnetischen Trägerpartikeln ist in Tabelle 4 angegeben. Der Abscheidegrad der magnetisierbaren Trägerpartikel ist in Figur 7 dargestellt. Es ist jeweils der für die in Tabelle 4 aufgeführten Probennummer N ermittelte Abscheidungsgrad $A_a$, welcher auf die Menge an eingesetztem magnetischen Träger bezogen ist, und der Abscheidungsgrad $A_r$, welcher auf die magnetisch abscheidbare Menge des magnetischen Trägers bezogen ist, angegebenen.

Tabelle 4: Menge der aus einer Xanthanlösung (0,25%) abgeschiedenen magnetisierbaren Trägerpartikel; eingesetzte Menge an magnetischem Träger 70,00g; maximal abgeschiedene Menge 58,87 g; 84,1 %)

| Durchlauf | Probennr. N | Menge pro Durchlauf (g) | Summe (g) | Anteil an abgeschie dener Menge $A_r$ (%) | Anteil an eingesetz ter Menge $A_a$ (%) |
|---|---|---|---|---|---|
| 0 | 0 | 0 | 0 | 0,00 | 0,00 |
| 1 | 1 | 13,05 | 13,05 | 22,17 | 18,64 |
| 2 | 2 | 5,27 | 18,32 | 31,12 | 26,17 |
| 2 - 5 | 3 | 7,66 | 25,98 | 44,13 | 37,11 |

(fortgesetzt)

| Durchlauf | Probennr. N | Menge pro Durchlauf (g) | Summe (g) | Anteil an abgeschie dener Menge $A_r$ (%) | Anteil an eingesetz ter Menge $A_a$ (%) |
|---|---|---|---|---|---|
| 6 - 10 | 4 | 5,76 | 31,74 | 53,92 | 45,34 |
| 11 - 20 | 5 | 5,73 | 37,47 | 63,65 | 53,53 |

Von den magnetisch abscheidbaren Partikeln (100%) wurden nach 20 Durchläufen nur insgesamt 63,65% abgeschieden. Nach dem 1. Durchlauf waren es nur 22,17%; nach dem 2. Durchlauf weitere 8,95%. Die erhöhte Viskosität beeinträchtigt die Abscheidung der 0,1-0,3 mm großen magnetisierbaren Trägerpartikel erheblich.

Einfluss der Viskosität der Modellflüssigkeit und der Konzentration der magnetisierbaren Trägerpartikel auf den Abscheidegrad

Die Modellflüssigkeit mit den darin suspendierten magnetisierbaren Trägerpartikeln wurde nur einmal durch den Magnetseparator gepumpt. Die verwendeten Modellflüssigkeiten und die Menge sowie die Art der magnetisierbaren Trägerpartikel sind in Tabelle 5 zusammengefasst und in Figur 8 graphisch dargestellt. Dabei ist in der Spalte 1 der Abscheidegrad A für eine Menge von 0,1 % magnetisierbarem Träger, in Spalte 2 der Abscheidegrad A für eine Menge von 0,5% magnetisierbarem Träger und in Spalte 3 der Abscheidegrad A für eine Menge von 1,0 % magnetisierbarem Träger angegeben.

Tabelle 5: Modellflüssigkeiten und Abscheiderate bei einmaligem Durchlauf der Probe (V1) durch den Magnetseparator

| Versuchsnr. | Modellflüssigkeit | Konzentration Träger (Gew.-%) | Abscheidegrad (%) |
|---|---|---|---|
| 1 | Wasser | 0,1 | 75,08 |
| 2 | Wasser | 0,5 | 71,75 |
| 3 | Wasser | 1,0 | 72,80 |
| 4 | Xanthan (0,1%) | 0,1 | 45,01 |
| 5 | Xanthan (0,1%) | 0,5 | 47,90 |
| 6 | Xanthan (0,1%) | 1,0 | 50,55 |
| 7 | Xanthan (0,25%) | 0,1 | 23,10 |
| 8 | Xanthan (0,25%) | 0,5 | 22,80 |
| 9 | Xanthan (0,25%) | 1,0 | 26,65 |

Bei nur einem Abscheidevorgang und einer Erhöhung der Massenkonzentration magnetischer Partikel (0,1-0,3 mm) von 0,1% auf 0,5% und 1% veränderte sich der Abscheidegrad nur wenig.

Zu erwarten wäre gewesen, dass sich bei steigender Partikelmasse die Partikel im Separator gegenseitig behindern und abschirmen. Offensichtlich war der Massenkonzentrationsbereich von 0,1-1% so gering, dass der erwartete Effekt noch nicht eintrat.

Die Erhöhung der Viskosität dagegen hatte einen eindeutigen Effekt: je höher die Viskosität, desto niedriger fiel der Abscheidegrad aus.

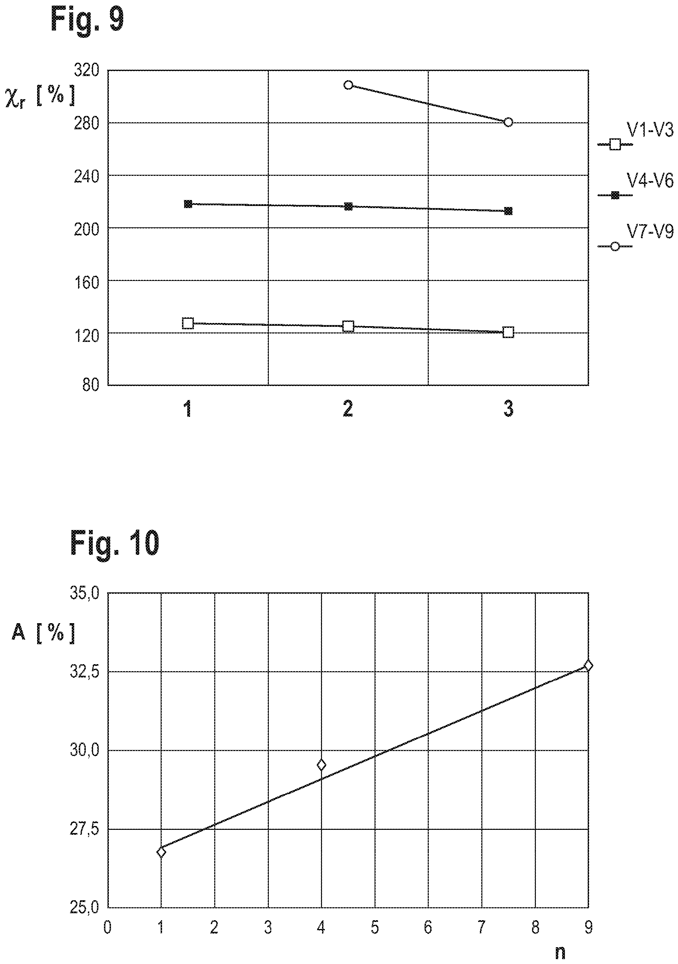

Die magnetische Suszeptibilität der jeweils abgeschiedenen magnetisierbaren Trägerpartikel wurde gemessen und in Bezug zur magnetischen Suszeptibilität des Ausgangsmaterials (100%) gesetzt. Je höher die Viskosität der Modellflüssigkeit war (und je weniger magnetisierbare Trägerpartikel abgeschieden worden waren), desto höher war auch die magnetische Suszeptibilität der abgeschiedenen magnetisierbaren Trägerpartikel. Danach selektioniert eine hohe Viskosität der Modellflüssigkeit auf Partikel mit hoher magnetischer Suszeptibilität (jedenfalls bei 0,1-0,3 mm Partikeldurchmesser). Die für die abgeschiedenen magnetisierbaren Trägerpartikel gemessene relative magnetische Suszeptibilität $\chi_r$ ist in Tabelle 6 für die verschiedenen Versuche zusammengefasst und in Figur 9 graphisch wiedergegeben. Dabei ist für die verschiedenen Modellflüssigkeiten in Spalte 1 die relative magnetische Suszeptibilität $\chi_r$ für eine Menge von 0,1 % magnetisierbarem Träger, in Spalte 2 die relative magnetische Suszeptibilität $\chi_r$ für eine Menge von 0,5 % magnetisierbarem Träger und in Spalte 3 die relative magnetische Suszeptibilität $\chi_r$ für eine Menge von 1,0 % magnetisierbarem Träger angegeben.

Tabelle 6: relative magnetische Suszeptibilität $\chi_r$ der bei einmaligem Durchlauf der Probe (V1) durch den Magnetseparator abgeschiedenen magnetisierbaren Trägerpartikel

| Versuchsnr. | Modellflüssigkeit | Konzentration Träger (Gew.-%) | Relative magnetische Suszeptibilität $\chi_r$ (%) |
|---|---|---|---|
| 1 | Wasser | 0,1 | 124,95 |
| 2 | Wasser | 0,5 | 128,22 |
| 3 | Wasser | 1,0 | 120,31 |
| 4 | Xanthan (0,1%) | 0,1 | 215,13 |
| 5 | Xanthan (0,1%) | 0,5 | 219,76 |
| 6 | Xanthan (0,1%) | 1,0 | 209,84 |
| 7 | Xanthan (0,25%) | 0,1 | n.b. |
| 8 | Xanthan (0,25%) | 0,5 | 308,57 |
| 9 | Xanthan (0,25%) | 1,0 | 282,79 |

2. Auswirkung des Volumenstroms auf den Abscheidegrad

Gemessen wurde der Abscheidegrad A der magnetisierbaren Trägerpartikel in einer wässrigen 0,25 %-igen Xanthanlösung bei einmaligem Durchlauf durch den Magnetseparator. Die Ergebnisse sind in Tabelle 7 zusammengefasst und in Fig. 10 graphisch wiedergegeben.

Tabelle 7: Abscheidegrad von magnetisierbaren Trägerpartikeln aus Xanthan/Wasser 2,5 : 1000 bei mehrmaligem Durchleiten durch einen Abscheider

| Anzahl der Durchleitungen n | Abscheidegrad A (%) |
|---|---|
| 1 | 27,0 |
| 4 | 29,3 |
| 9 | 32,5 |

Je höher der Volumenstrom (d.h. je höher die Fließgeschwindigkeit im Magnetseparator), desto niedriger fiel der Abtrenngrad der magnetisierbaren Trägerpartikel aus.

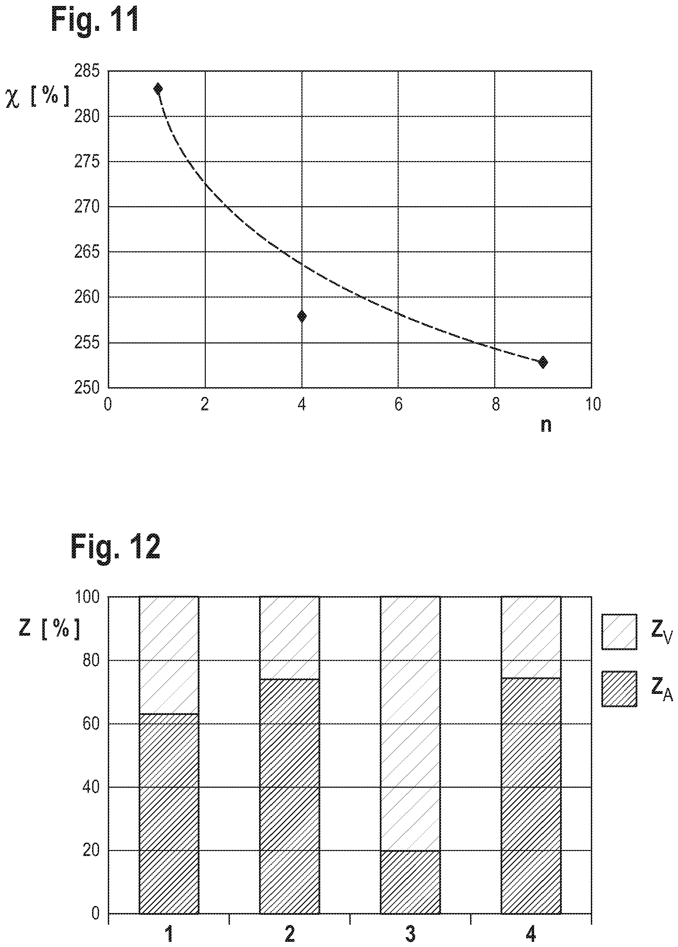

Ferner wurde die magnetische Suszeptibilität der abgeschiedenen magnetisierbaren Trägerpartikel bestimmt. Die Ergebnisse sind in Tabelle 8 zusammengefasst und in Figur 11 graphisch wiedergegeben.

Tabelle 8: magnetische Suszeptibilität der aus einer wässrigen Xanthanlösung (0,25%) abgeschiedenen Trägerpartikel in Abhängigkeit von der Anzahl der Durchleitungen durch einen Abscheider

| Anzahl Durchleitungen | Magnetische Suszeptibilität $\chi$ in % bezogen auf die Suszeptibilität der ursprünglich eingesetzten Trägerpartikel |
|---|---|
| 1 | 283 |
| 4 | 258 |
| 9 | 256 |

Je höher der Volumenstrom im Magnetseparator, desto höher war die magnetische Suszeptibilität der abgeschiedenen magnetisierbaren Trägerpartikel.

Die Erfinder erklären diesen Effekt in der Weise, dass nur auf magnetisierbare Trägerpartikel mit hoher magnetischer Suszeptibilität ausreichend hohe Magnetkräfte wirken, um sie trotz der starken Strömung abscheiden zu können.

3. Auswirkung von Partikelgröße und Viskosität auf den Abtrenngrad

Es wurden magnetisierbare Trägerpartikel mit einer Partikelgröße von 1-2 mm (V2) mit magnetisierbaren Trägerpartikeln mit einer Partikelgröße 0,1-0,3 mm (V1) verglichen. Es wurde die niedrigste Massenkonzentration von 0,1% gewählt. Als Modellflüssigkeiten wurden Wasser und 0,25%-ige wässrige Xanthan-Lösung eingesetzt. Gemessen wurde bei einem konstanten Volumenstrom von 30 1/min. Die Ergebnisse sind in Tabelle 9 sowie Figur 12 zusammengefasst.

Tabelle 9: Korngrößeneinfluss bei der Abscheidung mit 0,1 Massen-% magnetisierbarer Trägerpartikel

| Nr. | Partikelfraktion | Medium | Abgetrennter Anteil $Z_a$ [%] | Verlust $Z_v$ [%] |
|---|---|---|---|---|
| 1 | 0,1 - 0,3 mm | Wasser | 63,14 | 36,86 |
| 2 | 1 - 2 mm | Wasser | 74,14 | 25,86 |
| 3 | 0,1 - 0,3 mm | Xanthan 2,5 : 1000 | 19,43 | 80,57 |
| 4 | 1 - 2 mm | Xanthan 2,5 : 1000 | 74,43 | 25,57 |

Während bei der Korngröße 0,1-0,3 mm der Abtrenngrad durch die Viskositätserhöhung drastisch sank (von 63% auf 19%), blieb er bei der Korngröße 1-2 mm trotz Viskositätserhöhung fast unbeeinflusst (74% und 74%). Erklärung: mit dem Partikeldurchmesser nimmt auch das Partikelvolumen zu. Dadurch vergrößert sich die auf den Partikel wirkende Magnetkraft (ein gleichbleibend hoher Anteil eingemischten magnetisch suszeptiblen Materials vorausgesetzt.) Die erhöhte Magnetkraft reicht dann aus, um die erhöhten Reibungskräfte im hochviskosen Medium zu überwinden.

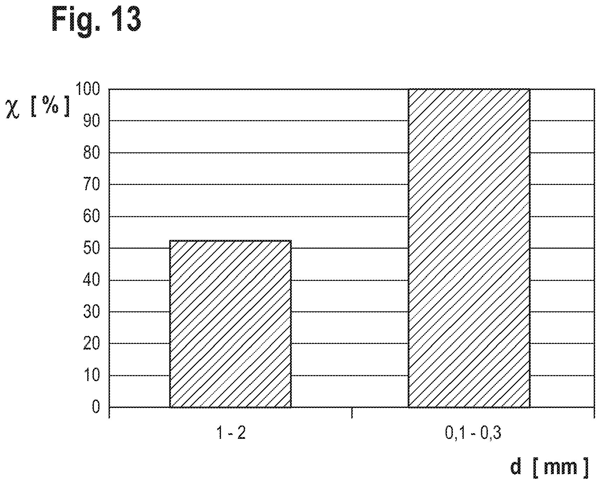

Ferner wurde die magnetische Suszeptibilität der beiden in den Versuchen eingesetzten magnetisierbaren Trägerpartikel-Typen untersucht. Die Ergebnisse sind in Tabelle 10 und graphisch in Figur 13 wiedergegeben.

Tabelle 10: Einfluss der Korngröße auf die magnetische Suszeptibilität

| Magnetische Suszeptibilität $\chi$ [%] | Partikeldurchmesser d |
|---|---|
| 52,38 | 1 - 2 mm |
| 100 | 0,1 - 0,3 mm |

Obwohl beide Körnungen aus derselben Mischung von Magnetpigment und Glaspulver hergestellt worden waren (also theoretisch den gleichen Anteil magnetisierbaren Materials enthalten sollten) und eine Korngrößenfraktionierung durch Siebung vorgenommen wurde, wies die Korngröße 0,1-0,3 mm eine fast doppelt so hohe magnetische Suszeptibilität auf wie die Korngröße 1-2 mm. Es ist unklar, ob es sich um eine Funktion der Partikelgröße handelt oder um Phänomene der Mischung / Entmischung bei der Herstellung.

Beispiel 3: Vergärung von Rübensilage in 50 Liter-Fermentern unter Zuhilfenahme von magnetisierbaren Trägerpartikeln

Um abzuklären, ob die Vergärung von Rübensilage zu Biogas durch den Einsatz von auf magnetisierbaren Trägerpartikeln immobilisierten Mikroorganismen gesteigert werden kann, wurden parallel zwei baugleiche Rührkesselfermenter mit einem Arbeitsvolumen von jeweils 50 Litern betrieben, die sich lediglich in 2 Merkmalen unterschieden. Dem "Magnetfermenter" (im Folgenden als MF abgekürzt) wurde zu Versuchsbeginn 1 Massenprozent magnetisierbare Trägerpartikel zugegeben. Um die magnetisierbaren Trägerpartikel aus dem Ablauf wieder abtrennen und rückführen zu können, war in den Ablauf des MF ein Magnetseparator eingebaut. Dem "Kontrollfermenter" (im Folgenden als MFK abgekürzt) wurden keine magnetisierbaren Trägerpartikel zudosiert, und in seinem Ablauf war kein Magnetseparator installiert. Die Betriebsweise beider Fermenter wurde während der etwa siebenmonatigen Versuchsdauer so identisch wie möglich gestaltet.

Material und Methoden

Versuchsaufbau

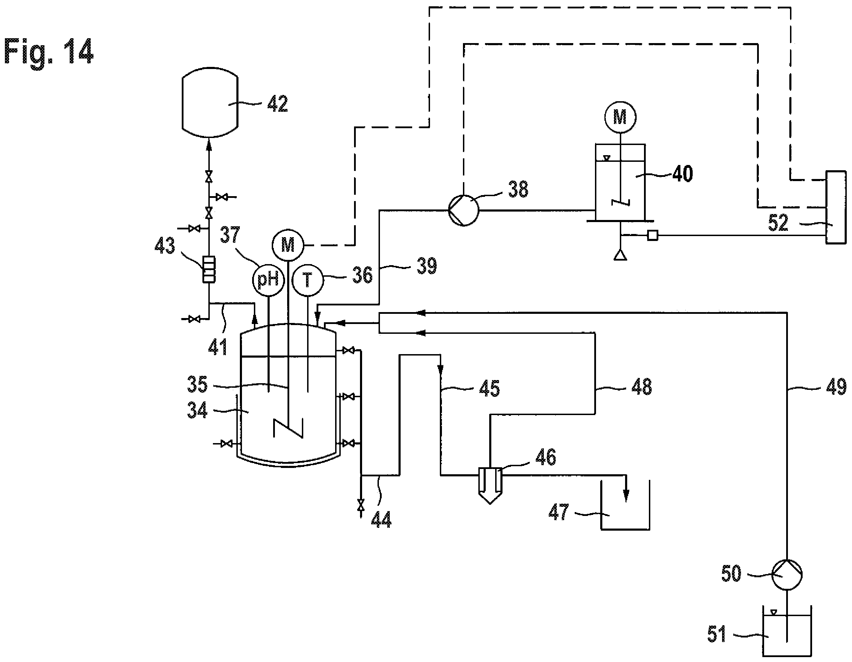

In Figur 14 ist schematisch der Aufbau der Versuchsanordnung wiedergegeben.

Ein temperierbarer Fermenter 34, welcher mit einem Rührwerk 35, sowie Sonden zur Messung der Temperatur 36 und des pH-Werts 37 ausgestattet ist, kann mit Hilfe einer Dosierpumpe 38 über Leitung 39 mit Substrat beschickt werden, welches einem Substratvorratsbehälter 40 entnommen wird. Das Substrat wird im Fermenter 34 fermentiert, wobei sich die bei der Fermentation freigesetzten Gase im Kopfraum des Fermenters 34 sammeln und von dort über Leitung 41 in einen Gassammelbehälter 42 abgeführt werden. Die Gasmenge kann über Gasuhr 43 gemessen werden. Verbrauchtes Substrat wird über Leitung 44 einem Ablauftrichter 45 zugeführt. Beim "Magnetfermenter MF" ist zusätzlich eine magnetische Abscheidevorrichtung 46 angeordnet, welcher das verbrauchte Substrat vom Ablauftrichter 45 aus zugeführt wird. In der magnetischen Abscheidevorrichtung 46 erfolgt eine Abtrennung der magnetisierbaren Trägerpartikel mit den darauf aufgewachsenen Mikroorganismen. Das abgereicherte Substrat wird dann einem Gärrestbehälter 47 zugeführt. Die aus dem verbrauchten Substrat abgetrennten magnetisierbaren Partikel können über Leitung 48 wieder in den Fermenter 34 zurückgeführt werden. Über Leitung 49 kann ferner mit Hilfe der Dosierpumpe 50 dem Fermenter 34 Wasser aus dem Wasservorlagebehälter 51 zugeführt werden. Die Substratzufuhr zum Fermenter 34 kann mit Hilfe einer Steuerung 52 gesteuert werden.

Beim Kontrollfermenter "MFK" wurde das verbrauchte Substrat direkt dem Gärrestbehälter 47 zugeführt.

Magnetisierbare Trägerpartikel

Für die Durchführung der Versuche wurden die wie in Beispiel 1 beschrieben hergestellten magnetisierbaren Trägerpartikel mit einer Korngröße von 0,1 - 0,3 mm eingesetzt.

Magnetseparator

Liquimag LM9-E-050-7. Hersteller S+S Separation and Sorting Technology GmbH, Regener Straße 130, 94513 Schönberg (2009).

Substrat

Als Substrat wurde Rübensilage eingesetzt, die mit einem Fleischwolf zerkleinert worden war.

Animpfmaterial

Zum Animpfen wurde ausgefaulte Impfgülle aus einem anderen Fermentationsreaktor verwendet.

Hilfsmittel

Spurenelementelösung, N-haltige Lösung mit $NH_4HCO_3 \cdot NH_4COONH_2$, S-haltige Lösung mit $Na_2SO_4$ zur Bekämpfung von Mangelzuständen.

Analytische Methoden

Für die Durchführung der während des Versuchs beobachteten Parameter wurden die folgenden Vorrichtungen und Methoden eingesetzt:

Gasmenge: Trommelgaszähler TG 05; Ritter Apparatebau,

Biogaszusammensetzung: Mehrkanalmessgerät SSM 6000; Pronova Analysentechnik

Trockensubstanz: gravimetrisch durch Trocknung bei 105°C bis zur Gewichtskonstanz;

organische Trockensubstanz (oTS): gravimetrisch durch Veraschung bei 550°C;

leichtflüchtige organische Säuren und leichtflüchtige Alkohole: gaschromatographische Bestimmung unter Verwendung eines FID durch Vergleich mit Retentionszeiten bekannter Verbindungen;

Milchsäure: HPLC mit RI-Detektor;

```
korrigierte organische Substanz = oTS + flüchtige Säuren +
flüchtige Alkohole + flüchtiger Anteil von Milchsäure;
```

Zucker: GC+RI-Detektor;

FOS/TAC (Verhältnis von flüchtigen organischen Säuren zu totalen anorganischen Carbonaten): Titration mit 0,05 M $H_2SO_4$, Nordmann-Methode;

$NH_4$-N: Überführung in $NH_3$, Wasserdampfdestillation, Rücktitration;

$N_{ges}$: DIN EN 25663: 1933-11;

C,N,S-Gehalt: Simultanbestimmung mit Elementaranalysator vario EL II;

P-Gehalt: nach Aufschluss nach DIN EN ISO 15681-2:2005-05;

Mikroskopie zur Beurteilung von Ausmaß und Qualität der Besiedlung der magnetisierbaren Trägerpartikel: Mikroskop Zeiss Axiostar plus FL, Kamera Zeiss AxioCam MRc5, Bildanalysesystem Zeiss AxioVision 4.8. Mikroskopische Verfahren Phasenkontrast (um die Partikel und ihre äußeren Umrisse sichtbar zu machen) und Auflicht-Fluoreszenz (1. Anregung des Nucleinsäure-Fluoreszenzfarbstoffs SYTO 13 mit blauem Licht, Emission von Grünfluoreszenz; 2. Anregung der spezifischen Cofaktoren der Methangasbakterien mit UV-Licht, Emission der blauen, türkisen, grünen oder gelben Autofluoreszenz im gesamten sichtbaren Spektralbereich). Die Probenahme für die Mikroskopie der magnetisierbaren Partikel erfolgte 1 Mal pro Monat. Bei Bedarf wurden zusätzlich die Kulturbrühen der Fermenter untersucht.

Versuchsdurchführung:

Die Versuchsdauer wurde in 2 Versuchszeiträume unterteilt.

1.Versuchszeitraum:

Der 1. Versuchszeitraum dauerte 14 Wochen.

Zu Versuchsbeginn (Tag 0) wurden beide Fermenter mit Impfgülle befüllt. Ab dem Tag 8 wurde manuell Rübensilage in den Fermenter gegeben. Am Tag 14 wurden nur beim MF 1 Gew.-% magnetisierbare Trägerpartikel, bezogen auf die Gesamtmasse des Fermenterinhalts, zugegeben. Während der gesamten, etwa siebenmonatigen Versuchsdauer wurden keine magnetisierbaren Trägerpartikel mehr nachdosiert. Die Soll-Temperatur der Reaktoren wurde auf 38,5-39°C eingestellt. Ab Beginn der fünften Woche wurde Rübensilage als Substrat kontinuierlich zudosiert, wobei die Raumbelastung stufenweise gesteigert wurde. Der 1. Versuchszeitraum diente zum Anfahren beider Fermenter.

Die magnetisierbaren Trägerpartikel erwiesen sich während des 1. Versuchszeitraums als mechanisch stabil.

Rechnerisch wurde ein Abtrenngrad von 92% für die magnetisierbaren Trägerpartikel ermittelt. Am Ende des 1. Versuchszeitraums waren von ursprünglich 1 Massenprozent magnetisierbaren Trägerpartikeln noch 0,7% im MF.

Die mikroskopische Analyse ergab, dass die magnetisierbaren Trägerpartikel langsam besiedelt wurden. Von Monat zu Monat nahm der Besiedlungsgrad allmählich zu, wobei die Bakterien zunächst geschützte Bereiche, also Ritzen, Vertiefungen, Stellen neben Oberflächenerhebungen der magnetisierbaren Trägerpartikel bevorzugten und erst später die exponierteren Oberflächenbereiche der magnetisierbaren Trägerpartikel besiedelten. Kurz vor Ende des 1. Versuchszeitraums besaßen die am besten besiedelten magnetisierbaren Trägerpartikel einen Oberflächenbedeckungsgrad von maximal 50-60%. Der überwiegende Anteil der magnetisierbaren Trägerpartikel wies nur einen dünnen, lückenhaften Bewuchs aus Einzelzellen oder kleinen Zellgruppen auf. Auch Fadenbakterien traten im Aufwuchs auf. Vollständig unbesiedelte magnetisierbare Trägerpartikel wurden nicht mehr detektiert. Jeder magnetisierbare Trägerpartikel trug auf seiner Oberfläche zumindest einzelne immobilisierte Bakterien. Im Aufwuchs konnten blau, türkis und gelb autofluoreszierende Bakterien zwischen nicht autofluoreszenten Bakterien nachgewiesen werden. Dies ist ein Hinweis darauf, dass ein Teil des Aufwuchses aus den erwünschten Methangasbakterien bestand. Insgesamt war die Besiedlung am Ende des 1. Versuchszeitraums noch so gering, dass eine deutliche Leistungssteigerung des MF gegenüber dem MFK noch nicht zu erwarten war.

Leistungsparameter von MF und MFK: Biogasmenge, Methananteil, Methanausbeute, pH-Wert, organische Säuren und FOS/TAC-Werte wurden zur Bewertung herangezogen. In den ersten Wochen verhielten sich beide Fermenter erwartungsgemäß fast identisch, weswegen auf die Darstellung verzichtet wird.

2. Versuchszeitraum

Der 2. Versuchszeitraum dauerte ca. 13,5 Wochen. Die Rübensilage aus dem ersten Versuchszeitraum wurde durch frische Rübensilage ersetzt, wobei im Unterschied zum ersten Versuchszeitraum kleinere Gebinde eingesetzt wurden, bei denen von Anfang an auf Luftabschluss und Kühlung geachtet wurde. Die Rübensilage wurde mit Wasser verdünnt, um die Pumpfähigkeit zu gewährleisten. Zusätzlich wurde Wasser in die Fermenter dosiert, um durch die verringerten mittleren Verweilzeiten den Einfluss der magnetisierbaren Trägerpartikel deutlicher sichtbar machen zu können. Außerdem sollte durch die Verringerung der Viskosität des Mediums die Abtrennung der magnetisierbaren Trägerpartikel erleichtert werden. Jeder Fermenter erhielt einen eigenen Umlaufthermostaten, damit bei gesunkenen Außentemperaturen die Soll-Temperatur auf 41°C erhöht werden konnte, um das evtl. Wachstum von Hefen zu unterdrücken. Die Raumbelastung wurde behutsam auf ca. 3,5 g oS/(1*d)gesteigert. Während der ersten 9 Wochen des 2. Versuchszeitraums konnten als hemmend geltende Konzentrationen von undissoziierter Essigsäure (2 g/l) bzw. Propionsäure (0,7 g/l) sicher unterschritten werden.

Im MFK wurde eine relativ starke Schaumbildung beobachtet, dem jedoch durch verstärktes Rühren zum Zerstören des Schaums entgegengewirkt werden konnte. Im MF bildete sich zwar auch Schaum durch Ausgasen von $CO_2$ beim Dosieren der sauren Rübensilage. Dieser Schaum war jedoch nicht so haltbar wie beim MFK. Flotierte magnetisierbare Trägerpartikel im Schaum führten zu einem schnelleren Platzen der Bläschen.

Nach Ablauf von etwa 7 Wochen hatten sich in den Fermentern stabile Bedingungen eingestellt. Die magnetisierbaren Trägerpartikel erwiesen sich weiterhin als stabil. Mit bloßem Auge konnte kein Bruchkorn entdeckt werden. Mikroskopisch konnten in einzelnen Flocken eingewachsene kleine magnetisierbare Trägerpartikel-Splitter gefunden werden. Sofern die Flocke aufgrund des eingeschlossenen Splitters im Magnetseparator abgeschieden werden kann, bedeutet dies keinen Verlust an magnetisierbaren Trägerpartikeln bzw. wertvoller Biomasse.

Der mittlere Abtrenngrad der magnetisierbaren Trägerpartikel betrug nur noch 33%. Es hatte sich ein zäher brauner Schaum gebildet und im Magnetseparator hatte sich durch Sedimentation Schlamm abgelagert, welcher auch magnetisierbare Trägerpartikel enthielt. Am Ende des zweiten Versuchszeitraums wurde ein Gehalt von 0,41 Gew.-% magnetisierbare Trägerpartikel im Fermenter gemessen - das sind nur noch 41% von den ursprünglich eingesetzten 1 Gew.-%. Diese hohen Verluste sind auf einen noch nicht optimierten Magnetseparator sowie die noch nicht optimalen Eigenschaften der magnetisierbaren Trägerpartikel zurückzuführen.

Die erste mikroskopische Untersuchung 4 Wochen nach Beginn des 2. Versuchszeitraums zeigte, dass der Bewuchs auf den magnetisierbaren Trägerpartikeln trotz der zahlreichen Milieu-Änderungen erhalten geblieben war und insgesamt sogar geringfügig zugenommen hatte. Allerdings fiel die Zunahme der immobilisierten Biomasse auf den magnetisierbaren Trägerpartikeln deutlich geringer aus als in allen bis dahin untersuchten Intervallen.

Eine mikroskopische Untersuchung etwa 6 Wochen nach Beginn des zweiten Versuchszeitraums zeigte, dass die Besiedlung der magnetisierbaren Trägerpartikel wieder kräftiger zugenommen hatte. Der am besten besiedelte Partikel wies einen Bedeckungsgrad von 90% und einen stellenweise bis zu 42 μm dicken Biofilm mit ausgeprägter Matrix aus extrazellulären polymeren Substanzen auf und beinhaltete zahlreiche, kräftig gelb autofluoreszierende Methangasbakterien. Der überwiegende Anteil der magnetisierbaren Trägerpartikel wies eine "Ritzenbesiedlung" bzw. einen dünnen, lückenhaften Bewuchs aus Einzelzellen, kleinen Zellgruppen und Fadenbakterien auf. Der überwiegende Anteil der magnetisierbaren Trägerpartikel wies eine Oberflächenbedeckung von etwa 30%-70% auf. Es wurden jedoch auch magnetisierbare Trägerpartikel beobachtet, welche einen geringeren Bewuchs aufwiesen. Erst bei einer mikroskopischen Untersuchung etwa 2 Wochen vor Ende des 2. Versuchszeitraums konnten hinreichend gut entwickelte Biofilme nachgewiesen werden. Die Besiedlungsdichte hatte im Vergleich zum vorhergehenden mikroskopischen Untersuchung deutlich zugenommen. Bei den am besten besiedelten magnetisierbaren Trägerpartikeln waren gut 90% der Oberfläche bedeckt. Ein deutlicher Anteil der magnetisierbaren Trägerpartikel wies einen Besiedlungsgrad von etwa 70-90% auf. Der überwiegende Anteil der magnetisierbaren Trägerpartikel wies einen Besiedlungsgrad von etwa 30%-70% Oberflächenabdeckung auf. Es wurden keine magnetisierbaren Trägerpartikel mehr beobachtet, die keine Besiedlung aufwiesen. Die Biofilme hatten an Dicke und Komplexität zugenommen. Erstmals hatten sich hoch organisierte Bakterienkolonien in Pilz- oder Turmform gebildet. Auch die flächendeckenden, matrixumhüllten, gemischten Biofilme (mit kräftig autofluoreszenten Methangasbakterien) sowie Fadenbakterien hatten zugenommen. Zum Ende des zweiten Versuchszeitraums hatte sich dem mikroskopischen Bild nach zu urteilen zumindest auf einem Teil der magnetisierbaren Trägerpartikel ein leistungsfähiger Biofilm entwickelt.

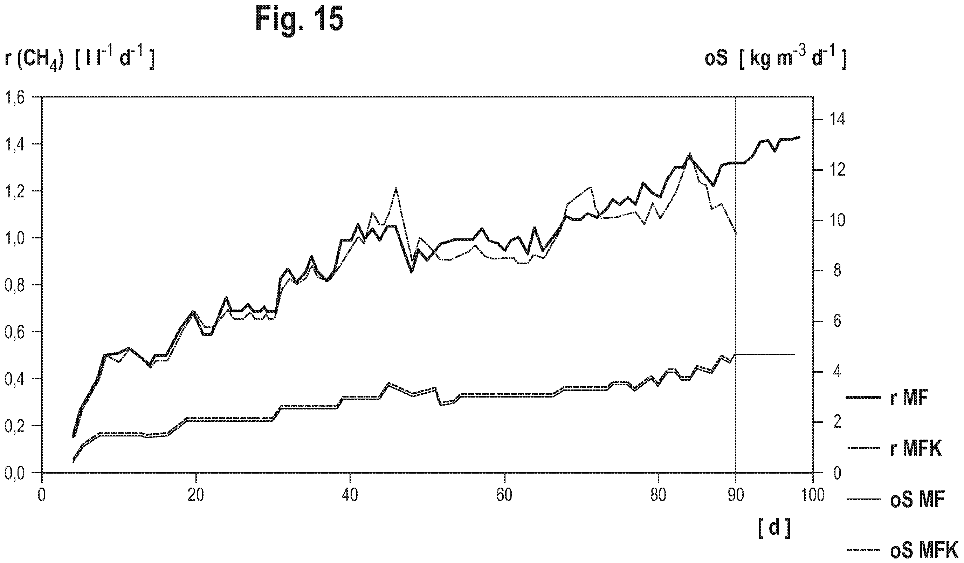

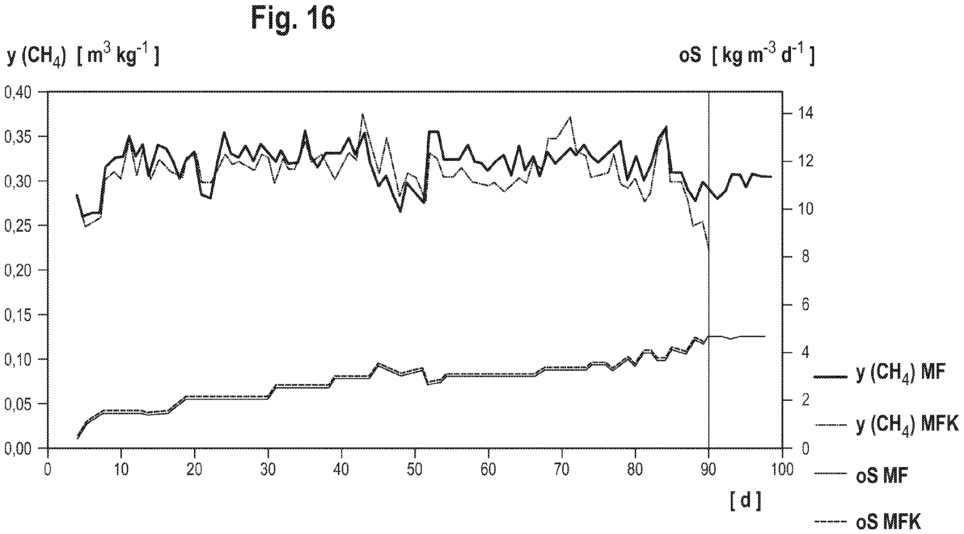

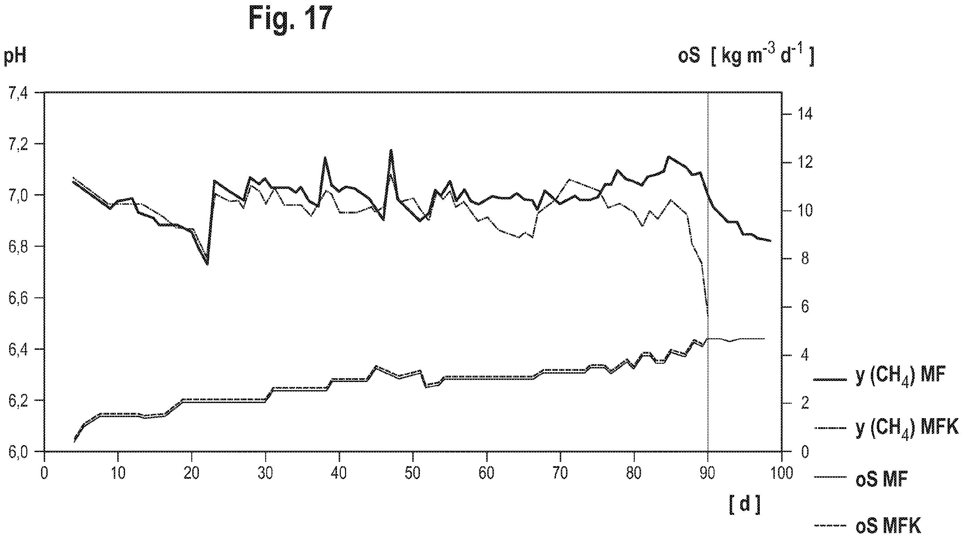

In den Figuren 15 bis 17 sind Methanbildungsrate [r(CH$_4$)], Methanausbeute [y(CH$_4$)] und pH-Wert in Bezug zur Raumbelastung [oS] während des gesamten 2. Versuchszeitraums für die Reaktoren MF und MFK dargestellt. Die Raumbelastung wurde allmählich (stufenweise) erhöht. Methanbildungsrate und Methanausbeute verliefen in beiden Reaktoren über einen langen Zeitraum recht ähnlich. Die Methanbildung stieg erwartungsgemäß mit Erhöhung der Raumbelastung an. Die Methanausbeute schwankte um einen Wert von gut 0,3 m$^3$ Methan pro kg organischer Substanz, wobei Spitzenwerte von 0,36 (MF) und 0,37 (MFK) erreicht wurden.

Etwa zwei Monate nach Beginn des 2. Versuchszeitraums und bei einer Raumbelastung von 3 kg m$^{-3}$d$^{-1}$ bis zum Ende des dritten Versuchsmonats im 2. Versuchszeitraum zeichnete sich die Tendenz ab, dass sowohl Methanbildungsrate als auch Methanausbeute des MF bis auf wenige Ausnahmen geringfügig höher lagen als beim MFK.

Erst bei höheren Raumbelastungen wurden deutliche Unterschiede erkennbar. Ab der Erhöhung der Raumbelastung auf 4,17 kg m$^{-3}$d$^{-1}$ sanken die Werte des MFK stark ab. Um eine irreversible Prozessüberlastung zu vermeiden, wurde die Substratzugabe des MFK gut drei Monate nach Versuchsbeginn eingestellt. Zu diesem Zeitpunkt waren die Methanbildungsrate und die Methanausbeute des MFK im Vergleich zum MF um mehr als 20% geringer.

Ein Vergleich mit der Entwicklung der pH-Werte zeigt die biologische Instabilität des MFK auf. Hydrolysierende, acidogene und acetogene Bakterien senken durch ihre Stoffwechselaktivität den pH-Wert ab und stellen den methanogenen Bakterien die Substrate ($H_2$, $CO_2$, Acetat, ...) zur Verfügung. Die methanogenen Bakterien am Ende der Stoffwechselkette metabolisieren diese niedermolekularen Substanzen und erhöhen durch ihre Stoffwechselaktivität den pH-Wert. In Fermentern mit Mischbiozönosen aus den 4 genannten Bakteriengruppen stellen sich im Gleichgewichtszustand üblicherweise pH-Werte zwischen 7 und 8 ein. Als unterer Grenzwert für einen ungestörten Methanbildungsprozess gilt ein pH-Wert von 6,7. Unterhalb von pH 6,7 ist der Anteil der protonierten, flüchtigen organischen Säuren so hoch, dass die undissoziierten organischen Säuren die Methanbakterien hemmen. Eine Hemmung der methanogenen Bakterien führt zu einer verringerten Beseitigung von Acidität bzw. zu einer verringerten Bildung von Alkalität. Dadurch sinkt der pH-Wert weiter ab, wodurch die Hemmwirkung der flüchtigen organischen Säuren auf die methanogenen Bakterien sich verstärkt und ein Teufelskreis in Gang gesetzt wird, der ohne Gegenmaßnahmen bis zum völligen Zusammenbruch des Systems führen kann.

Unter stabilen Betriebsbedingungen hatte sich in MF und MFK ein pH-Wert von 7,0 oder etwas höher eingestellt. Schon ab einer Raumbelastung von ca. 3,5 kg $m^{-3}d^{-1}$ begann der pH-Wert des MFK ganz allmählich abzusinken, was als Anzeichen für eine leichte, schleichende Überlastung gewertet werden kann. Bei der weiteren Steigerung der Raumbelastung auf ca. 4 kg $m^{-3}d^{-1}$ fiel der pH-Wert rapide bis auf 6,52 ab. Der Versuchsreaktor mit den magnetisierbaren Trägerpartikeln (MF) vertrug diese Belastungssteigerung deutlich besser. Raumbelastungen von um die 3,5 kg $m^{-3}d^{-1}$ und etwas mehr bewirkten kein allmähliches Absinken des pH-Werts, sondern der pH-Wert blieb stabil bzw. stieg sogar allmählich etwas an. Bei Raumbelastungen oberhalb von 4 kg $m^{-3}d^{-1}$ kam es im MF zwar zunächst auch zu einer Verringerung der Methanausbeute und einem Absinken des pH-Werts, aber der Reaktor erholte sich wieder. Der pH-Wert näherte sich asymptotisch einem unteren Grenzwert an. Demnach ist davon auszugehen, dass sich der MF biologisch stabilisierte und mit dieser Raumbelastung kontinuierlich weiter betrieben werden könnte.

Die beobachteten Unterschiede lassen sich so erklären: ab einer bestimmten Raumbelastung überschritt im MFK die Auswaschrate die Wachstumsrate der langsam wachsenden Methangasbakterien, so dass diese mit dem Ablauf ausgeschwemmt wurden. Die schneller wachsenden, anderen Bakteriengruppen (säurebildend) konnten sich jedoch noch im System halten, so dass es zu einem allmählichen Absinken des pH-Werts und allen Folgeerscheinungen kam. Im MF dagegen wurden die auf den magnetisierbaren Trägerpartikeln immobilisierten Methangasbakterien wieder in den Fermenter zurückgeführt, so dass es bei einer Raumbelastung von 4,17 kg $m^{-3}d^{-1}$ noch nicht zum Mangel an Methangasbakterien kam.

Die Konzentrationen flüchtiger Fettsäuren erlauben ergänzende Aussagen zur Prozessstabilität. Die Konzentrationen an freien Fettsäuren bestätigen im Wesentlichen die bereits aus Methanbildung, Methanausbeute und pH-Wert abgeleiteten Schlussfolgerungen. So lagen die Säurekonzentrationen im Reaktorinhalt des MFK bei höheren Raumbelastungen deutlich über denen des MF.

Beispiel 4: Screening-Versuche zur Untersuchung verschiedener magnetischer Träger

Es wurden einfache Screening-Versuche mit unterschiedlichen magnetisierbaren Trägerpartikeln aus einem Schaumglas durchgeführt, um den Einfluss verschiedener Faktoren auf die Ausbildung eines Biofilms zu untersuchen.

Versuchsdurchführung:

Die etwa 4 Monate dauernden Versuche wurden in einer Klimakammer bei 39°C durchgeführt. Als Bioreaktoren dienten Gärballons aus PE mit einem Nennvolumen von 30 l, die an Gassammelsäcke angeschlossen wurden. Das Soll-Füllvolumen betrug 15 l. Zu Versuchsbeginn wurden je 14 l abgekochtes und mit Dithionit reduzierend eingestelltes Leitungswasser vorgelegt. Dann wurden je 500 ml magnetisierbare Partikel hinzugefügt. Beim Kontrollansatz ohne magnetisierbare Partikel wurde ein entsprechendes Wasser-Volumen zugegeben. Anschließend wurde mit etwa 500 ml Animpfmaterial angeimpft. Um eine möglichst große Vielfalt an Mikroorganismen, insbesondere an Methangasbakterien und Erstbesiedlern zu gewährleisten, stammte das Animpfmaterial aus 3 unterschiedlichen Quellen. Zu 2 späteren Terminen bei höheren Raumbelastungen wurde nochmals nachgeimpft. Als Substrat diente Zuckerrübensilage. Die Raumbelastung wurde langsam linear gesteigert (von 0,0 auf 1,0 g oTS/(l*d) in 90 Tagen. Am Ende wurde eine maximale Raumbelastung von ca. 1,1 g oTS/(l*d) erreicht.

Die Beschreibungen der für die Versuche verwendeten magnetisierbaren Träger sind in Tabelle 11 zusammengefasst.

Tabelle 11: Beschreibung der magnetisierbaren Träger

| Nr. | magnetisierbarer Trägers |
|-----|--------------------------|
| 1 | Magnetisches Schaumglas, Sprühkorn, 0,1-0,3 mm D |
| 2 | Magnetisches Schaumglas, Bruchkorn, 0,25-1,5 mm D |
| 3 | Magnetisches Schaumglas, Bruchkorn 0,25-1,5 mm D, mit Xanthan beschichtet |
| 4 | Magnetisches Schaumglas, Bruchkorn 0,25-1,5 mm D, mit Xanthan und Spurenelementen beschichtet |
| 5 | Magnetisches Schaumglas, Bruchkorn 0,25-1,5 mm D, mit Gelatine beschichtet |
| 6 | Magnetisches Schaumglas, Bruchkorn 0,25-1,5 mm D, mit anorganischer chemischer Oberflächenmodifizierung |
| 7 | Magnetisches Schaumglas, Bruchkorn 0,25-1,5 mm D, reduzierend erschmolzene Körnung |
| 8 | Magnetisches Schaumglas, Bruchkorn 0,25-1,5 mm D, alternatives Blähmittel |
| 9 | $Fe_2O_3$-Granulat pur, ungebläht |
| 10 | Schaumglas aus der Serienproduktion, vom Magnetabscheider, 0,5-1,0 mm D |
| 11 | Referenz ohne magnetische Partikel |

Analytik:

Das Ausmaß der Besiedlung wurde fluoreszenzmikroskopisch untersucht und fotografisch dokumentiert (6 Probenahmetermine). Die Auswertung erfolgte subjektiv nach visuellem Eindruck. Zur Prozesskontrolle wurden Redoxpotential, pH-Wert und FOS/TAC-Werte bestimmt. Das gebildete Gärgasvolumen wurde durch Abschätzung der Ausdehnung der Gassammelsäcke bestimmt. An Stichproben wurde die Gaszusammensetzung analysiert.

Ergebnisse:

Die wichtigsten Ergebnisse sind in Tabelle 11 zusammengefasst.

Die Menge des Gärgasvolumens wurde durch Abschätzen des Füllgrades des Gassammelsacks bestimmt und in die Kategorien 0, +, ++ eingeteilt.

Der Methananteil im Biogas wurde jeweils 4 Wochen nach Animpfen bestimmt.

Die Besiedlung der magnetisierbaren Träger wurde anhand lichtmikroskopischer Aufnahmen bestimmt und in die Kategorien 0, +, ++, +++ und ++++ eingeteilt. 0 bezeichnet dabei keine Besiedlung und ++++ die Ausbildung eines weitgehend kontinuierlichen Biofilms.

Tabelle 11: Zusammenfassung der Ergebnisse der Screening-Versuche

| Nr. | Gärgasvolumen | Methananteil [%] | Effekte | Besiedlung, |
|-----|---------------|------------------|---------|-------------|
| 1 | + | 49, 9 | Tendenz zu Flotation | + |
| 2 | ++ | 45,3 | Tendenz zu Sedimentation | ++ |
| 3 | ++ | Nicht bestimmt | Tendenz zu Sedimentation | +++ |
| 4 | ++ | 49,7 | Tendenz zu Sedimentation | ++++ |
| 5 | +, viele Leckagen | Nicht bestimmt | Tendenz zu Sedimentation | +++ |
| 6 | + | Nicht bestimmt | Tendenz zu Sedimentation | +++ |
| 7 | ++ | Nicht bestimmt | Tendenz zu Sedimentation | ++ (+) |
| 8 | ++ | 51,8 | Tendenz zu Sedimentation | ++++ |

(fortgesetzt)

| Nr. | Gärgasvolumen | Methananteil [%] | Effekte | Besiedlung, |
|---|---|---|---|---|
| 9 | Versuchsabbruch nach wenigen Wochen wegen Zerfall des Granulats | 48,7 | $Fe_2O_3$ wurde zu FeS reduziert. Das Granulat zerfiel zu feinst-verteiltem, schwarzen, völlig unmagnetischen FeS. Positiv: $H_2S$-Bildung war unterdrückt. | Nicht anwendbar |
| 10 | ++ | Nicht bestimmt | Beste Verteilung in der Kulturbrühe; Schweben | ++ |
| 11 | Leckagen über mehrere Wochen | Nicht bestimmt | Nicht anwendbar | Nicht anwendbar |

Die Gasanalytik an Stichproben belegt, dass tatsächlich eine Methangasgärung stattfand.

Bei der maximal erreichten Raumbelastung von 1,1 g oTS/l(*d) wären theoretisch 12,9 Normliter Biogas/d zu erwarten gewesen. Der höchste praktisch erreichte Wert von ca. 12 l Biogas/d (nicht normiert) zeigt, dass das Substrat weitgehend umgesetzt wurde. Die pH-Werte und FOS/TAC-Werte lagen am Ende der Versuchsperiode in einem guten Bereich (pH 7-8 und FOS/TAC < 0,3, oft im als optimal erachteten Bereich zwischen 0,2 und 0,3).

Besiedlung: wenige Stunden nach Animpfen ließ sich bereits die reversible Adhäsion beobachten. Im Lauf von 12 Wochen nach dem Animpfen nahm das Ausmaß der Besiedlung in allen Proben (außer Ansatz 9) allmählich zu. Nach 12 Wochen waren die Biofilme in allen Proben am besten ausgeprägt. Besonders positiv fielen die mit ++++ gekennzeichneten Proben auf. Überraschenderweise lieferte die reduzierend erschmolzene Körnung ebenso gute Ergebnisse wie die oxidierend erschmolzenen Körnungen.

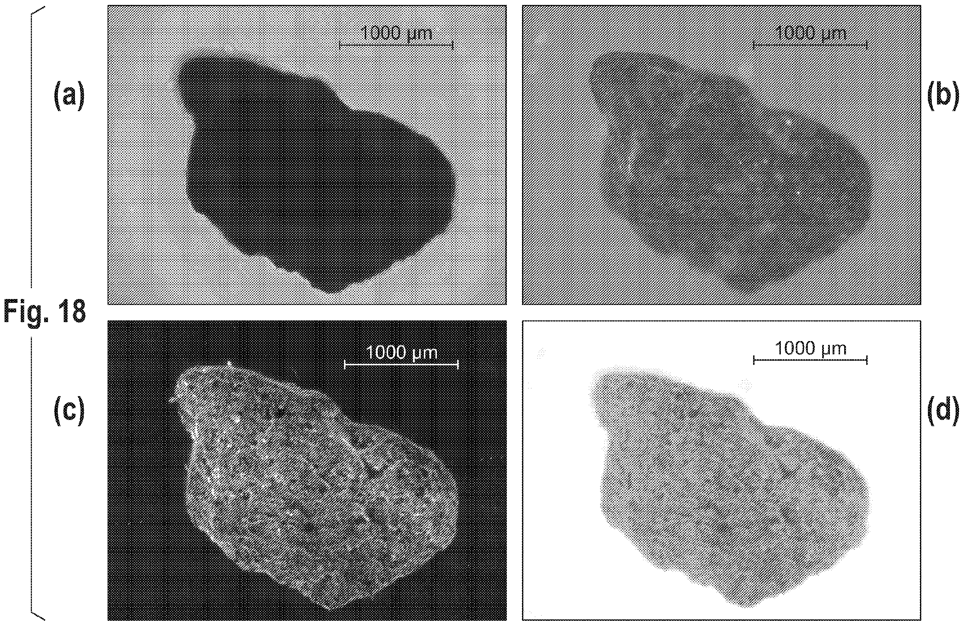

In Figur 18 sind lichtmikroskopische Aufnahmen eines einzelnen großen magnetischen Schaumglaspartikels (Probe 4, beschichtet mit Xanthan und Spurenelementen) nach 12 Wochen Inkubationszeit gezeigt. Fig. 18A zeigt ein Einzelbild, das Phasenkontrastverfahren aufgenommen wurde. Erkennbar sind die äußeren Umrisse des dunklen Partikels. Der den Partikel umhüllende Biofilm ist auf dem Umriss andeutungsweise zu erkennen. Auffällig sind insbesondere drei vom Partikel wegstehende Kolonien.

Fig. 18B zeigt ein Einzelbild, das im Auflicht-Fluoreszenzverfahren aufgenommen wurde. Dabei wurde mit UV-Anregung gearbeitet und Emission über das gesamte visuelle Spektrum aufgenommen. Erkennbar sind extrem hell fluoreszierende Cellulose-Fasern und cellulosehaltige Pflanzenteile. Diese zeigen in den Originalaufnahmen eine hellblaue Fluoreszenz. Etwas weniger hell erscheinende Bereiche, welche in der Originalaufnahme intensiv blau, intensiv gelb oder türkis bzw. grünlich fluoreszieren, entsprechen Kolonien von Methangasbakterien. Schwach hell erscheinende Bereiche, welche in der Originalaufnahme matt blau fluoreszieren, entsprechen extrazellulären polymeren Substanzen der Bakterienkolonien mit ihren Einschlüssen. Sie bilden die wolkigen hellen Stellen auf der Oberfläche des Magnetpartikels. Die Hintergrundfluoreszenz (Autofluoreszenzen von Objektträger, Deckgläschen, Puffer und darin gelösten bzw. feinstverteilten Substanzen usw.) ist beträchtlich und lässt den Hintergrund grau bzw. in der Originalaufnahme blaugrau und nicht tiefschwarz aussehen. Absolut keine Autofluoreszenz besitzt das magnetische Schaumglaspartikel, sodass unbesiedelte bzw. nur dünn besiedelte Stellen schwarz erscheinen.

Fig. 18C zeigt eine Auflicht-Fluoreszenzaufnahme mit Blau-Anregung und Emission im Grünen, welche auf der Aufnahme als helle Bereiche zu erkennen ist. Das Bild ist aus einem Z-Stapel berechnet und zeigt daher eine erweiterte Tiefenschärfe. Die Färbung mit einem Nucleinsäure-Fluoreszenzfarbstoff lässt DNA und RNA kräftig grün fluoreszieren, sodass alle Mikroorganismen im Biofilm deutlich sichtbar werden. Das magnetische Schaumglaspartikel wurde rundum besiedelt. Der Biofilm ist fast flächendeckend, aber noch dünn. Lediglich im Bereich von Vertiefungen und Poren befinden sich nur wenige Bakterien.

Fig. 18D zeigt ein Mischbild der 3 vorhergehenden Kanäle (Addition von A + B + C).

Bezugszeichenliste

| | | | |
|---|---|---|---|
| 1 | magnetisierbarer Träger | 27 | magnetischer Abscheider |
| 2 | kontinuierliche Glasphase | 28 | magnetische Abscheidevorrichtung |
| 3 | geschlossene Pore | 29 | Leitung |
| 4 | magnetischer Partikel | 30 | Vorratsbehälter |
| 5 | Außenseite | 31 | Leitung |
| 6 | Bruchfläche | 32 | Ablaufbecken |
| 7 | Vertiefung | 33 | Ventil |

(fortgesetzt)

| 8 | Grat | 34 | Fermenter |
|---|---|---|---|
| 9 | Kern | 35 | Rührwerk |
| 10 | Mikroorganismus | 36 | Thermometer |
| 11 | Kolonie | 37 | pH-Meter |
| 12 | Biofilm | 38 | Dosierpumpe |
| 13 | Enzym | 39 | Leitung |
| 14 | Spacer | 40 | Substratvorratsbehälter |
| 15 | Vesikel | 41 | Leitung |
| 16 | Polymerschicht | 42 | Gassammelbehälter |
| 17 | Affinitätsgruppe | 43 | Gasuhr |
| 18 | Zielmolekül | 44 | Leitung |
| 19 | Antikörper | 45 | Ablauftrichter |
| 20 | DNA-Sonde | 46 | magnetischer Abscheider |
| 21 | Ziel-DNA-Sequenz | 47 | Gärrestbehälter |
| 22 | Rührkesselreaktor | 48 | Leitung |
| 23 | Heizung | 49 | Leitung |
| 24 | Rührwerk | 50 | Dosierpumpe |
| 25 | Ablauf | 51 | Wasservorlagebehälter |
| 26 | Leitung | 52 | Steuerung |

## Patentansprüche

1. Verfahren zur Behandlung eines organischen und/oder anorganischen Substrats, wobei

- in einem Reaktionsraum eine Substratmischung bereitgestellt wird, welche das organische und/oder anorganische Substrat enthält,
- die Substratmischung mit einem magnetisierbaren Aggregat versetzt wird, wobei das magnetisierbare Aggregat einen magnetisierbaren Träger und eine auf dem magnetisierbaren Träger immobilisierte aktive Komponente umfasst;
- die Substratmischung mit dem magnetisierbaren Aggregat zu einer Produktmischung umgesetzt wird, und
- das magnetisierbare Aggregat mit einer magnetischen Abscheidevorrichtung von der Produktmischung abgetrennt wird,

dadurch gekennzeichnet, dass der magnetisierbare Träger in Form eines partikulären magnetisierbaren Trägers vorliegt, wobei der partikuläre magnetisierbare Träger aus einem festen Schaum mit einer kontinuierlichen Phase aus einem anorganischen Material, nämlich einem Glas aufgebaut ist, welche Poren des festen Schaums umgibt, wobei in der kontinuierlichen Phase magnetisierbare Bereiche angeordnet sind, und wobei zumindest im Kern des magnetisierbaren Trägers der feste Schaum geschlossenporig ist.

2. Verfahren nach Anspruch 1, dadurch gekennzeichnet, dass die Produktmischung in eine magnetische Abscheidevorrichtung überführt wird, in welcher das magnetisierbare Aggregat von der Produktmischung abgetrennt wird.

3. Verfahren nach einem der vorhergehenden Ansprüche, dadurch gekennzeichnet, dass das von der Produktmischung abgetrennte magnetisierbare Aggregat in den Reaktionsraum zurückgeführt wird.

4. Verfahren nach einem der vorhergehenden Ansprüche, dadurch gekennzeichnet, dass die aktive Komponente ein biokatalytisch aktives System ist.

5. Verfahren nach Anspruch 4, dadurch gekennzeichnet, dass das biokatalytisch aktive System durch zumindest einen Mikroorganismus gebildet ist.

6. Verfahren nach Anspruch 5, dadurch gekennzeichnet, dass der zumindest eine Mikroorganismus in Form eines Biofilms bereitgestellt ist.

**7.** Verfahren nach einem der vorhergehenden Ansprüche, **dadurch gekennzeichnet, dass** der partikuläre magnetisierbare Träger auf seiner Oberfläche Grate und Vertiefungen aufweist.

**8.** Verfahren nach einem der vorhergehenden Ansprüche, **dadurch gekennzeichnet, dass** der partikuläre magnetisierbare Träger eine magnetische Massensuszeptibilität im Bereich von 5 x $10^{-9}$ bis 3,7 x $10^{-7}$ $m^3$/kg aufweist.

**9.** Verfahren nach Anspruch 5, **dadurch gekennzeichnet, dass** die Umsetzung der Substratmischung durch den zumindest einen Mikroorganismus zu einer Produktmischung unter anaeroben Bedingungen durchgeführt wird.

**10.** Magnetisierbares Aggregat, umfassend einen partikulären magnetisierbaren Träger, auf dessen Oberfläche zumindest eine aktive Komponente immobilisiert ist,
**dadurch gekennzeichnet, dass** der partikuläre magnetisierbare Träger aus einem festen Schaum mit einer kontinuierlichen Phase aufgebaut ist, welche aus einem Netzbildner in Form eines anorganischen Materials, nämlich eines Glases aufgebaut ist und welche Poren des festen Schaums umgibt, wobei in der kontinuierlichen Phase magnetisierbare Bereiche angeordnet sind, und wobei zumindest im Kern des magnetisierbaren Trägers der feste Schaum geschlossenporig ist.

**11.** Magnetisierbares Aggregat nach Anspruch 10, **dadurch gekennzeichnet, dass** der partikuläre magnetisierbare Träger eine Kornrohdichte von weniger als 2 g/ml aufweist.

**12.** Verfahren zur Herstellung eines magnetisierbaren Aggregats nach einem der Ansprüche 10 oder 11, wobei ein Netzbildner in Form eines anorganischen Materials, nämlich eines Glases, ein magnetisierbares Material sowie ein Blähmittel zu einem Granulat verarbeitet werden, das Granulat zu einem magnetisierbaren Träger gebläht wird und auf dem magnetisierbaren Träger eine aktive Komponente immobilisiert wird.

**13.** Verfahren nach Anspruch 12, wobei der magnetisierbare Träger zum Aufrauen seiner Oberfläche gebrochen wird, sodass ein magnetisierbarer Träger erhalten wird, dessen Kern aus einem geschlossenporigen Schaum gebildet ist und auf der Oberfläche des magnetisierbaren Trägers zumindest abschnittsweise Grate und Vertiefungen bereitgestellt werden.

**Claims**

**1.** Method for treating an organic and/or inorganic substrate, wherein

- a substrate mixture is provided in a reaction chamber, said substrate mixture containing the organic and/or inorganic substrate,
- the substrate mixture is mixed with a magnetisable aggregate, wherein the magnetisable aggregate comprises a magnetisable carrier and an active component which is immobilised on the magnetisable carrier;
- the substrate mixture with the magnetisable aggregate is converted into a product mixture, and
- the magnetisable aggregate is separated from the product mixture using a magnetic separating device,

**characterised in that** the magnetisable carrier is present in the form of a particulate magnetisable carrier, wherein the particulate magnetisable carrier is formed from a solid foam with a continuous phase made from an inorganic material, in particular a glass, which surrounds pores of the solid foam, wherein magnetisable regions are arranged in the continuous phase, and wherein the solid foam has closed pores at least in the core of the magnetisable carrier.

**2.** Method according to claim 1, **characterised in that** the product mixture is transferred to a magnetic separating device, in which the magnetisable aggregate is separated from the product mixture.

**3.** Method according to one of the preceding claims, **characterised in that** the magnetisable aggregate which is separated from the product mixture is led back to the reaction chamber.

**4.** Method according to one of the preceding claims, **characterised in that** the active component is a biocatalyticallyally active system.

**5.** Method according to claim 4, **characterised in that** the biocatalytically active system is formed by at least one microorganism.

**6.** Method according to claim 5, **characterised in that** the at least one microorganism is provided in the form of a biofilm.

**7.** Method according to one of the preceding claims, **characterised in that** the particulate magnetisable carrier has ridges and indentations on its surface.

**8.** Method according to one of the preceding claims, **characterised in that** the particulate magnetisable carrier has a magnetic mass susceptibility ranging from 5 x $10^{-9}$ to 3.7 x $10^{-7}$ m$^3$/kg.

**9.** Method according to claim 5, **characterised in that** the conversion of the substrate mixture into a product mixture by means of the at least one microorganism is carried out under anaerobic conditions.

**10.** Magnetisable aggregate, comprising a particulate magnetisable carrier, on the surface of which at least one active component is immobilised,
**characterised in that** the particulate magnetisable carrier is formed from a solid foam with a continuous phase which is formed from a network builder in the form of an inorganic material, in particular a glass and which surrounds pores of the solid foam, wherein magnetisable regions are arranged in the continuous phase, and wherein the solid foam has closed pores at least in the core of the magnetisable carrier.

**11.** Magnetisable aggregate according to claim 10, **characterised in that** the particulate magnetisable carrier has a grain gross density of less than 2 g/ml.

**12.** Method for the production of a magnetisable aggregate according to one of claims 10 to 11, wherein a network builder in the form of an inorganic material, in particular a glass, a magnetisable material and a blowing agent are processed into a granulate, wherein the granulate is blown into a magnetisable carrier and an active component is immobilised on the magnetisable carrier.

**13.** Method according to claim 12, wherein the magnetisable carrier is broken down to roughen its surface such that a magnetisable carrier is obtained, the core of which is formed from a foam with closed pores and ridges and indentations are provided on the surface of the magnetisable carrier at least in sections.

**Revendications**

**1.** Procédé pour le traitement d'un substrat organique et/ou inorganique, où

- il est préparé dans un espace de réaction un mélange de substrat, lequel contient le substrat organique et/ou inorganique,
- le mélange de substrat est additionné d'un agrégat magnétisable, l'agrégat magnétisable comprenant un support magnétisable et un constituant actif immobilisé sur le support magnétisable ;
- le mélange de substrat réagit avec l'agrégat magnétisable en un mélange de produit, et
- l'agrégat magnétisable est séparé du mélange de produit avec un dispositif de séparation magnétique,

**caractérisé en ce que** le support magnétisable est présent dans la forme d'un support magnétisable particulaire, le support magnétisable particulaire étant constitué d'une mousse solide avec une phase continue constituée d'un matériau inorganique, plus précisément d'un verre, laquelle entoure des pores de la mousse solide, des domaines magnétisables étant disposés dans la phase continue, et la mousse solide étant à pores fermés au moins dans le noyau du support magnétisable.

**2.** Procédé selon la revendication 1, **caractérisé en ce que** le mélange de produit est transféré dans un dispositif de séparation magnétique, dans lequel l'agrégat magnétisable est séparé du mélange de produit.

**3.** Procédé selon l'une quelconque des revendications précédentes, **caractérisé en ce que** l'agrégat magnétisable séparé du mélange de produit est renvoyé dans l'espace de réaction.

**4.** Procédé selon l'une quelconque des revendications précédentes, **caractérisé en ce que** le constituant actif est un système biocatalytiquement actif.

**5.** Procédé selon la revendication 4, **caractérisé en ce que** le système biocatalytiquement actif est formé par au moins un micro-organisme.

6. Procédé selon la revendication 5, **caractérisé en ce que** le au moins un micro-organisme est préparé dans la forme d'un biofilm.

7. Procédé selon l'une quelconque des revendications précédentes, **caractérisé en ce que** le support magnétisable particulaire présente sur sa surface des arêtes et des creux.

8. Procédé selon l'une quelconque des revendications précédentes, **caractérisé en ce que** le support magnétisable particulaire présente une susceptibilité massique magnétique dans le domaine de 5 x $10^{-9}$ à 3,7 x $10^{-7}$ m$^3$/kg.

9. Procédé selon la revendication 5, **caractérisé en ce que** la réaction du mélange de substrat est réalisée par le au moins un micro-organisme en un mélange de produit dans des conditions anaérobies.

10. Agrégat magnétisable, comprenant un support particulaire magnétisable, sur la surface duquel au moins un constituant actif est immobilisé, **caractérisé en ce que** le support magnétisable particulaire est constitué d'une mousse solide avec une phase continue, laquelle est constituée d'un agent de réticulation dans la forme d'un matériau inorganique, précisément d'un verre et laquelle entoure des pores de la mousse solide, des domaines magnétisables étant disposés dans la phase continue, et la mousse solide étant à pores fermés au moins dans le noyau du support magnétisable.

11. Agrégat magnétisable selon la revendication 10, **caractérisé en ce que** le support magnétisable particulaire présente une masse volumique apparente de grain inférieure à 2 g/ml.

12. Procédé pour la préparation d'un agrégat magnétisable selon l'une quelconque des revendications 10 ou 11, un agent de réticulation dans la forme d'un matériau inorganique, précisément d'un verre, un matériau magnétisable ainsi qu'un agent gonflant étant transformés en un granulé, le granulé étant gonflé en un support magnétisable et un constituant actif étant immobilisé sur le support magnétisable.

13. Procédé selon la revendication 12, où le support magnétisable est cassé pour rendre sa surface rugueuse, de sorte qu'il est obtenu un support magnétisable dont le noyau est formé d'une mousse à pores fermés et des arêtes et des creux étant préparés au moins par portions sur la surface du support magnétisable.

**Fig. 1a**

**Fig. 1b**

Fig. 2a

Fig. 2b

Fig. 2c

Fig. 3a

13a

13

1

Fig. 3b

13

14

3

4

Fig. 3c

13

14

1

Fig. 3d

13

15

Fig. 3e

17

18

16

1

Fig. 3f

19

18

Fig. 3g

19

18

Fig. 3h

21

20

14

Fig. 4

# Fig. 5

# Fig. 6

## Fig. 7

## Fig. 8

# Fig. 9

# Fig. 10

Fig. 11

Fig. 12

## Fig. 13

Fig. 14

# Fig. 15

r (CH$_4$) [ l l$^{-1}$ d$^{-1}$]

oS [ kg m$^{-3}$ d$^{-1}$]

— r MF

—·— r MFK

—— oS MF

----- oS MFK

[ d ]

EP 2 603 577 B1

Fig. 16

Fig. 17

Fig. 18

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102005024886 B3
- WO 0171732 A
- DE 19531801 A1
- DE 19734791 A1
- DE 19817268 A1
- EP 1900698 A1
- EP 1900697 A1
- US 5734020 A
- WO 2004113245 A
- DE 102004012598 A1
- EP 2022768 A2
- WO 2006092153 A1
- WO 9310162 A1
- WO 2007093138 A2

**In der Beschreibung aufgeführte Nicht-Patentliteratur**

- **M.-H. LIAO ; B.-H. CHEN.** *Biotechnology Letters,* 2002, vol. 24, 1913-1917
- **X.-D. TONG ; B. XUE ; Y. SUN.** *Biotechnol. Prog.,* 2001, vol. 17, 134-139
- **A. SUAREZ ; B. GUIEYSSE ; B. MATTIASSON.** *Biotechnology Letters,* 2003, vol. 25, 927-933
- **H. ZILOUEI ; B. GUIEYSSE ; B. MATTHIASSON.** *Process Biochemistry,* 2006, vol. 41, 1083-1089
- **KLOSE, S ; TÖLLE, R ; BÄUCKER, E ; MAKESCHIN, F.** Stratigraphic distribution of lignite-derived atmospheric deposits in forest soils of the upper Lusatian Region, East Germany. *Water, Air and Soil Pollution,* 2003, vol. 142, 3-25